# EUROPÄISCHE PATENTSCHRIFT

(11) **EP 3 060 623 B1**
(45) Veröffentlichungstag und Bekanntmachung des Hinweises auf die Patenterteilung: **07.11.2018**
(21) Anmeldenummer: 14772289.6
(22) Anmeldetag: 24.09.2014
(51) Int. Cl.: C09K 11/06, C07C 209/00, C07C 211/00, H05B 33/10

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 23.10.2013 EP 13005070
(43) Veröffentlichungstag der Anmeldung: 31.08.2016
(73) Patentinhaber: Merck Patent GmbH, 64293 Darmstadt (DE)
(72) Erfinder: BUESING, Arne, 65929 Frankfurt (DE); MARTYNOVA, Irina, 64347 Gruesheim (DE); VOGES, Frank, 67098 Bad Duerkheim (DE); KROEBER, Jonas Valentin, 60311 Frankfurt am Main (DE)
(86) Internationale Anmeldenummer: PCT/EP2014/002586
(87) Internationale Veröffentlichungsnummer: WO 2015/058826

(56) Entgegenhaltungen:
- CN-A- 102 030 701
- LINGLING RONG ET AL: "Fluoradenes via palladium-catalyzed intramolecular arylation", CHEMICAL COMMUNICATIONS, Bd. 47, Nr. 7, 1. Januar 2011 (2011-01-01) , Seite 2155, XP055153455, ISSN: 1359-7345, DOI: 10.1039/c0cc05004k
- CHRISTIAN TUCHSCHERER ET AL: "Preparation of 2H-cyclopenta[j,k]fluorene and substituted fluoradenes", TETRAHEDRON LETTERS, Bd. 11, 1. Januar 1973 (1973-01-01), Seiten 865-868, XP055153488,
- AIBING XIA ET AL: "Two fluoradene derivatives: pseudosymmetry, eccentric ellipsoids and a phase transition Two fluoradene derivatives: pseudosymmetry, eccentric ellipsoids and a phase transition", ACTA CRYSTALLOGRAPHICA SECTION B, STRUCTURAL SCIENCE, Bd. B57, 1. Januar 2001 (2001-01-01), Seiten 507-516, XP055153468,

## Beschreibung

Die vorliegende Erfindung betrifft Materialien für die Verwendung in elektronischen Vorrichtungen, insbesondere in organischen Elektrolumineszenzvorrichtungen.

Die Entwicklung von funktionellen Verbindungen zur Verwendung in elektronischen Vorrichtungen ist aktuell Gegenstand intensiver Forschung. Ziel ist hierbei insbesondere die Entwicklung von Verbindungen, mit denen verbesserte Eigenschaften der elektronischen Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Insbesondere bei blau emittierenden OLEDs besteht Verbesserungspotential bezüglich der Lebensdauer der Vorrichtungen. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

Die Schrift CN 102030701A und die Publikationen Rong et al., Chem. Comm. 2011, 47, 2155; Tuchscherer et al., Tetrahedron Lett. 1973, 11, 865; und Xia et al., Act. Cryst. Sect. B, 2001, B57, 507 beschreiben Verbindungen mit Indeno[1,2,3-jk]fluoren-Gerüst. In der Schrift US 2006/0094859 A1 werden Polymere mit mehrfach verbrückten Biphenyl-Einheiten beschrieben.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial und/oder als Lochtransport-/ Elektronenblockiermaterial bzw. Exzitonenblockiermaterial und/oder als Elektronentransport- bzw. Lochblockiermaterial.

Der Übersichtlichkeit halber ist die Nummerierung des Indeno[1,2,3-jk]fluorens im Folgenden abgebildet:

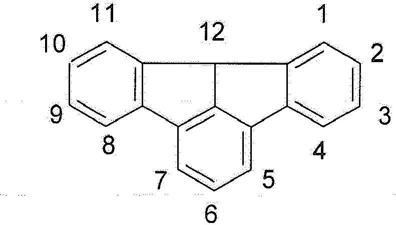

Gegenstand der vorliegenden Erfindung ist daher eine Verbindung gemäß Formel (1), gemäß Anspruch 1.

Eine Arylgruppe im Sinne dieser Erfindung enthält 6 bis 60 aromatische Ringatome; eine Heteroarylgruppe im Sinne dieser Erfindung enthält prinzipiell 5 bis 60 aromatische Ringatome, von denen mindestens eines ein Heteroatom darstellt. Die Heteroatome sind bevorzugt ausgewählt aus N, O und/oder S. Dies stellt die grundlegende Definition dar. Werden in der Beschreibung der vorliegenden Erfindung andere Bevorzugungen angegeben, wie beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder ein kondensierter (anellierter) aromatischer bzw. heteroaromatischer Polycyclus, beispielsweise Naphthalin, Anthracen, Phenanthren, Chinolin, Isochinolin, oder Carbazol, verstanden. Ein kondensierter (anellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen. Miteinander durch Einfachbindung verknüpfte Aromaten, wie zum Beispiel Biphenyl oder Bipyridin, werden dagegen nicht als Aryl- oder Heteroarylgruppe, sondern als aromatisches Ringsystem bezeichnet.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Füran, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 aromatische Ringatome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N- Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Füran, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1 ,2-Thiazol, 1 ,3-Thiazol, Benzothiazol, Pyridazin, Benzopyridazin, Pyrimidin, Benzpyrimidin, Chinoxalin, 1 ,5-Diazaanthracen, 2,7-Diazapyren, 2,3-Diazapyren, 1,6-Diazapyren, 1,8- Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3- Oxadiazol, 1,2,4-Oxadiazol, 1,2,5-Oxadiazol, 1,3,4-Oxadiazol, 1,2,3- Thiadiazol, 1,2,4-Thiadiazol, 1,2,5-Thiadiazol, 1,3,4-Thiadiazol, 1,3,5- Triazin, 1,2,4-Triazin, 1,2,3-Triazin, Tetrazol, 1,2,4,5-Tetrazin, 1,2,3,4- Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂₋Gruppen durch die oben bei der Definition der Reste genannten Gruppen substituiert sein können, bevorzugt die Reste Methyl, Ethyl, n-Propyl, iPropyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, nOctyloxy, Cyclooctyloxy, 2-Ethylhexyloxy, Pentafluorethoxy, 2,2,2- Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyciooctylthio, 2- Ethylhexylthio, Trifluormethylthio, Pentafluorethylthio, 2,2,2- Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

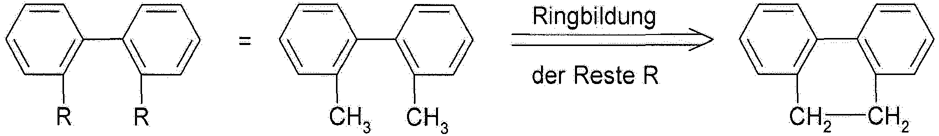

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

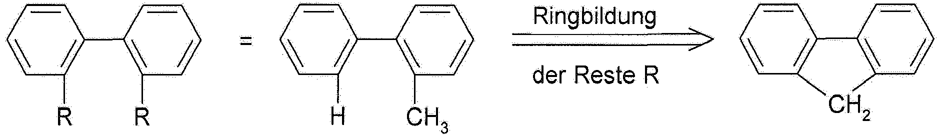

Wenn mindestens ein R¹ mindestens ein aromatisches oder heteroaromatisches Ringsystem umfasst, beträgt die Gesamtanzahl von aromatischen Ringatomen in allen R¹ und R² mindestens 12, bevorzugt mindestens 18, besonders bevorzugt mindestens 24.

Wenn R¹ kein aromatisches oder heteroaromatisches Ringsystem umfasst, umfasst R² mindestens 24 aromatische Ringatome, sowie kein weiteres Indeno[1,2,3-jk]fluoren-Gerüst.

Dadurch wird eine gewisse Flexibilität und Mindestgröße von R² erreicht, was sich positiv auf die Stabilität der Verbindung auswirkt, insbesondere bei der Verwendung bei Sublimation. Insbesondere die 12-Position stellt in lndeno[1,2,3-jk]fluoren-Gerüsten die acideste Position des Gerüsts dar. Daher sind diese Bindungen besonders instabil. Wenn noch weitere Indeno[1,2,3-jk]fluoren-Gerüste in der Verbindung sind, wird diese nicht nur sehr groß, sondern auch potentiell instabiler.

Bevorzugt umfassen R¹ und/oder R² keine anellierten aromatischen oder heteroaromatischen Ringsysteme mit mehr als 14 aromatischen Ringatomen.

Ebenso bevorzugt umfassen alle R¹ und R² zusammen eine Gesamtzahl von aromatischen Ringatomen von nicht mehr als 84, bevorzugt nicht mehr als 60.

Bevorzugte Ausführungsform der Verbindung der Formel (1) ist daher eine Verbindung der folgenden Formel (2): 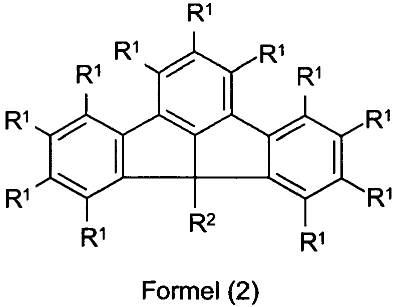 wobei die verwendeten Symbole die oben genannten Bedeutungen aufweisen.

In einer bevorzugten Ausführungsform der Erfindung ist R¹ in den oben genannten Formeln bei jedem Auftreten gleich oder verschieden H, D, F, Cl, Br, CN, NO₂, N(Ar¹)₂, N(R³)₂, C(=O)Ar¹, C(=O)R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die Alkyl-, Alkoxy-, Thioalkyl-, Alkenyl- oder Alkinylgruppe jeweils mit einem oder mehreren Resten R³ substituiert sein kann und wobei eine oder mehrere nicht benachbarte CH₂-Gruppen durch Si(R³)₂, -S- und -O-ersetzt sein können und wobei ein oder mehrere H-Atome durch D oder F ersetzt sein können, oder ein aromatisches Ringsystem mit 6 bis 30 aromatischen Ringatomen, das jeweils mit einem oder mehreren Reste(n) R³ substituiert sein kann, oder ein heteroaromatisches Ringsystem mit 5 bis 30 aromatischen Ringatomen, das jeweils mit einem oder mehreren Reste(n) R³ substituiert sein kann, wobei zwei oder mehr Reste R¹ miteinander verknüpft sein können und einen Ring bilden können.

In einer weiteren bevorzugten Ausführungsform der Erfindung ist R¹ in den oben genannten Formeln bei jedem Auftreten gleich oder verschieden H, D, F, CN, NO₂, N(Ar¹)₂, N(R³)₂, C(=O)Ar¹, C(=O)R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die Alkyl-, Alkoxy-, Thioalkyl-, Alkenyl- oder Alkinylgruppe jeweils mit einem oder mehreren Resten R³ substituiert sein kann und wobei eine oder mehrere nicht benachbarte CH₂-Gruppen durch Si(R³)₂, -S- und -O-ersetzt sein können und wobei ein oder mehrere H-Atome durch D oder F ersetzt sein können, oder ein aromatisches Ringsystem mit 6 bis 30 aromatischen Ringatomen, das jeweils mit einem oder mehreren Reste(n) R³ substituiert sein kann, oder ein heteroaromatisches Ringsystem mit 5 bis 30 aromatischen Ringatomen, das jeweils mit einem oder mehreren Reste(n) R³ substituiert sein kann, wobei zwei oder mehr Reste R¹ miteinander verknüpft sein können und einen Ring bilden können.

In einer weiteren bevorzugten Ausführungsform der Erfindung ist R¹ in den oben genannten Formeln bei jedem Auftreten gleich oder verschieden H, D, F, CN, NO₂, N(Ar¹)₂, N(R³)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen oder eine Alkenyl- oder Alkinylgruppe mit 2 bis 10 C-Atomen, wobei die Alkyl-, Alkoxy-, Alkenyl- oder Alkinylgruppe jeweils mit einem oder mehreren Resten R³ substituiert sein kann und wobei eine oder mehrere nicht benachbarte CH₂-Gruppen durch Si(R³)₂, -S- und -O- ersetzt sein können und wobei ein oder mehrere H-Atome durch D oder F ersetzt sein können, oder ein aromatisches Ringsystem mit 6 bis 30 aromatischen Ringatomen, das jeweils mit einem oder mehreren Reste(n) R³ substituiert sein kann, oder ein heteroaromatisches Ringsystem mit 5 bis 30 aromatischen Ringatomen, das jeweils mit einem oder mehreren Reste(n) R³ substituiert sein kann, wobei zwei oder mehr Reste R¹ miteinander verknüpft sein können und einen Ring bilden können.

In einer weiteren Ausführungsform der Erfindung ist R³ in den oben genannten Formeln bei jedem Auftreten gleich oder verschieden H, D, F, CN, N(R⁴)₂, C(=O)R⁴, Si(R⁴)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die Alkyl-, Alkoxy-, Thioalkyl-, Alkenyl- oder Alkinylgruppe jeweils mit einem oder mehreren Resten R⁴ substituiert sein kann und wobei eine oder mehrere benachbarte oder nicht benachbarte CH₂-Gruppen durch Si(R⁴)₂, NR⁴, -O- oder -S- ersetzt sein können und wobei ein oder mehrere H-Atome durch D, F oder CN ersetzt sein können, oder ein aromatisches Ringsystem mit 6 bis 18 aromatischen Ringatomen, das jeweils mit einem oder mehreren Reste(n) R⁴ substituiert sein kann, oder ein heteroaromatisches Ringsystem mit 5 bis 18 aromatischen Ringatomen, das jeweils mit einem oder mehreren Reste(n) R⁴ substituiert sein kann, wobei zwei oder mehr Reste R⁴ miteinander verknüpft sein können und einen Ring bilden können.

In einer weiteren Ausführungsform der Erfindung ist R¹ an mindestens einer der Positionen 5 oder 10 des lndeno[1,2,3-jk]fluoren-Gerüsts bei jedem Auftreten gleich oder verschieden H, D, F, CN, NO₂, N(Ar¹)₂, N(R³)₂, C(=O)Ar¹, P(=O)(Ar¹)₂, P(=O)(R³)₂, Si(R³)₂, OSO₂R³, S(=O)R³, S(=O)₂R³, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen oder eine Alkenyl- oder Alkinylgruppe mit 2 bis 40 C-Atomen, wobei die Alkyl-, Alkoxy-, Thioalkyl-, Alkenyl- oder Alkinylgruppe jeweils mit einem oder mehreren Resten R³ substituiert sein kann und wobei eine oder mehrere benachbarte oder nicht benachbarte CH₂-Gruppen durch -R³C=CR³-, -C≡C-,C=O, C=S, C=Se, C=NR³, -C(=O)-NR³-, P(=O)(R³), -C(=O)-O-, Si(R³)₂, NR³, -O-, -S-, SO oder SO₂ ersetzt sein können und wobei ein oder mehrere H-Atome durch D, F, Cl, Br, I, CN oder NO₂ ersetzt sein können, oder ein aromatisches Ringsystem mit 6 bis 60 aromatischen Ringatomen, das jeweils mit einem oder mehreren Reste(n) R³ substituiert sein kann, oder ein heteroaromatisches Ringsystem mit 5 bis 60 aromatischen Ringatomen, das jeweils mit einem oder mehreren Reste(n) R³ substituiert sein kann, wobei zwei oder mehr Reste R¹ miteinander verknüpft sein können und einen Ring bilden können. Besonders bevorzugt enthält R¹ an den Positionen 5 oder 10 des Indeno[1,2,3-jk]fluoren-Gerüsts, soweit jeweils vorhanden, keine Reste ausgewählt aus der Gruppe enthaltend Cl, Br, I, B(OR³)₂ und CHO.

Dabei haben für Verbindungen, die durch Vakuumverdampfung verarbeitet werden, die Alkylgruppen bevorzugt nicht mehr als 4 C-Atome. Für Verbindungen, die aus Lösung verarbeitet werden, eignen sich auch Verbindungen, die mit Alkylgruppen mit bis zu 10 C-Atomen substituiert sind oder die mit Oligoarylengruppen, beispielsweise ortho-, meta-, para- oder verzweigten Terphenylgruppen oder Quaterphenylgruppen, substituert sind.

In einer weiteren Ausführungsform der Erfindung ist mindestens ein R¹ nicht H, bevorzugt umfasst mindestens ein R¹ ein aromatisches oder heteroaromatisches Ringsystem.

Besonders bevorzugte aromatische Ringsysteme für R¹, bzw. R² sind ausgewählt aus der Gruppe Phenyl, ortho-, meta- oder para-Biphenyl, ortho-, meta- oder para-Terphenyl, ortho-, meta-, para- oder verzweigtes Quaterphenyl, Fluoren, Spirobifluoren, welche jeweils durch einen oder mehrere Reste R³ substituiert sein können.

Besonders bevorzugte aromatische Ringsysteme für R¹, bzw. R² sind insbesondere ausgewählt aus den Strukturen der folgenden Formeln: 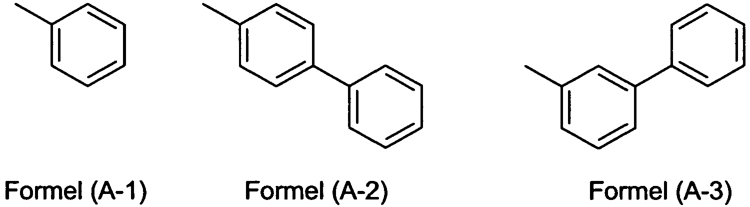 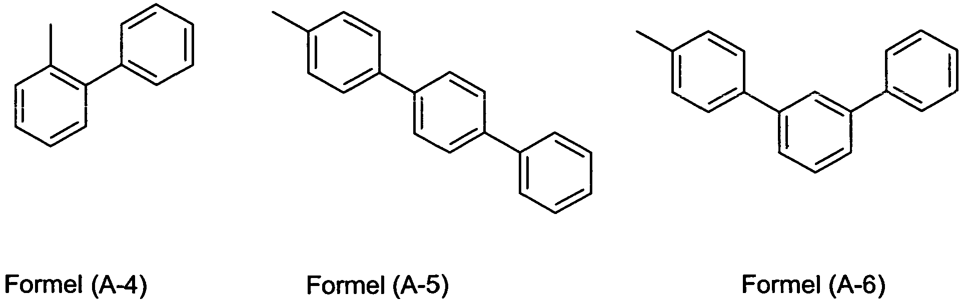 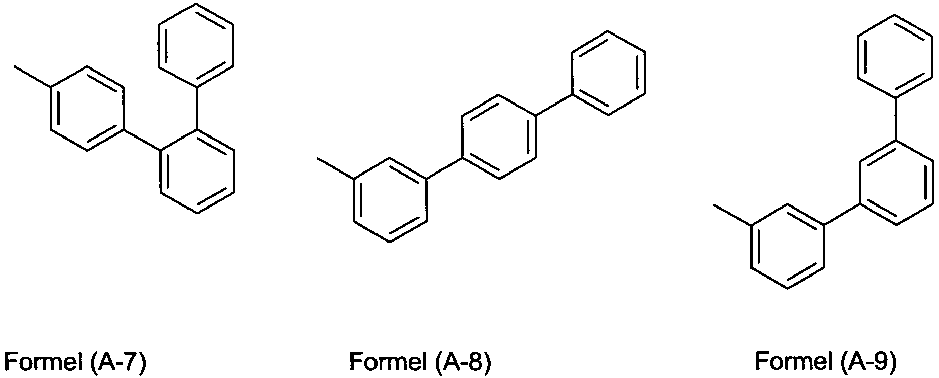 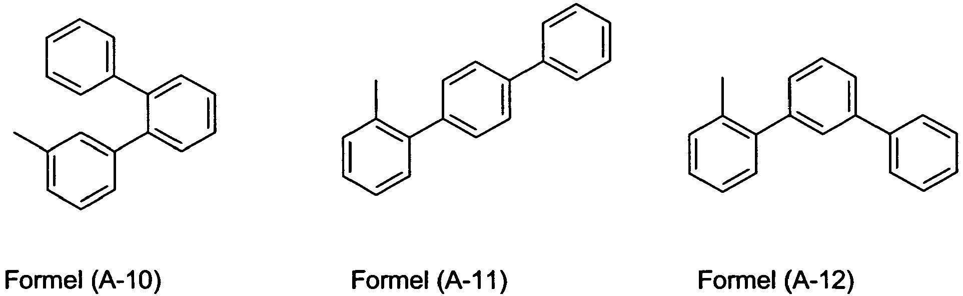 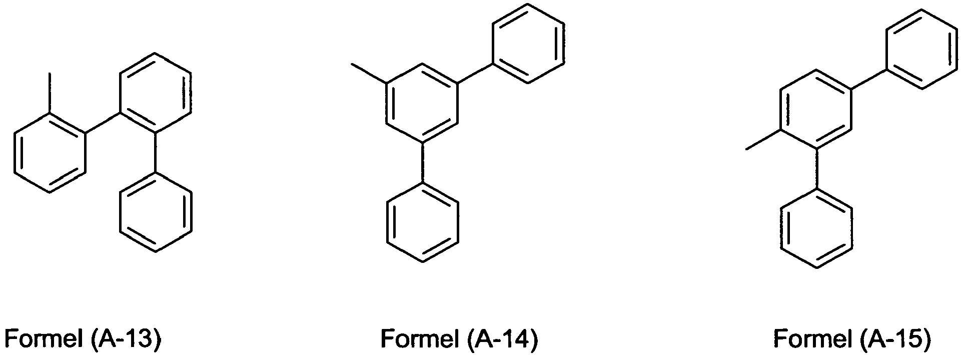 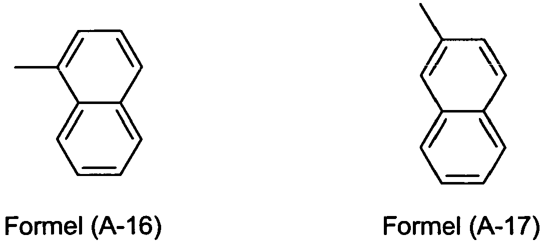 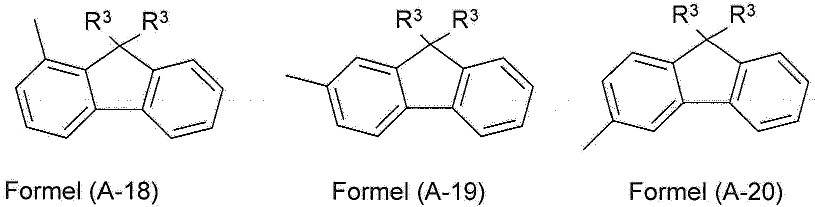 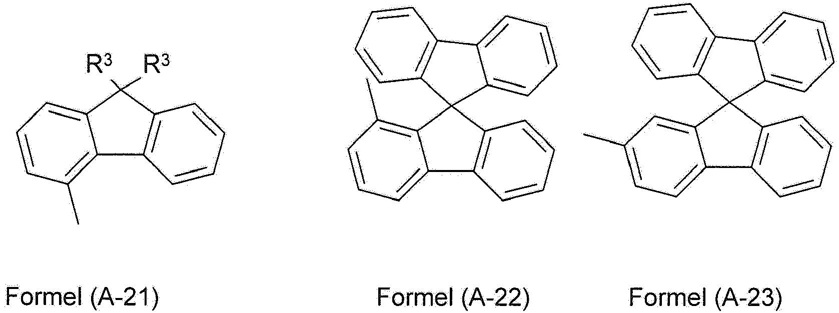 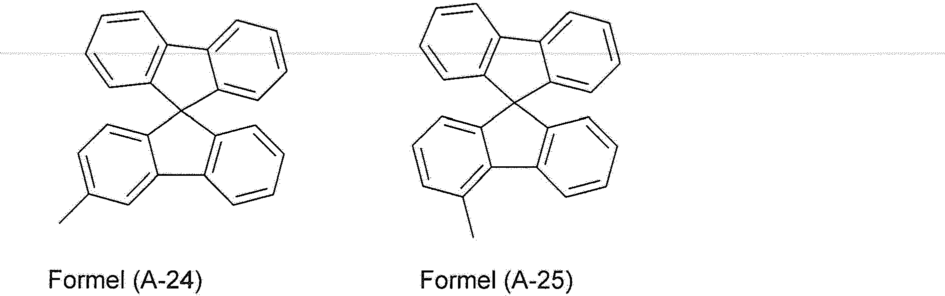 wobei die Strukturen mit einem oder mehreren Resten R³ substituiert sein können, und R³ definiert ist wie oben angegeben. Bevorzugt ist R³ dabei ein aliphatischer Rest mit 1 bis 20 C-Atomen, oder ein aromatisches Ringsystem mit 6 bis 24 aromatischen Ringatomen oder ein heteroaromatisches Ringsystem mit 5 bis 24 aromatischen Ringatomen, bevorzugt ein aliphatischer Rest mit 1 bis 10 C-Atomen oder ein aromatisches Ringsystem mit 6 bis 18 aromatischen Ringatomen.

Besonders bevorzugte heteroaromatische Ringsysteme R¹ enthalten als Heteroarylgruppe Triazin, Pyrimidin, Pyrazin, Pyridazin, Pyridin, Benzothiophen, Benzofuran, Indol, Carbazol, Azacarbazol, Diazacarbazol, Dibenzothiophen und/oder Dibenzofuran. Dabei sind die heteroaromatischen Ringsysteme insbesondere ausgewählt aus den Strukturen der folgenden Formeln (H-1) bis (H-14): 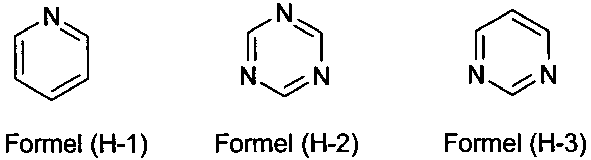 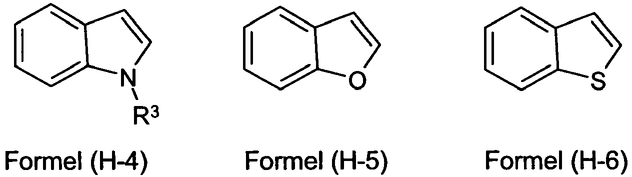 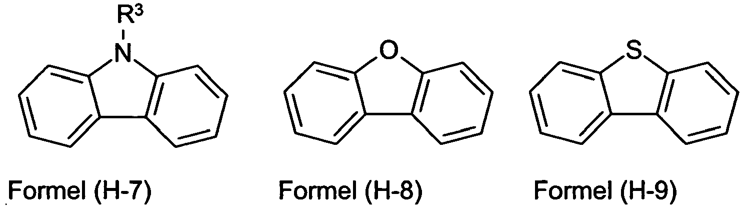 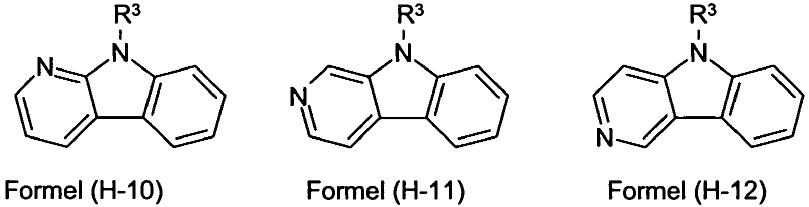 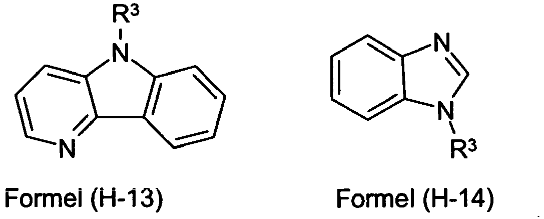 wobei die heteroaromatischen Gruppen an allen freien Positionen mit Resten R³ substituiert sein können und wobei die heteroaromatischen Gruppen an einer beliebigen Position mit dem Indeno[1,2,3-jk]fluoren-Gerüst verbunden sein können, wobei die Bindung auch an Stelle von NR³ treten kann und wobei R³ wie für Formel (1) definiert ist und die Bindung zum lndeno[1,2,3-jk]fluoren-Gerüst auch über ein divalentes aromatisches oder heteroaromatisches Ringsystem erfolgen kann, bevorzugt ein divalentes Ringsystem gemäß einer der Formeln (Ar3-1) bis (Ar3-12), besonders bevorzugt einer der Formeln (Ar3-1) bis (Ar3-4). Bevorzugt ist R³ ein aliphatischer Rest mit 1 bis 20 C-Atomen, oder ein aromatisches Ringsystem mit 6 bis 24 aromatischen Ringatomen oder ein heteroaromatisches Ringsystem mit 5 bis 24 aromatischen Ringatomen, bevorzugt ein aliphatischer Rest mit 1 bis 10 C-Atomen oder ein aromatisches Ringsystem mit 6 bis 18 aromatischen Ringatomen.

Wenn R¹ für eine Gruppe N(Ar¹)₂ steht, dann ist diese Gruppe bevorzugt ausgewählt aus den Strukturen der folgenden Formeln (3) oder (4), 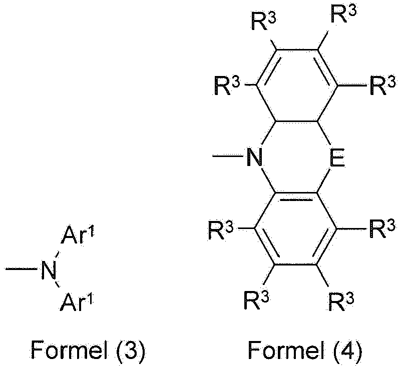 und wenn R¹, bzw. R² für ein aromatisches oder heteroaromatisches Ringsystem steht, welches eine Triarylamingruppe bzw. eine Triheteroarylamingruppe ist, dann ist diese Gruppe bevorzugt ausgewählt aus den Strukturen der folgenden Formel (5): 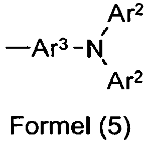 wobei die verwendeten Symbole die oben genannten Bedeutungen aufweisen, die gestrichelte Bindung die Bindung an den Indeno[1,2,3-jk]fluoren-Grundkörper darstellt und weiterhin gilt:
- Ar²: ist gleich oder verschieden und bei jedem Auftreten ein aromatisches oder heteroaromatisches Ringsystem mit 5 bis 24 aromatischen Ringatomen, welches mit einem der mehreren Resten R³ substituiert sein kann; dabei ist die Summe der aromatischen Ringatome aller Gruppen Ar² und Ar³ zusammen nicht größer als 60;
- Ar³: ist bei jedem Auftreten ein aromatisches oder heteroaromatisches Ringsystem mit 5 bis 24 aromatischen Ringatomen, welches mit einem der mehreren Resten R³ substituiert sein kann;
- E: ist ausgewählt aus der Gruppe bestehend aus einer Einfachbindung, C(R³)₂, NR³, O oder S.

Bevorzugte Ringsysteme für die Formeln Ar¹ in Formel (3) bzw. Ar² in Formel (5) sind Strukturen der Formeln (H-1) bis (H-15), wobei diese an allen freien Positionen mit Resten R³ substituiert sein können und die heteroaromatischen Gruppen an einer beliebigen freien Position mit dem N-Atom der Formel verbunden sein können, und Strukturen der Formeln (A-1) bis (A-25), wobei die Strukturen mit einem oder mehreren Resten R³ substituiert sein können und die gestrichelte Bindung die Bindung zum N-Atom der Formel darstellt, und R³ wie oben angegeben definiert ist.

Ganz besonders bevorzugt ist mindestens ein Ar² in Formel (5) ausgewählt aus den Strukturen (A-2) bis (A-25) oder (H-7) bis (H-9), insbesondere bevorzugt sind beide Ar² in Formel (5) ausgewählt aus den Strukturen (A-2) bis (A-25) oder (H-7) bis (H-9).

Ganz besonders bevorzugt ist mindestens ein Ar¹ in Formel (3) ausgewählt aus den Strukturen (A-2) bis (A-17) oder (H-7) bis (H-9), insbesondere bevorzugt sind beide Ar² in Formel (5) ausgewählt aus den Strukturen (A-2) bis (A-17) oder (H-7) bis (H-9).

Bevorzugt ist Ar³ ausgewählt aus divalenten Gruppen der folgenden Formeln (Ar3-1) bis (Ar3-12): 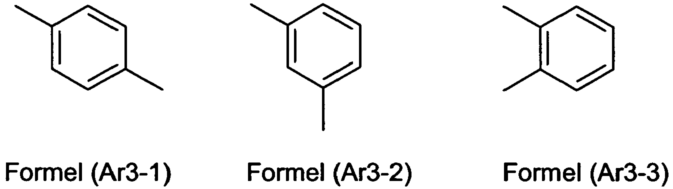 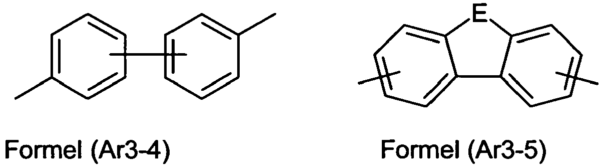 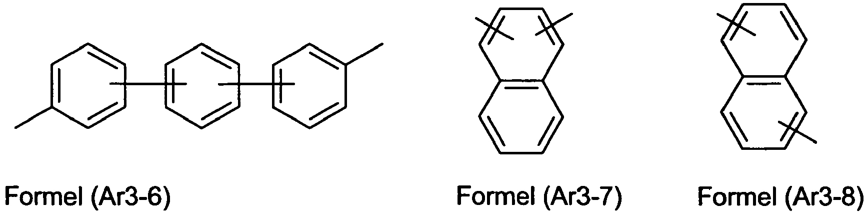 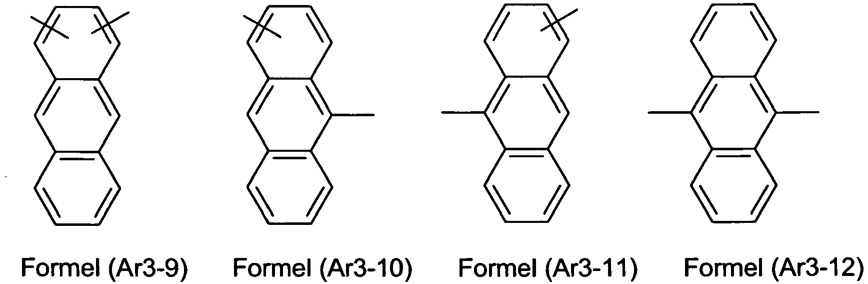 wobei E bei jedem Auftreten gleich oder verschieden eine divalente Gruppe ausgewählt aus -C(R³)₂-, -C(=O)-, -O-, -S-, -(S=O)-, -S(=O)₂-,-NR³- ist, wobei R³ wie für Formel (1) definiert ist. Besonders bevorzugt ist Ar³ einer der Strukturen (Ar3-1) bis (Ar3-4).

In einer bevorzugten Ausführungsform umfasst die Verbindung genau 1, 2, 3, 4 oder 5, bevorzugt 1, 2 oder 3, besonders bevorzugt 1 oder 2, Reste R¹ und/oder R² ausgewählt aus den Formeln (3), (4) und (5), bevorzugt aus den Formeln (3) und (5).

In einer weiteren Ausführungsform enthält die Verbindung genau einen Rest R¹ oder R² ausgewählt aus den Formeln (3), (4) und (5).

Bevorzugte Ausführungsformen der Verbindung nach Formel (1) sind die Verbindungen der Formeln (1-3a) bis (1-3g) und Formeln (1-5a) bis (1-5g): 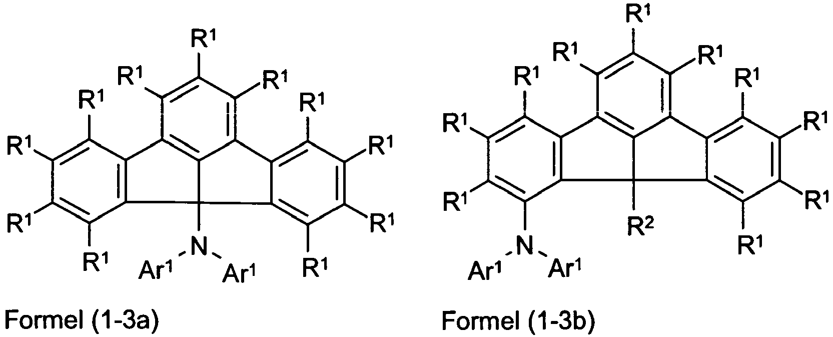 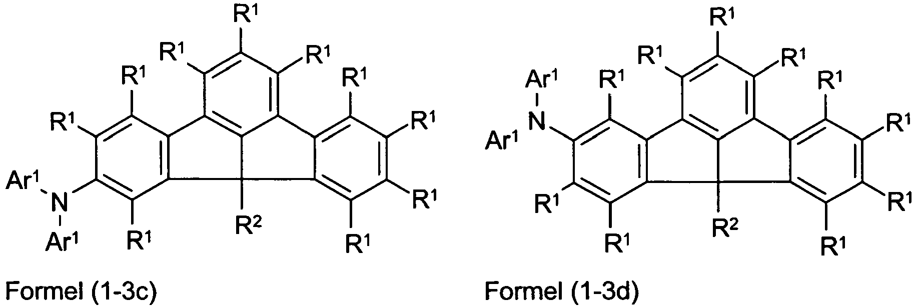 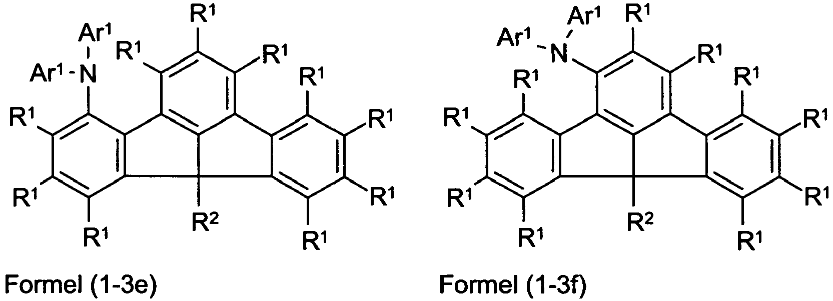 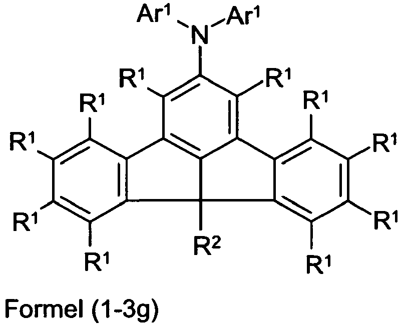 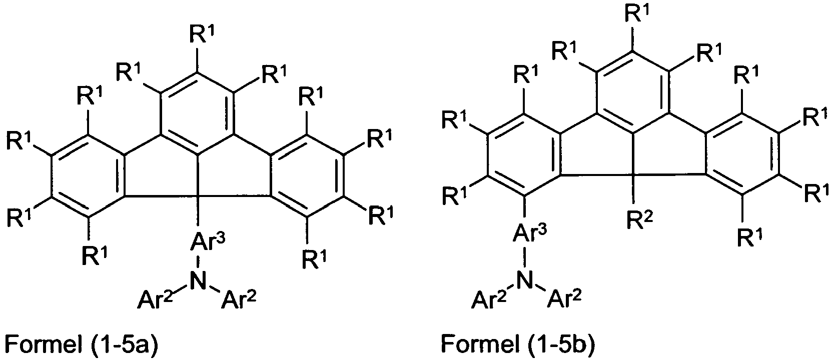 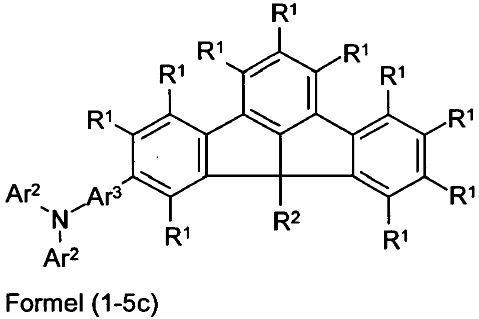 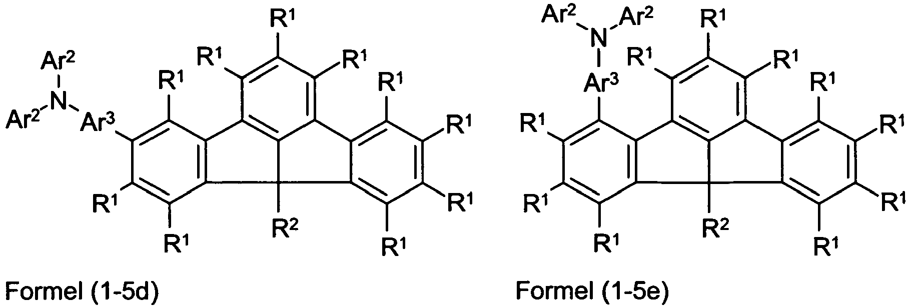 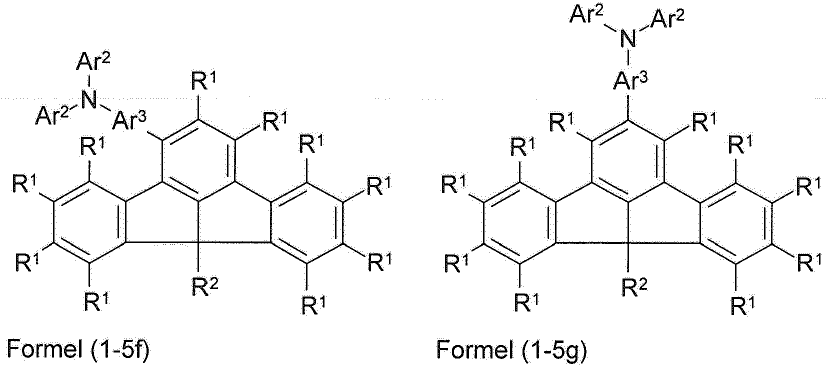 wobei R¹, R², Ar¹, Ar² und Ar³ wie vorstehend definiert sind.

In einer bevorzugten Ausführungsform der Erfindung ist in R² soweit vorhanden in Bezug auf das Indeno[1,2,3-jk]fluoren-Gerüst kein Stickstoffatom, bevorzugt Heteroatom, in para-Stellung an einem 6-Ring-Arylsystem direkt am lndeno[1,2,3-jk]fluoren-Gerüst angeordnet. Dies gilt auch für annelierte aromatische Ringsysteme, welche Carbazolstrukturen enthalten, welche an 3-Position an das Indeno[1,2,3-jk]fluoren-Gerüst gebunden sind. Überraschenderweise wurde gefunden, dass solche Verbindungen deutlich instabiler sind als Verbindungen, welche das Stickstoffatom in ortho- oder meta-Stellung aufweisen.

In einer bevorzugten Ausführungsform der Erfindung ist Ar³ in Formel (1-5a) ausgewählt aus den Strukturen (Ar3-2) bis (Ar3-11), bevorzugt ist dabei die Bindung zum lndeno[1,2,3-jk]fluoren-Gerüst in Formal (Ar3-5) an den Positionen 1, 2 oder 4 und in Formel (Ar3-7) und (Ar3-9) nicht in para-Position zur Bindung zum Stickstoff. Besonders bevorzugt ist Ar³ ausgewählt aus den Strukturen (Ar3-2) und (Ar3-3).

Daher ist eine bevorzugte Ausführungsform der Verbindung nach Formel (1-5a) eine Verbindung der Formeln 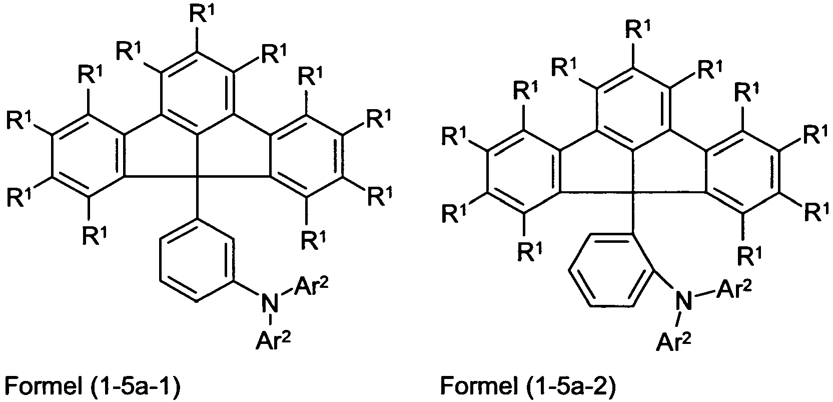 wobei R¹ und Ar² wie vorstehend definiert sind und die Verbindung an den freien Positionen mit einem oder mehreren Resten R³ substituiert sein kann, bevorzugt jedoch unsubstituiert ist.

In einer bevorzugten Ausführungsform der Erfindung sind R¹ und R² soweit vorhanden, in den Formeln (1-3a) bis (1-3g), (1-5a) bis (1-5g), bzw. Formeln (1-5a-1) und (1-5a-2), keine oder genau eine weitere Gruppe ausgewählt aus den Formeln (3), (4) oder (5).

In einer bevorzugten Ausführungsform der Erfindung sind R¹ und R² soweit vorhanden, in den Formeln (1-3a) bis (1-3g) und (1-5a) bis (1-5g), bzw. den Formeln (1-5a-1) und (1-5a-2), keine oder genau eine weitere Gruppe ausgewählt aus den Formeln (3), (4) oder (5) und noch mindestens ein weiterer R¹ und/oder R² soweit vorhanden ein aromatisches oder heteroaromatisches Ringsystem, besonders bevorzugt ein aromatisches oder heteroaromatisches Ringsystem der Formeln (A-1) bis (A-25) oder (H-1) bis (H-14), ganz besonders bevorzugt ein aromatisches Ringsystem, insbesondere gemäß der Formeln (A-1) bis (A-25).

In einer bevorzugten Ausführungsform der Erfindung bilden die mehr als ein R¹ untereinander keine an das lndeno[1,2,3-jk]fluoren-Gerüst anellierte aromatische oder heteroaromatische Ringsysteme.

Je nach Verwendung der erfindungsgemäßen Verbindungen werden unterschiedliche Substituenten R¹ und R² ausgewählt.

Wenn die Verbindung der Formel (1) bzw. (2) als Matrixmaterial für einen phosphoreszierenden Emitter verwendet wird, steht bevorzugt mindestens ein R¹ und/oder R² für N(Ar¹)₂, C(=O)Ar¹, P(=O)(Ar¹)₂ oder ein aromatisches oder heteroaromatisches Ringsystem mit 5 bis 60 aromatischen Ringatomen, welches mit einem oder mehreren Resten R³ substituiert sein kann. Besonders bevorzugt sind in diesem Fall mindestens ein R¹ und/oder R² ausgewählt aus den Formeln (3), (4) und (5) und ihren vorstehend genannten Ausführungsformen.

Wenn die Verbindung der Formel (1), bzw. (2) als Matrixmaterial für einen fluoreszierenden Emitter verwendet wird, steht bevorzugt mindestens ein R¹ und/oder R² für ein aromatisches oder heteroaromatisches Ringsystem, welches mindestens eine Arylgruppe mit mindestens drei kondensierten Sechsringen enthält, bevorzugt Anthracen.

Wenn die Verbindung der Formel (1) bzw. (2) als fluoreszierender Emitter verwendet wird, umfasst bevorzugt mindestens ein R¹ und/oder R² für ein aromatisches oder heteroaromatisches Ringsystem, welches mindestens eine Arylgruppe oder ein aromatisches Ringsystem mit mindestens zwei kondensierten Sechsringen enthält, die bevorzugt direkt oder über eine Phenylgruppe an das Indeno[1,2,3-jk]fluoren-Gerüst gebunden ist. Dabei ist die kondensierte Arylgruppe bevorzugt ausgewählt aus Anthracen, Pyren, Phenanthren, Chrysen, Monobenzoindenofluoren oder Dibenzoindenofluoren.

Wenn die Verbindungen der Formel (1) bzw. (2) als Elektronentransportmaterial verwendet werden, steht bevorzugt mindestens ein Rest R¹ für ein elektronenarmes heteroaromatisches Ringsystem mit 5 bis 40 aromatischen Ringatomen, bevorzugt mit 5 bis 25 aromatischen Ringatomen, welches mit einem oder mehreren Resten R³ substituiert sein kann. Ein elektronenarmes heteroaromatisches Ringsystem im Sinne der vorliegenden Erfindung ist ein heteroaromatisches Ringsystem, welches mindestens eine elektronenarme Heteroarylgruppe enthält, wobei es sich dabei entweder um eine 6-Ringheteroarylgruppe mit mindestens einem Stickstoffatom oder um eine 5-Ringheteroarylgruppe mit mindestens zwei Heteroatomen handelt.

Besonders bevorzugte elektronenarme heteroaromatische Ringsysteme R¹ enthalten als Heteroarylgruppe mindestens eine Gruppe gewählt aus Triazin, Pyrimidin, Pyrazin, Pyridazin, Pyridin, Imidazol, Pyrazol, Oxazol, Oxadiazol, Triazol, Thiazol, Thiadiazol, Benzimidazol, Chinolin, Isochinolin und Chinoxalin. Dabei sind die heteroaromatischen Ringsysteme insbesondere ausgewählt aus den Strukturen der Formeln (H-1), (H-2), (H-3), (H-14), wobei R³ die dabei genannten Bedeutungen aufweist und die Strukturen an einer freien Position über eine Einfachbindung oder über eine Phenylgruppe an das Indeno[1,2,3-jk]fluoren-Gerüst gebunden sind.

Wenn die Verbindungen der Formel (1) bzw. (2) als Lochtransportmaterial oder als emittierende Verbindung verwendet werden, steht bevorzugt mindestens ein Rest R¹ und/oder R² für N(Ar¹)₂, für eine Triarylaminogruppe oder für ein elektronenreiches heteroaromatisches Ringsystem mit 5 bis 40 aromatischen Ringatomen, insbesondere 5 bis 25 aromatischen Ringatomen, welches mit einem oder mehreren Resten R³ substituiert sein kann, insbesondere für einen Rest gemäß der oben genannten Formeln (3), (4) oder (5). Ein elektronenreiches heteroaromatisches Ringsystem in Sinne der Erfindung ist ein heteroaromatisches Ringsystem, welches mindestens eine elektronenreiche Heteroarylgruppe enthält, wobei es sich dabei um eine 5-Ringheteroarylgruppe mit genau einem Heteroatom handelt, an die auch eine oder mehrere Arylgruppen ankondensiert sein können.

Besonders bevorzugte elektronenreiche heteroaromatisches Ringsysteme R¹ enthalten als Heteroarylgruppe Pyrrol, Furan, Thiophen, Benzothiophen, Benzofuran, Indol, Carbazol, Dibenzothiophen, Dibenzofuran und/oder Azacarbazol. Dabei sind die elektronenreichen heteroaromatischen Ringsysteme insbesondere ausgewählt aus den Strukturen der oben genannten Formeln (H-4) bis (H-13).

In einer Ausführungsform der Erfindung sind die oben genannten Bevorzugungen beliebig miteinander kombinierbar.

Beispiele für bevorzugte Verbindungen gemäß der oben aufgeführten Ausführungsformen, bzw. Verbindungen, wie sie bevorzugt in elektronischen Vorrichtungen eingesetzt werden können, sind die Verbindungen der folgenden Strukturen (1) bis (55).

| | | |
|---|---|---|
| 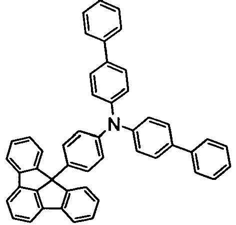 | 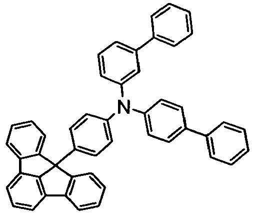 | 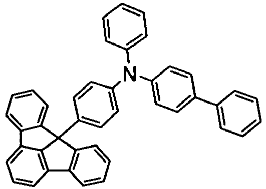 |
| 1 | 2 | 3 |
| 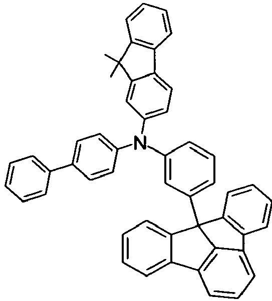 | 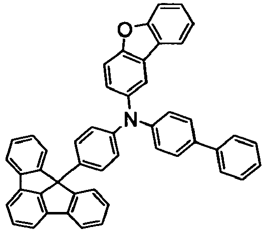 | 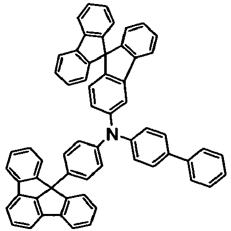 |
| 4 | 5 | 6 |
| 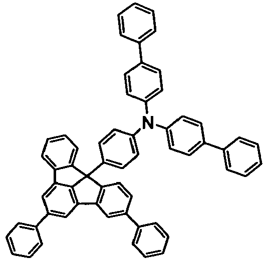 | 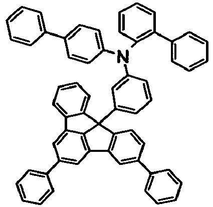 | 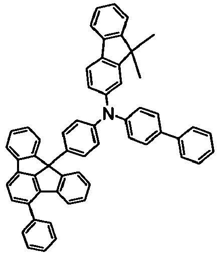 |
| 7 | 8 | 9 |
| 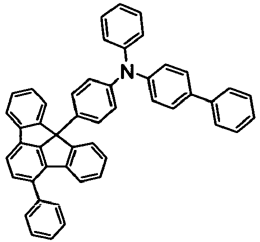 | 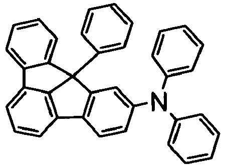 | 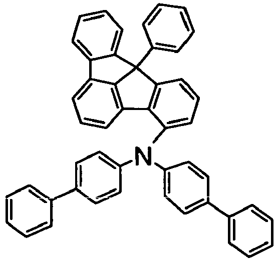 |
| 10 | 11 | 12 |
| 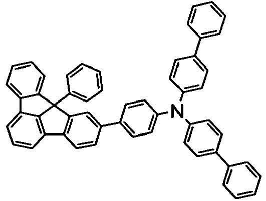 | 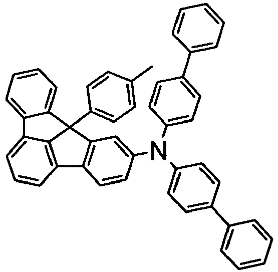 | 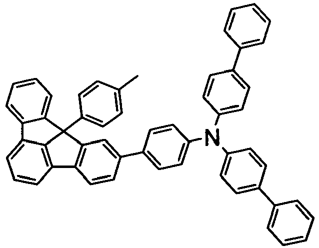 |
| 13 | 14 | 15 |
| 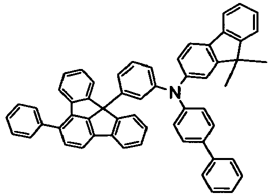 | 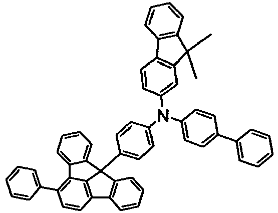 | 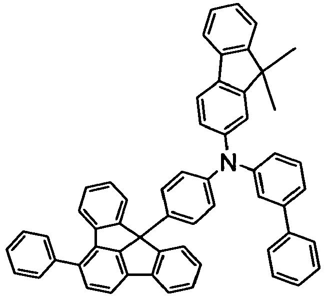 |
| 16 | 17 | 18 |
| 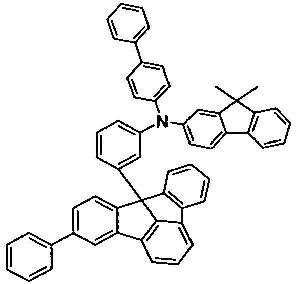 | 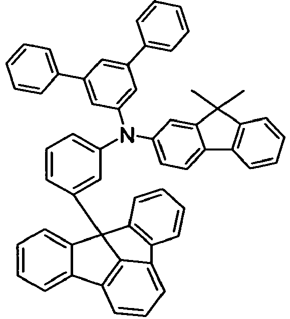 | 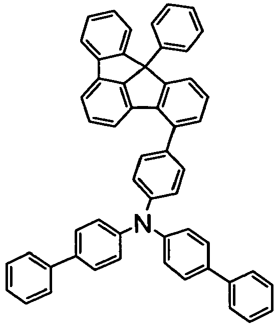 |
| 19 | 20 | 21 |
| 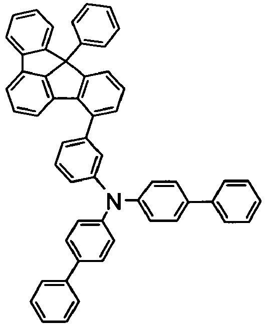 | 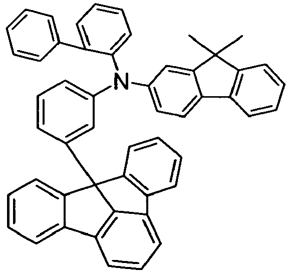 | 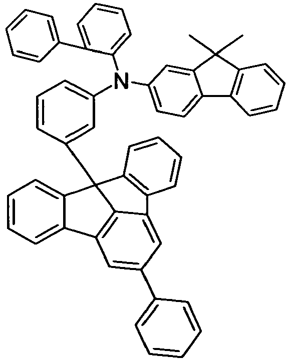 |
| 22 | 23 | 24 |
| 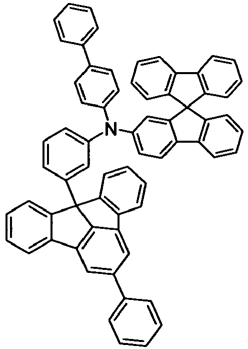 | 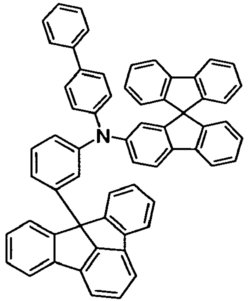 | 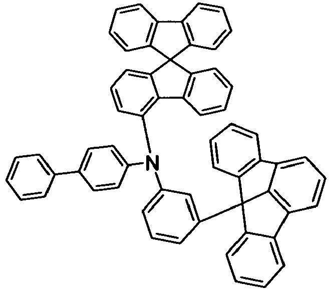 |
| 25 | 26 | 27 |
| 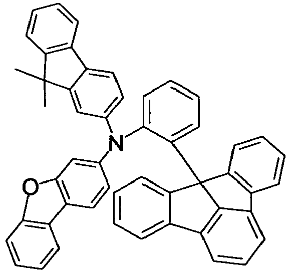 | 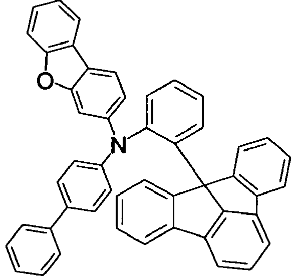 | 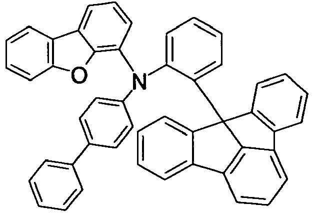 |
| 28 | 29 | 30 |
| 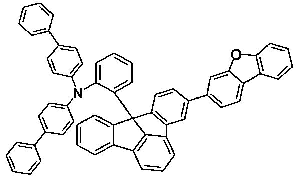 | 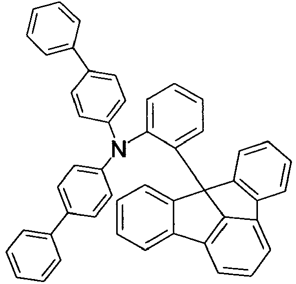 | 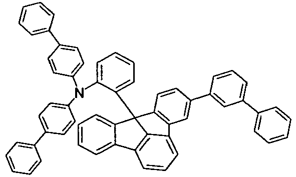 |
| 31 | 32 | 33 |
| 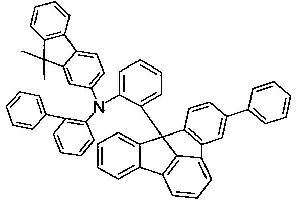 | 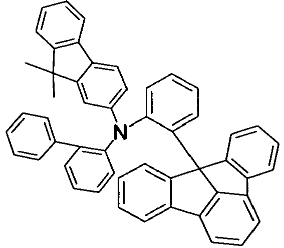 | 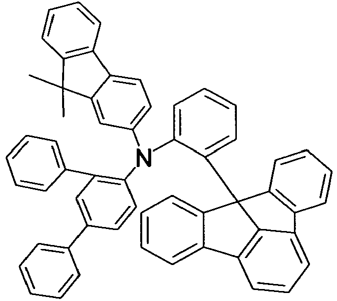 |
| 34 | 35 | 36 |
| 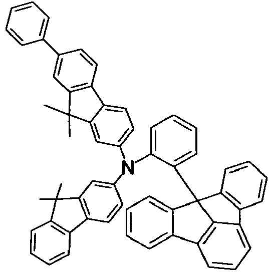 | 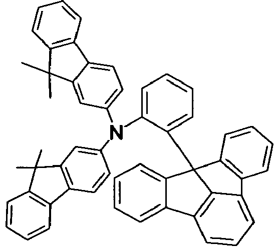 | 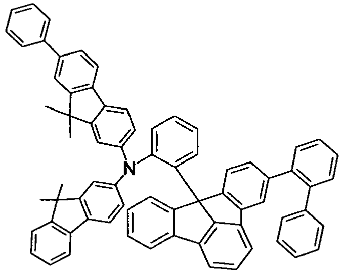 |
| 37 | 38 | 39 |
| 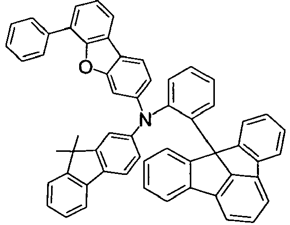 | 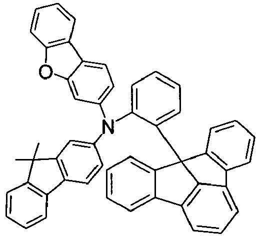 | 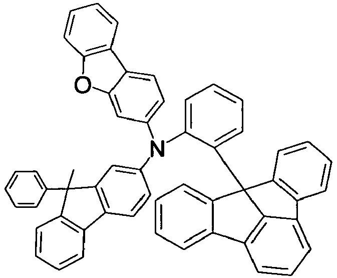 |
| 40 | 41 | 42 |
| 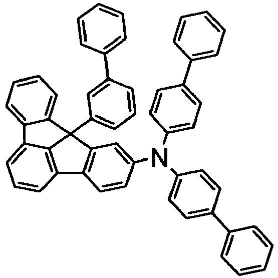 | 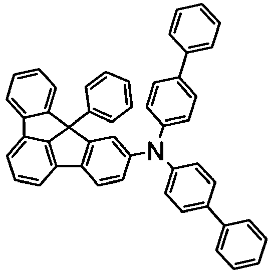 | 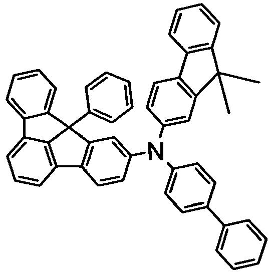 |
| 43 | 44 | 45 |
| 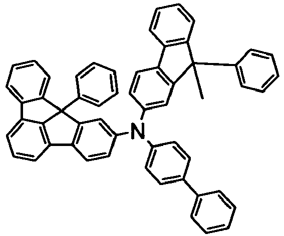 | 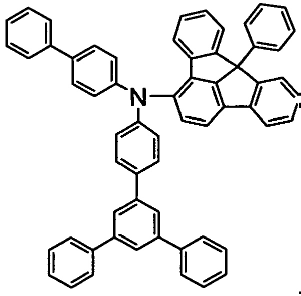 | 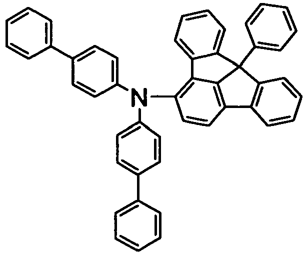 |
| 46 | 47 | 48 |
| 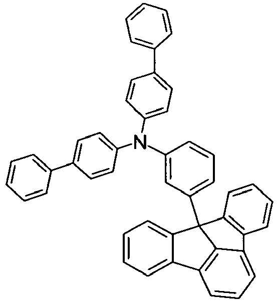 | 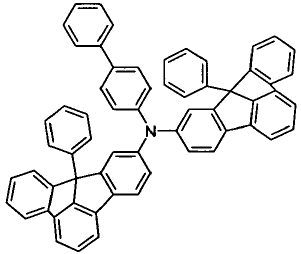 | 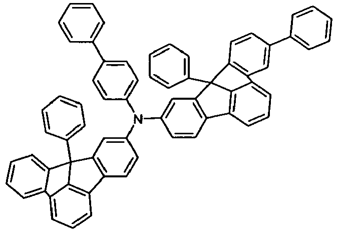 |
| 49 | 50 | 51 |
| 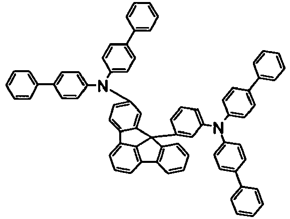 | 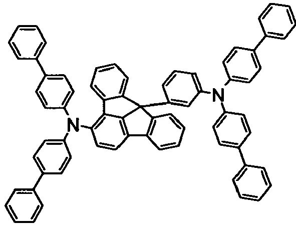 | 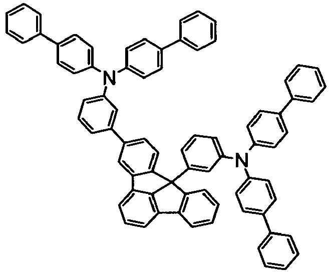 |
| 52 | 53 | 54 |
| 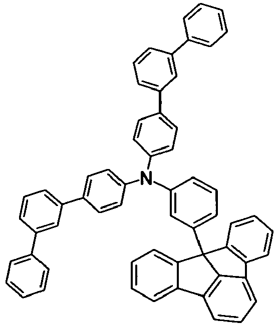 | | |
| 55 | | |

Die Synthese der erfindungsgemäßen Verbindungen kann gemäß im Stand der Technik bekannten Verfahren und Reaktionstypen, beispielsweise Friedel-Crafts Reaktion, Palladium-katalysierte intramolekulare C-H-Arylierung, Buchwald-Kupplung und Suzuki-Kupplung und Grignard-Reaktion erfolgen.

Ein bevorzugtes erstes Verfahren zur Herstellung der erfindungsgemäßen Verbindungen geht von den in Schema 1 als Edukte abgebildeten Grundstrukturen aus. Diese sind in einigen Fällen kommerziell erhältlich, in anderen Fällen können sie in wenigen Syntheseschritten aus einfachen, kommerziell erhältlichen Verbindungen hergestellt werden: 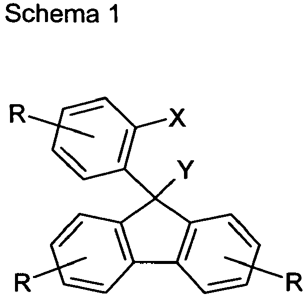
R: organischer Rest (soweit vorhanden)
X: Hal oder andere reaktive Abgangsgruppe
Y: OH, Hal oder andere reaktive Abgangsgruppe

Solche Verbindungen können beispielsweise durch Umsetzung von Fluorenon mit entsprechend substituiertem Arylhalogenid im Rahmen einer Grignard-Reaktion erhalten werden: 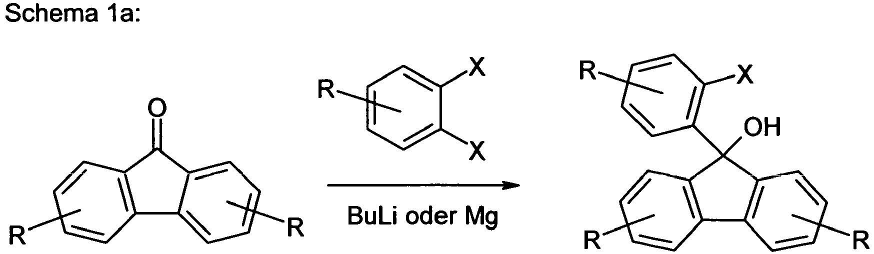
R: organischer Rest (soweit vorhanden)
X: Hal oder andere reaktive Abgangsgruppe

In einem nächsten Schritt wird in die erhaltene Verbindung der gewünschte R² anstatt der Gruppe Y eingeführt. Wenn R² ein aromatisches Ringsystem umfasst, geschieht dies bevorzugt über eine Friedel-Crafts-Reaktion. 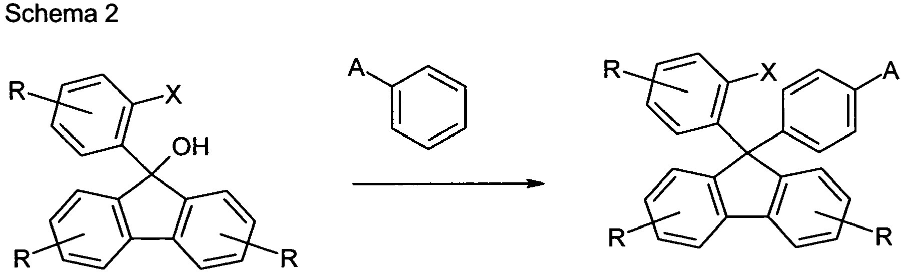
R: organischer Rest (soweit vorhanden)
X: Hal oder andere reaktive Abgangsgruppe
A: aktivierender organischer Rest (soweit vorhanden)

Aufgrund der Friedel-Crafts-Reaktion können dabei keine elektronenarmen Aromaten eingeführt werden. Insofern eignet sich dieser Reaktionsweg vor allem für unsubstituierte oder alkylierte Aromaten oder Arylamine (A ist N(Ar)₂). Gleichzeitig bestimmt die Friedel-Crafts-Reaktion auch die Position des eventuell vorhandenen aktivierenden Rests in para-Position.

In einem nächsten Schritt wird in einer intramolekularen Ringschlussreaktion das lndeno[1,2,3-jk]fluoren-Gerüst aufgebaut: 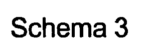 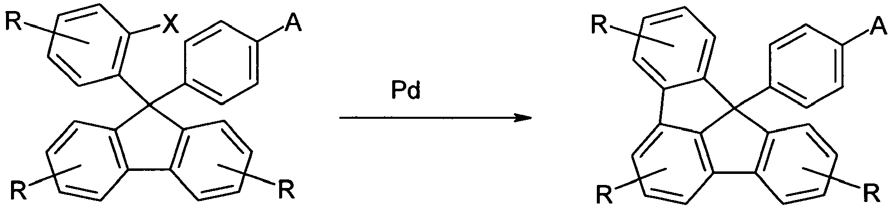
R: organischer Rest (soweit vorhanden)
X: Hal oder andere reaktive Abgangsgruppe
A: aktivierender organischer Rest (soweit vorhanden)

Ein bevorzugtes zweites Verfahren zur Herstellung der erfindungsgemäßen Verbindungen geht von dem in Schema 4 als Edukt abgebildeten Benzophenon-Derivat aus. Diese sind in einigen Fällen kommerziell erhältlich, in anderen Fällen können sie in wenigen Syntheseschritten aus einfachen, kommerziell erhältlichen Verbindungen hergestellt werden: 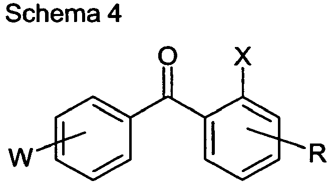
R: organischer Rest (soweit vorhanden)
W: organischer Rest (soweit vorhanden), insbesondere N(Ar)₂
X: Hal oder andere reaktive Abgangsgruppe

In einer bevorzugten Ausführungsform des zweiten Verfahrens wird die Ausgangsverbindung durch eine Grignard-Reaktion an eine CN-Gruppe aufgebaut: 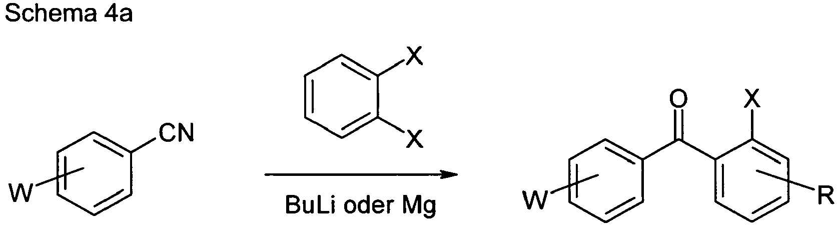
R: organischer Rest (soweit vorhanden)
W: organischer Rest (soweit vorhanden)
X: Hal oder andere reaktive Abgangsgruppe

Dabei stellt die Benzonitril-Verbindung die Vorläuferverbindung für den späteren R² dar. Mehrere Reste W können dabei auch Ringe bilden, wie auch kondensierte (anellierte) Ringe, oder zusammen ein aromatisches oder heteroaromatisches Ringsystem bilden, z.B. Carbazole. W ist bevorzugt eine R₂N-Gruppe, wobei R bevorzugt gleich oder verschieden eine Aryl- oder Heteroarylgruppe ist.

In einer besonderen Ausführungsform des Verfahrens ist der Rest W ein Alkyl- oder Arylamin. Diese kann über eine Buchwald-Kupplung eingeführt werden: 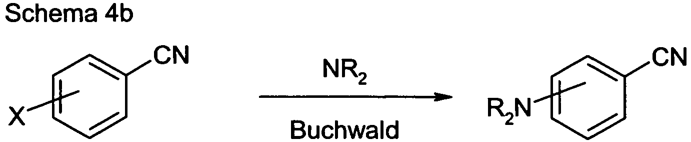
R: H oder organischer Rest, bevorzugt Aryl- oder Heteroarylgruppe
X: Hal oder andere reaktive Abgangsgruppe

In einer besonders bevorzugten Ausführungsform ist die Alkyl- oder Arylamingruppe in ortho- oder meta-Position zur Nitrilgruppe: 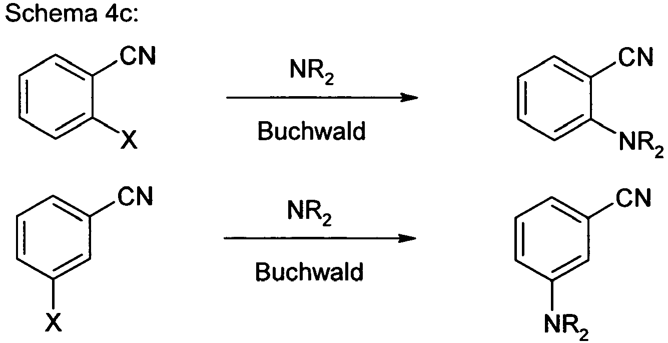 Die Verbindung aus Schema 4 wird im Rahmen einer Grignard-Reaktion mit einer substituierten Biphenylverbindung umgesetzt: 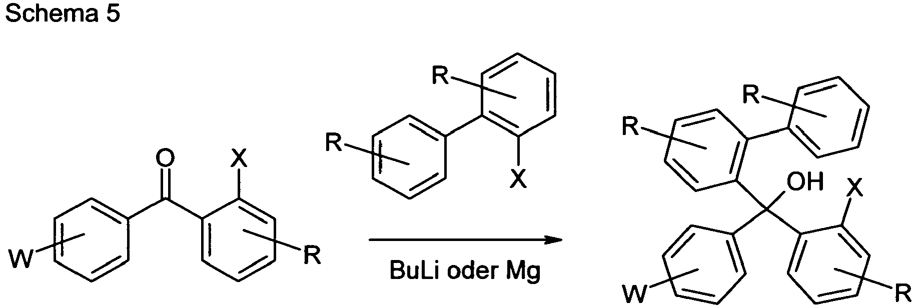
R: organischer Rest (soweit vorhanden)
W: organischer Rest (soweit vorhanden)
X: Hal oder andere reaktive Abgangsgruppe

In einem nächsten Schritt wird mit einer intramolekularen elektrophilen Reaktion der erste Teil des Indeno[1,2,3-jk]fluoren-Gerüsts aufgebaut:

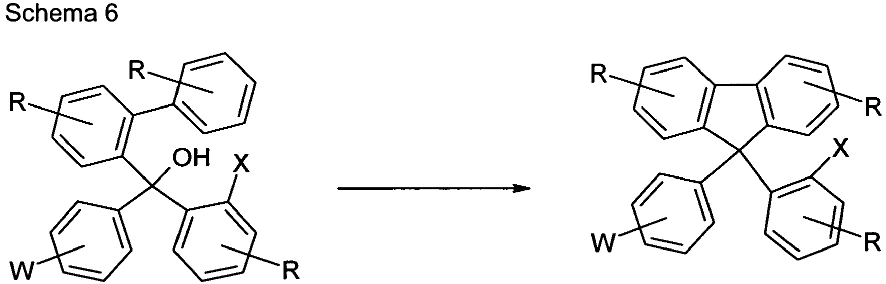

In einem nächsten Schritt wird in einer intramolekularen Ringschlussreaktion unter Übergangsmetallkatalyse das Indeno[1,2,3-jk]fluoren-Gerüst aufgebaut: 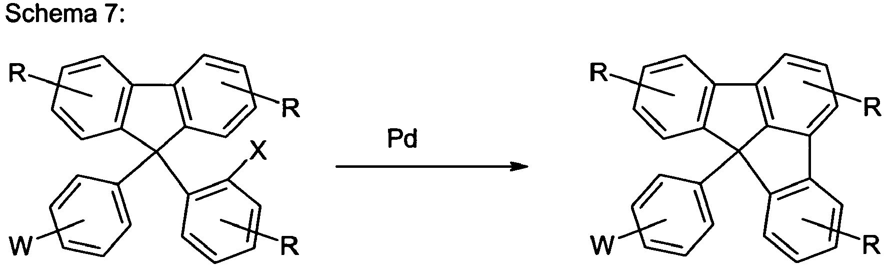
R: organischer Rest (soweit vorhanden)
W: organischer Rest (soweit vorhanden), insbesondere N(Ar)2
X: Hal oder andere reaktive Abgangsgruppe

Da bei diesem erfindungsgemäßen Verfahren der R² des späteren Indeno[1,2,3-jk]fluoren-Gerüsts nicht über eine Friedel-Crafts-Reaktion eingeführt wird, sind andere Substitutionsmuster an R² erhältlich. Besonders bevorzugt sind dabei Gruppen in meta- und ortho-Position in Bezug auf das Indeno[1,2,3-jk]fluoren-Gerüst, besonders bevorzugt Stickstoffsubstituenten wie Arylamine an diesen Positionen.

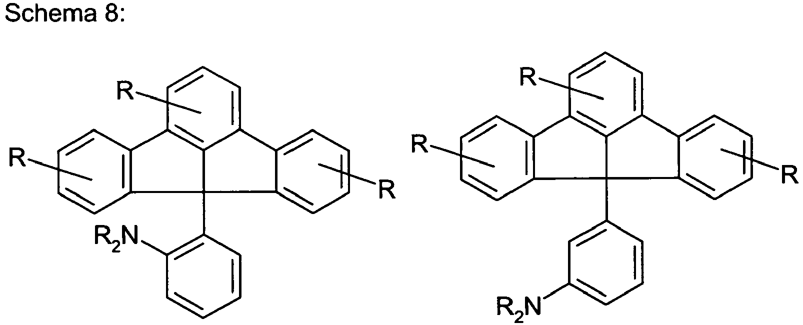

Die oben gezeigte Syntheseverfahren haben exemplarischen Charakter und können vom Fachmann auf dem Gebiet der organischen Synthese in geeigneter Weise abgewandelt werden, wenn dies für die Synthese bestimmter Ausführungsformen von erfindungsgemäßen Verbindungen vorteilhaft ist.

Einen weiteren Gegenstand der vorliegenden Erfindung stellt also ein Verfahren zur Herstellung von Verbindungen gemäß Formel (1), bzw. (2) dar, welches dadurch gekennzeichnet ist, dass ausgehend von einem Fluorenon-Derivat eine Addition an die Ketogruppe und eine bevorzugt übergangsmetallkatalysierte Kupplungsreaktion zum Ringschluss zum lndeno[1,2,3-jk]fluoren-Gerüst durchgeführt werden.

Einen weiteren Gegenstand der vorliegenden Erfindung stellt also ein weiteres Verfahren zur Herstellung von Verbindungen mit Indeno[1,2,3-jk]fluoren-Gerüst, bevorzugt Verbindungen gemäß Formel (1), bzw. (2) dar, welches dadurch gekennzeichnet ist, dass an einem Arylketon, welches in ortho-Position zur Ketogruppe eine Abgangsgruppe aufweist, mit einem Biaryl-Derivat eine Addition an die Ketogruppe durchgeführt wird und anschließend über eine intramolekulare elektrophile Reaktion und eine bevorzugt übergangsmetallkatalysierte intramolekulare Kupplungsreaktion das lndeno[1,2,3-jk]fluoren-Gerüst aufgebaut wird.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. CC-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (1), bzw. (2), wobei die Bindung(en) zum Polymer, Oligomer oder Dendrimer an beliebigen, in Formel (1), bzw (2) mit R¹ und/oder R² substituierten Positionen lokalisiert sein können. Je nach Verknüpfung der erfindungsgemäßen Verbindung ist die Verbindung Bestandteil einer Seitenkette des Oligomers oder Polymers oder Bestandteil der Hauptkette.

Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist.

Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein.

In den linear verknüpften Strukturen können die Einheiten gemäß Formel (1), bzw. (2) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein.

In verzweigten und dendritischen Strukturen können beispielsweise 3, 5 oder mehrere Einheiten gemäß Formel (1), bzw. (2) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (1), bzw. (2) in Oligomeren, Dendrimeren und Polymeren gelten dieselben Bevorzugungen wie oben für die erfindungsgemäßen Verbindungen beschrieben.

In einer Ausführungsform der Erfindung sind die Bindungen zum Oligomer, Polymer oder Dendrimer nicht an beiden Position 5 und 10 lokalisiert.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und translndenofluorenen (z. B. gemäß WO 2004/041901 oder WO 2004/113412), Ketonen (z. B. gemäß WO2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (1), bzw. (2) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1 995), 26(1-3), 127-36; Janssen, H. M.; Meijer, E. W., "The synthesis and characterization of dendritic molecules", Materials Science and Technology (1 999), 20 (Synthesis of Polymers), 403-458; Tomalia, Donald A., "Dendrimer molecules", Scientific American (1995), 272(5), 62-6; WO 2002/067343 A1 und WO 2005/026144 A1 .

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o , m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (1), bzw. (2), oder mindestens ein Polymer, Oligomer oder Dendrimer enthaltend mindestens eine Einheit gemäß Formel (1), bzw. (2), sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die Verbindungen gemäß Formel (1), bzw. (2) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und in unterschiedlichen Schichten der organischen Elektrolumineszenzvorrichtung eingesetzt. Bevorzugt werden die Verbindungen als Lochtransportmaterialien in einer Lochtransport- oder Lochinjektionsschicht, als Matrixmaterialien in einer emittierenden Schicht, als Elektronenblockiermaterialien, als Excitonenblockiermaterialien und/oder als Materialien für eine Zwischenschicht (interlayer) eingesetzt.

Weitere Gegenstände der Erfindung sind daher die Verwendung der Verbindungen gemäß Formel (1), bzw. (2) in elektronischen Vorrichtungen sowie elektronische Vorrichtungen selbst, welche eine oder mehrere Verbindungen gemäß Formel (1), bzw. (2) enthalten. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (OTFTs), organischen lichtemittierenden Transistoren (O-LETS), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt organischen Elektrolumineszenzvorrichtungen (OLEDs). Besonders bevorzugt sind organische Elektrolumineszenzvorrichtungen, enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (1), bzw. (2) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergangen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die organische Elektrolumineszenzvorrichtung kann auch mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, gelbes, grünes, orangefarbenes oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (1), bzw. (2) enthält und wobei die drei Schichten blaue, grüne und orangefarbene oder rote Emission zeigen (für den prinzipiellen Aufbau siehe z. B. WO 2005/011013). Alternativ und/oder zusätzlich können die erfindungsgemäßen Verbindungen auch in der Lochtransportschicht und/oder in einer Zwischenschicht (interlayer) vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (1), bzw. (2) in einer elektronischen Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht verwendet werden. Die Verbindung gemäß Formel (1), bzw. (2) kann aber auch erfindungsgemäß in einer elektronischen Vorrichtung enthaltend einen oder mehrere fluoreszierende Dotanden eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (1), bzw. (2) in organischen Elektrolumineszenzvorrichtungen einsetzen.

Explizite Beispiele für geeignete phosphoreszierende Emitterverbindungen können weiterhin einer im Folgenden abgebildeten Tabelle phosphoreszierender Dotanden entnommen werden.

In einer bevorzugten Ausführungsform der Erfindung werden die Verbindungen gemäß Formel (1), bzw. (2) als Lochtransportmaterial eingesetzt. Die Verbindungen werden dann bevorzugt in einer Lochtransportschicht und/oder in einer Lochinjektionsschicht eingesetzt.

Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Wenn die Verbindungen gemäß Formel (1), bzw. (2) als Lochtransportmaterial oder als Lochinjektionsmaterial verwendet werden, kann es bevorzugt sein, wenn sie mit Elektronenakzeptor-Verbindungen dotiert sind, beispielsweise mit F₄-TCNQ oder mit Verbindungen, wie sie in EP 1476881 oder EP 1596445 beschrieben werden. In einer weiteren bevorzugten Ausführungsform der Erfindung wird eine Verbindung gemäß Formel (1), bzw. (2) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Wird die Verbindung gemäß Formel (1), bzw. (2) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In einer weiteren Ausführungsform der vorliegenden Erfindung werden die Verbindungen der Formel (1), bzw. (2) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-System) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer bevorzugten Ausführungsform der Erfindung werden die Verbindungen gemäß Formel (1), bzw. (2) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Die beiden unterschiedlichen Matrixmaterialien können dabei in einem Verhältnis von 1 : 10 bis 1 : 1 vorhanden sein, bevorzugt in einem Verhältnis von 1 :4 bis 1 : 1.

Die Mixed-Matrix-Systeme können einen oder mehrere Dotanden umfassen. Die Dotandverbindung bzw. die Dotandverbindungen zusammen haben erfindungsgemäß einen Anteil von 0.1 bis 50.0 Vol.-% an der Gesamtmischung und bevorzugt einen Anteil von 0.5 bis 20.0 Vol.-% an der Gesamtmischung. Entsprechend haben die Matrixkomponenten zusammen einen Anteil von 50.0 bis 99.9 Vol-% an der Gesamtmischung und bevorzugt einen Anteil von 80.0 bis 99.5 Vol.-% an der Gesamtmischung.

Bevorzugt werden Mixed-Matrix-Systeme in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems eingesetzt werden können, sind aromatische Ketone, aromatische Phosphinoxide oder aromatische Sulfoxide oder Sulfone, z. B. gemäß WO 04/013080, WO 04/093207, WO 06/005627 oder WO 10/006680, Triarylamine, Carbazolderivate, z. B. CBP (N,N-Biscarbazolylbiphenyl) oder die in WO 05/039246, US 2005/0069729, JP 2004/288381, EP 1205527 oder WO 08/086851 offenbarten Carbazolderivate, Indolocarbazolderivate, z. B. gemäß WO 07/063754 oder WO 08/056746, Azacarbazolderivate, z. B. gemäß EP 1617710, EP 1617711, EP1731584, JP 2005/347160, bipolare Matrixmaterialien, z. B. gemäß WO 07/137725, Silane, z. B. gemäß WO 05/111172, Azaborole oder Boronester, z. B. gemäß WO 06/117052, Triazinderivate, z. B. gemäß der Anmeldung WO 2010/015306, WO 2007/063754 oder WO 2008/056746, Zinkkomplexe, z. B. gemäß EP 652273 oder WO 09/062578, Diazasilol- bzw. Tetraazasilol-Derivate, z. B. gemäß WO 10/054729, Diazaphosphol-Derivate, z. B. gemäß WO 10/054730, oder Indenocarbazolderivate, z. B. gemäß WO 10/136109.

Bevorzugte phosphoreszierende Dotanden zur Verwendung in Mixed-Matrix-Systemen enthaltend die erfindungsgemäßen Verbindungen sind die in einer folgenden Tabelle aufgeführten phosphoreszierenden Dotanden.

Gemäß einer weiteren bevorzugten Ausführungsform der Erfindung werden die Verbindungen gemäß Formel (1), bzw. (2) in einer Zwischenschicht (interlayer) eingesetzt. Zwischenschichten werden bevorzugt in organischen Elektrolumineszenzvorrichtungen enthaltend mehrere emittierende Schichten eingesetzt, beispielsweise in weiß emittierenden OLEDs, welche jeweils eine rot emittierende, eine grün emittierende und eine blau emittierende Schicht enthalten. Besonders bevorzugt sind Zwischenschichten zwischen zwei emittierenden Schichten angeordnet. Eine Zwischenschicht enthaltend eine erfindungsgemäße Verbindung ist gemäß einer bevorzugten Ausführungsform der Erfindung zwischen der blau emittierenden Schicht und der grün emittierenden Schicht einer weißes Licht emittierenden OLED, welche eine rot emittierende, eine grün emittierende und eine blau emittierende Schicht enthält, angeordnet. Besonders bevorzugt ist die blau emittierende Schicht dabei eine fluoreszierende Schicht, und die grün emittierende Schicht ist eine phosphoreszierende Schicht.

Die in der folgenden Tabelle aufgeführten Verbindungen stellen besonders geeignete phosphoreszierende Dotanden dar.

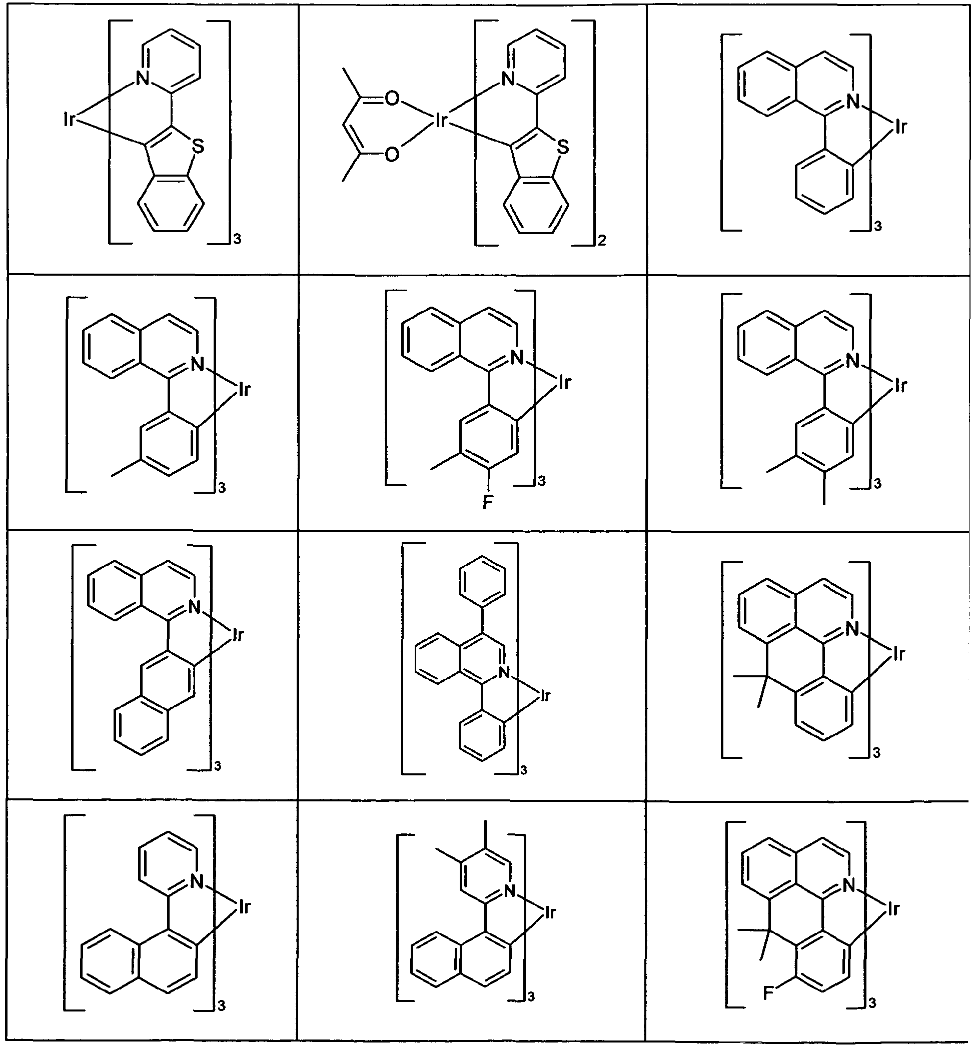

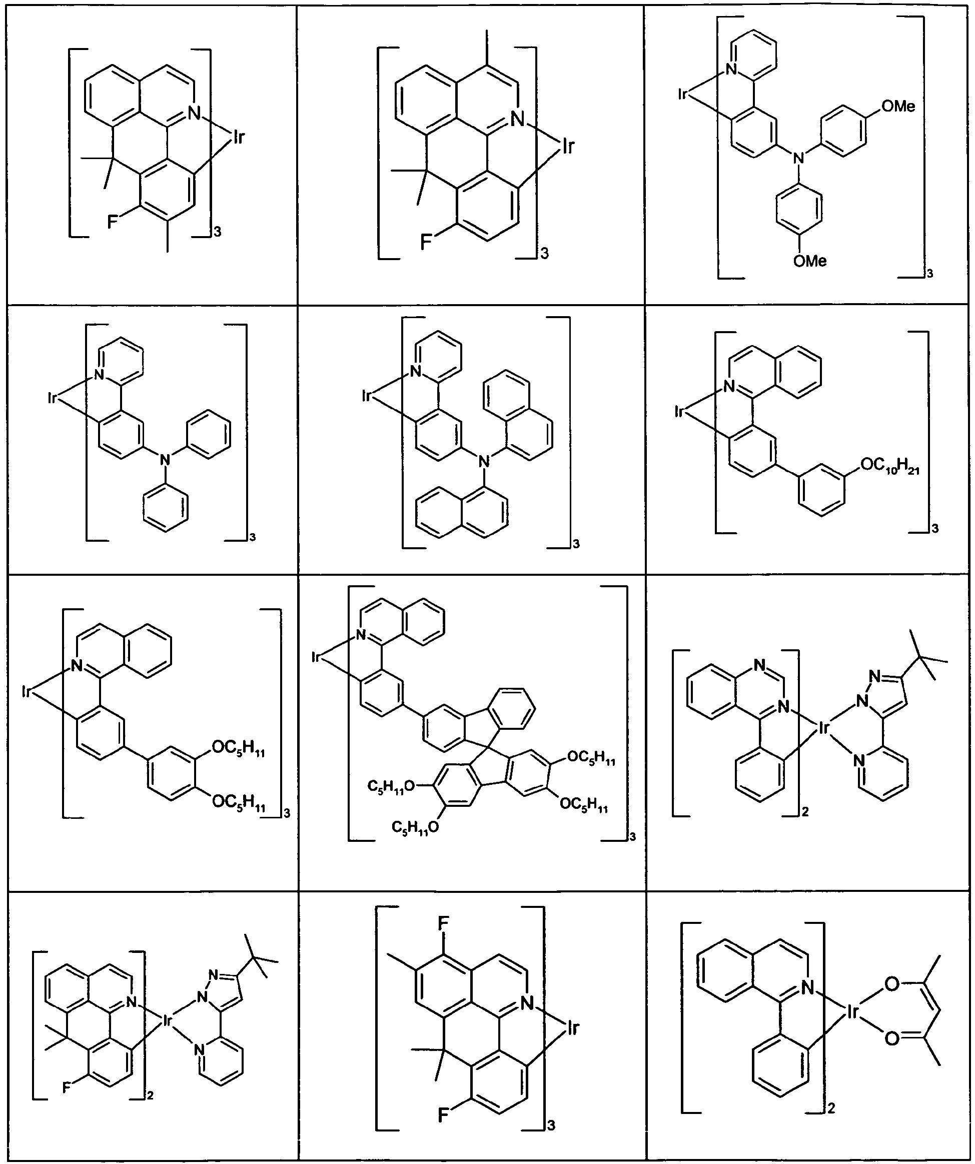

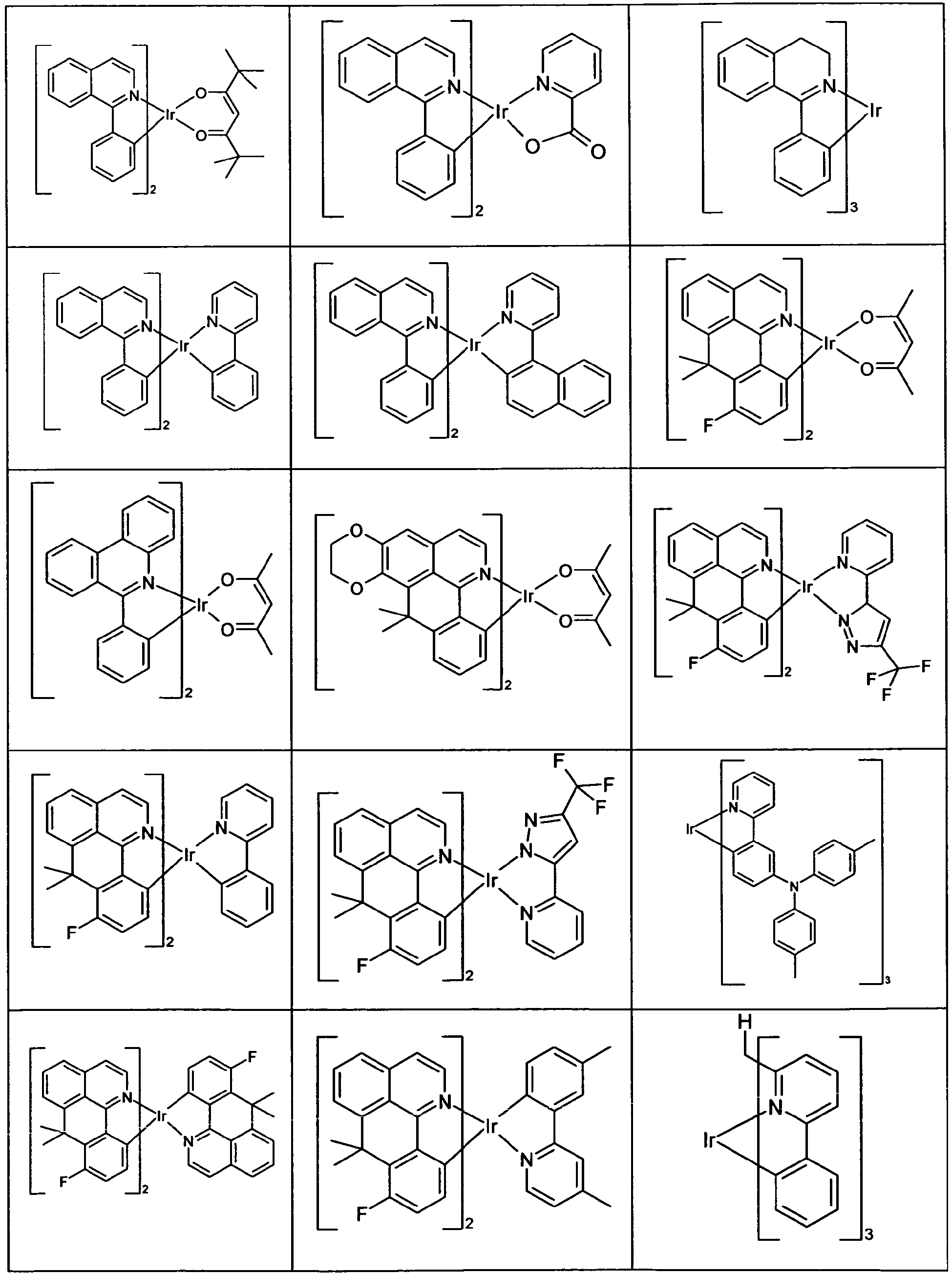

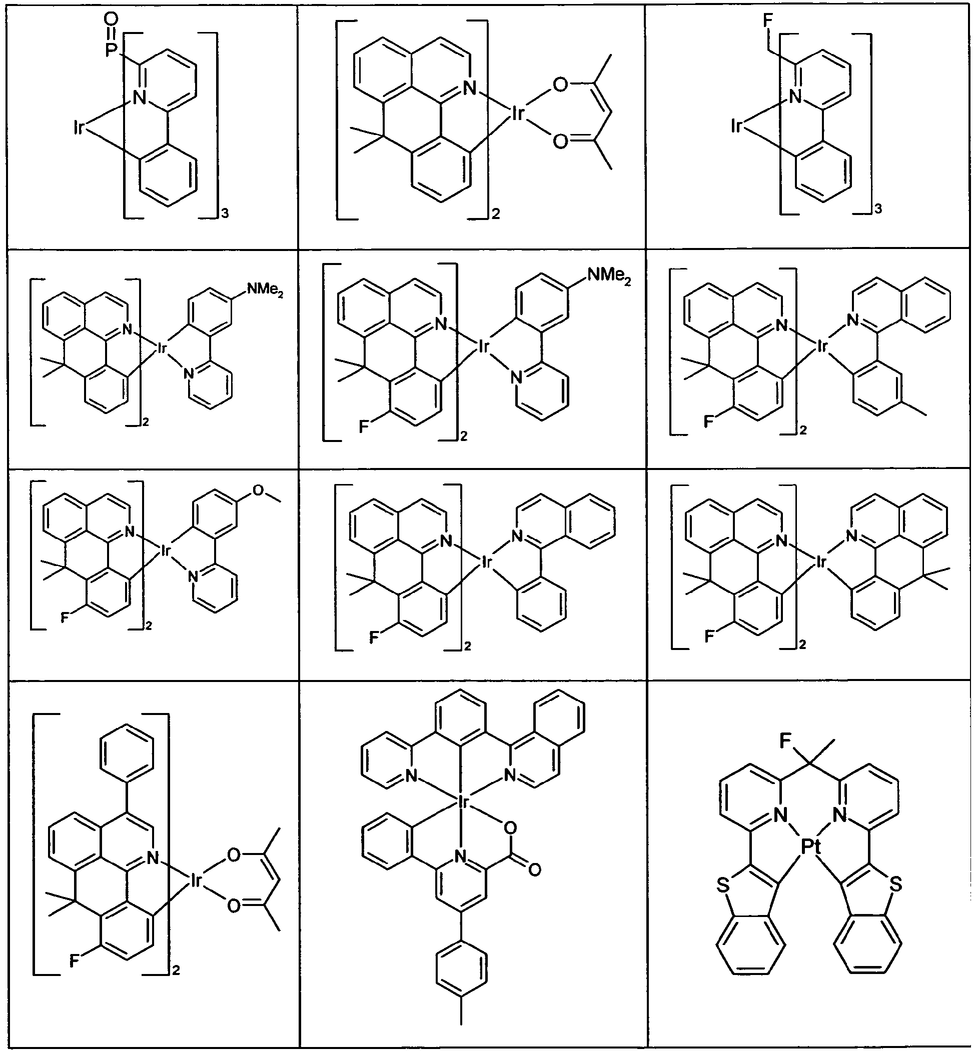

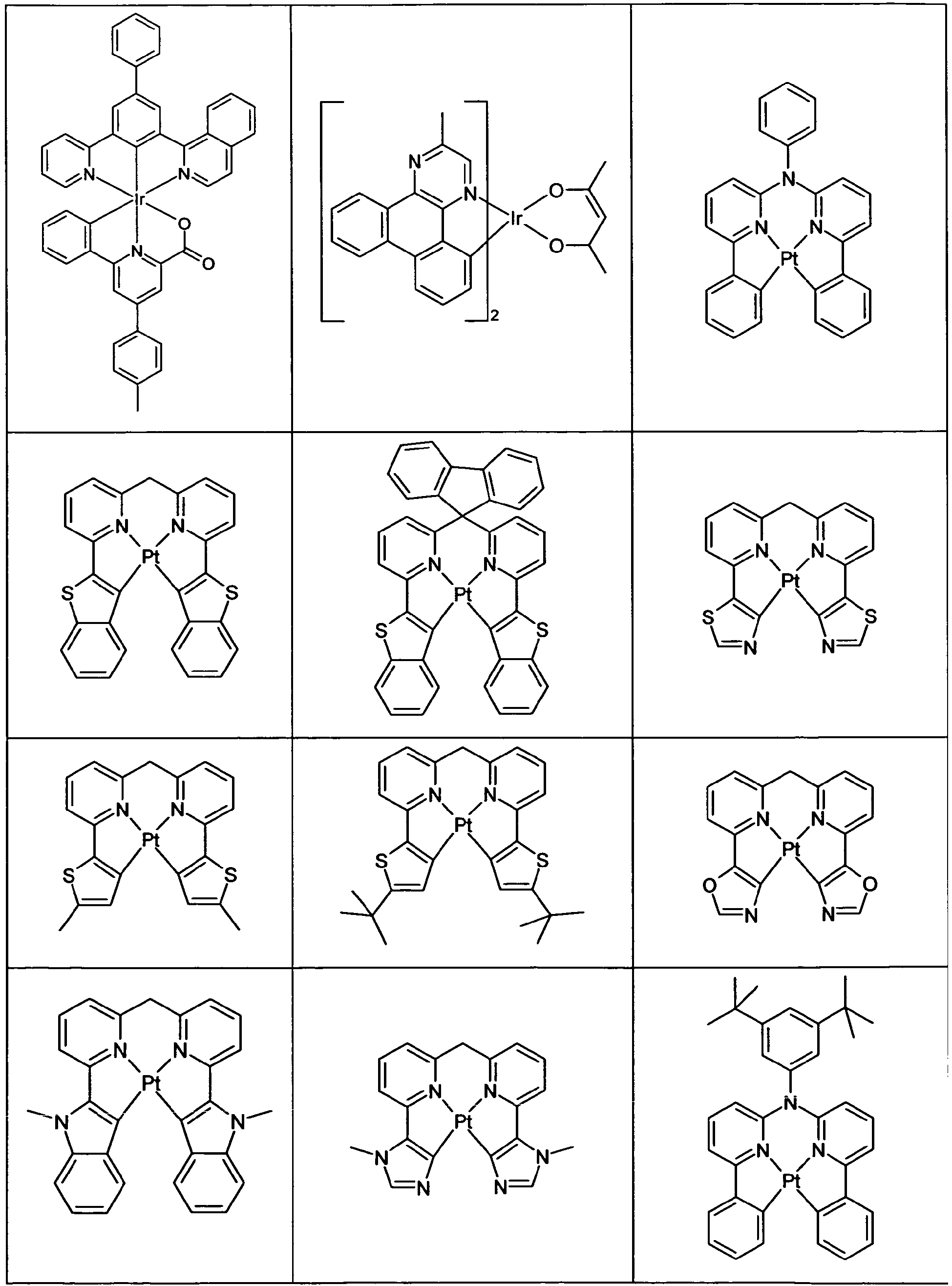

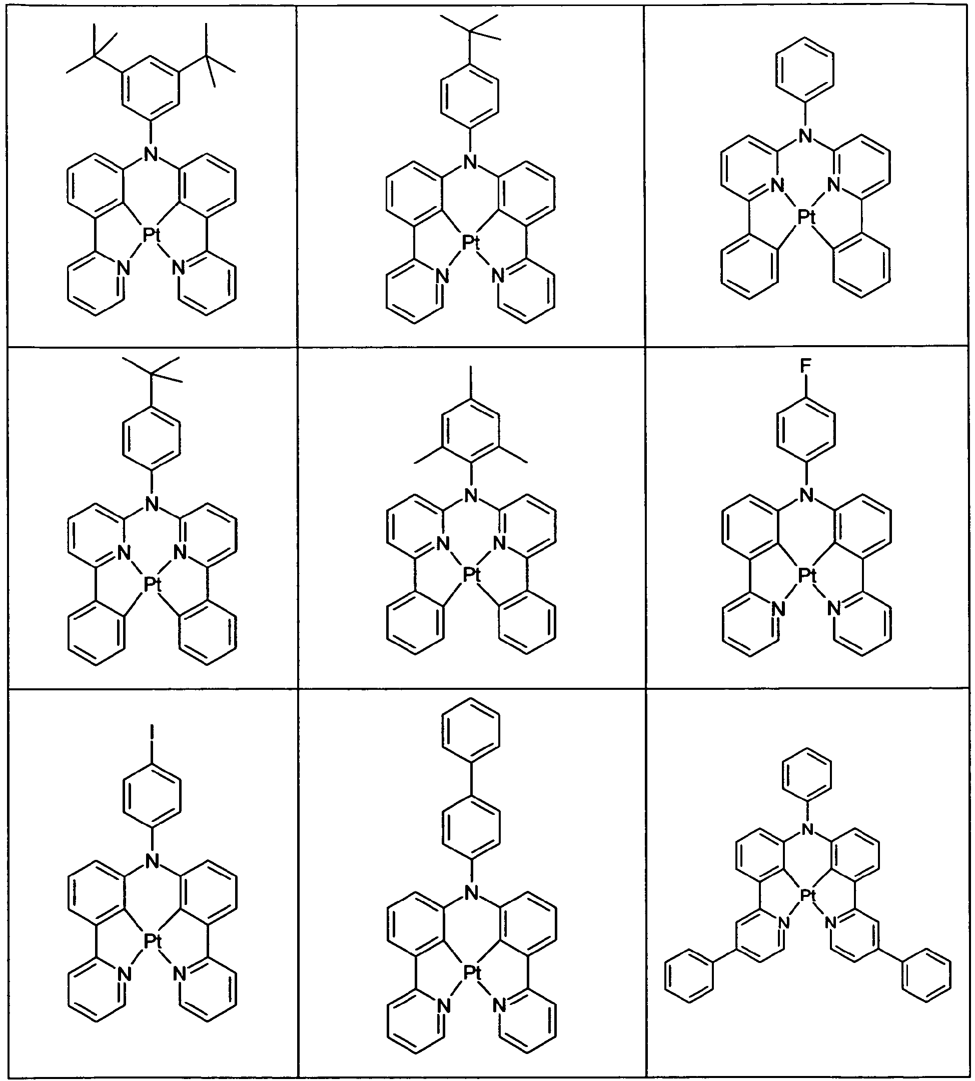

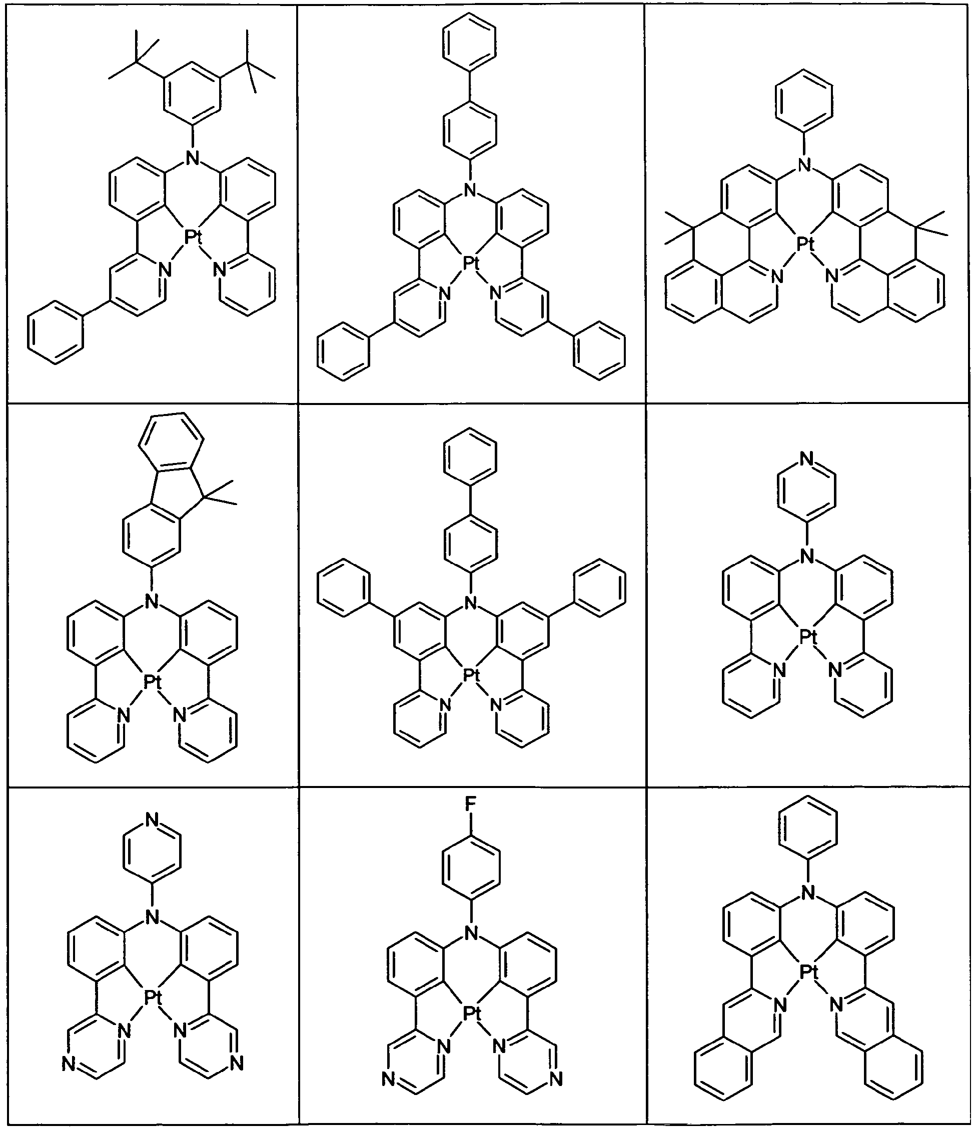

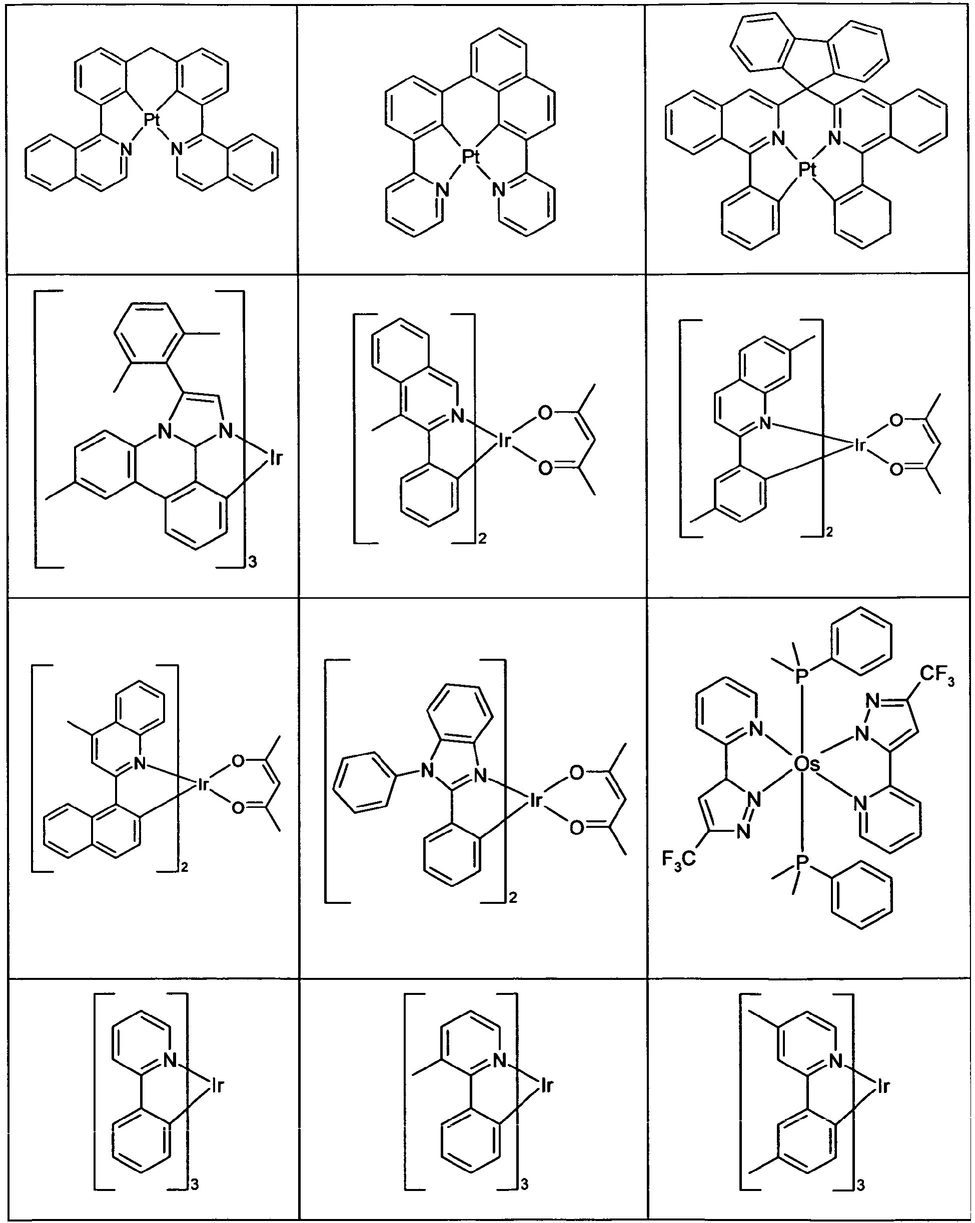

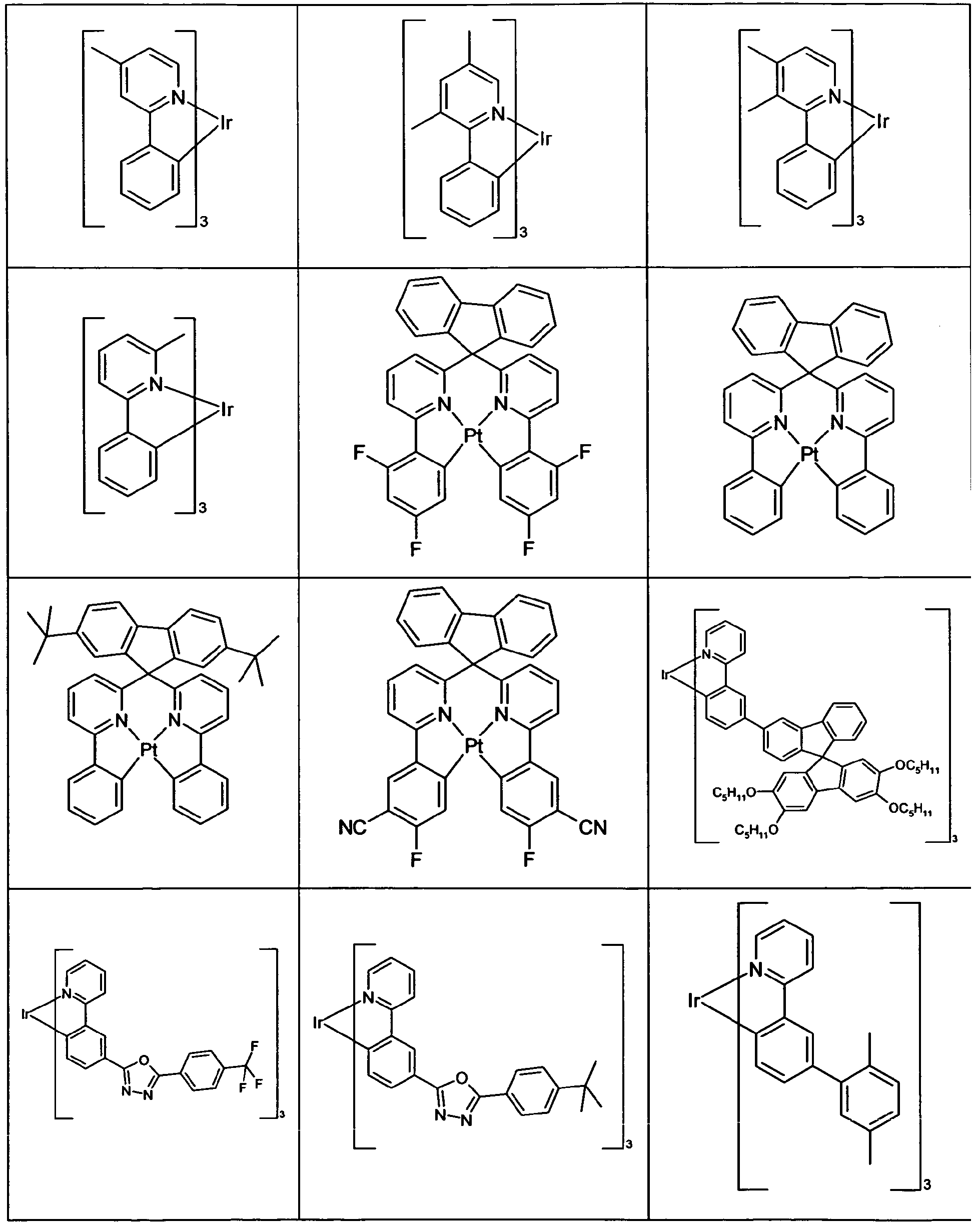

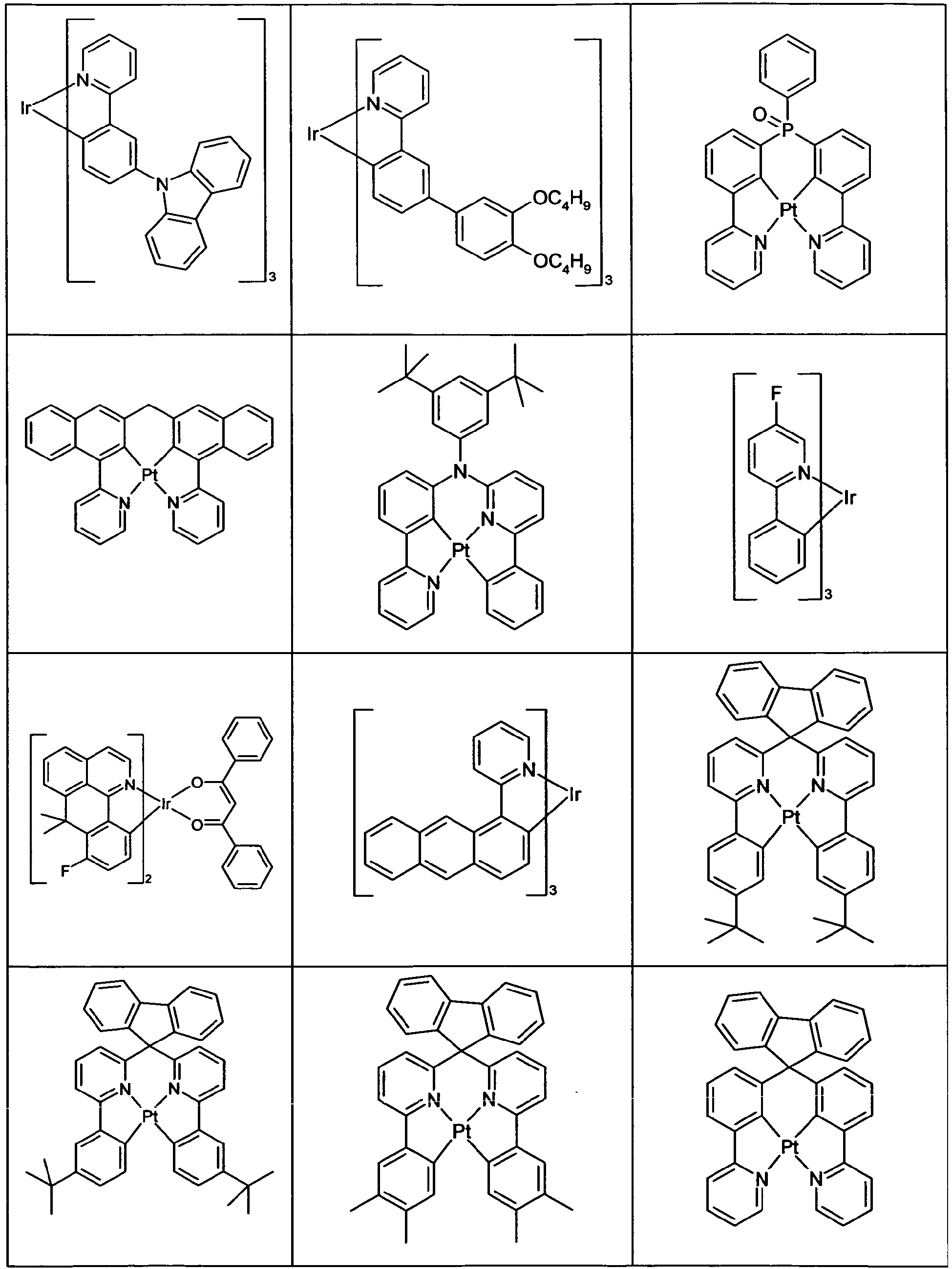

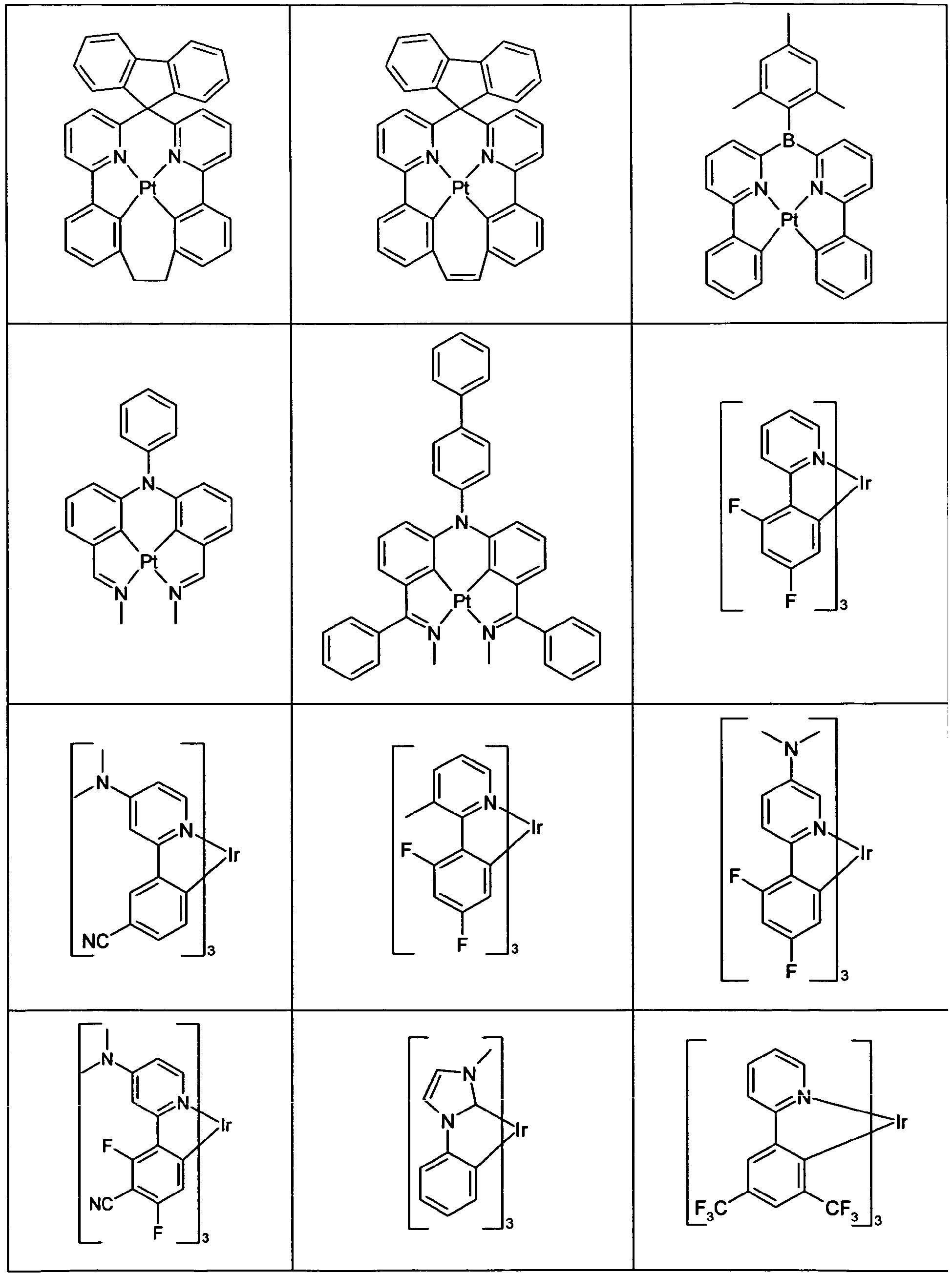

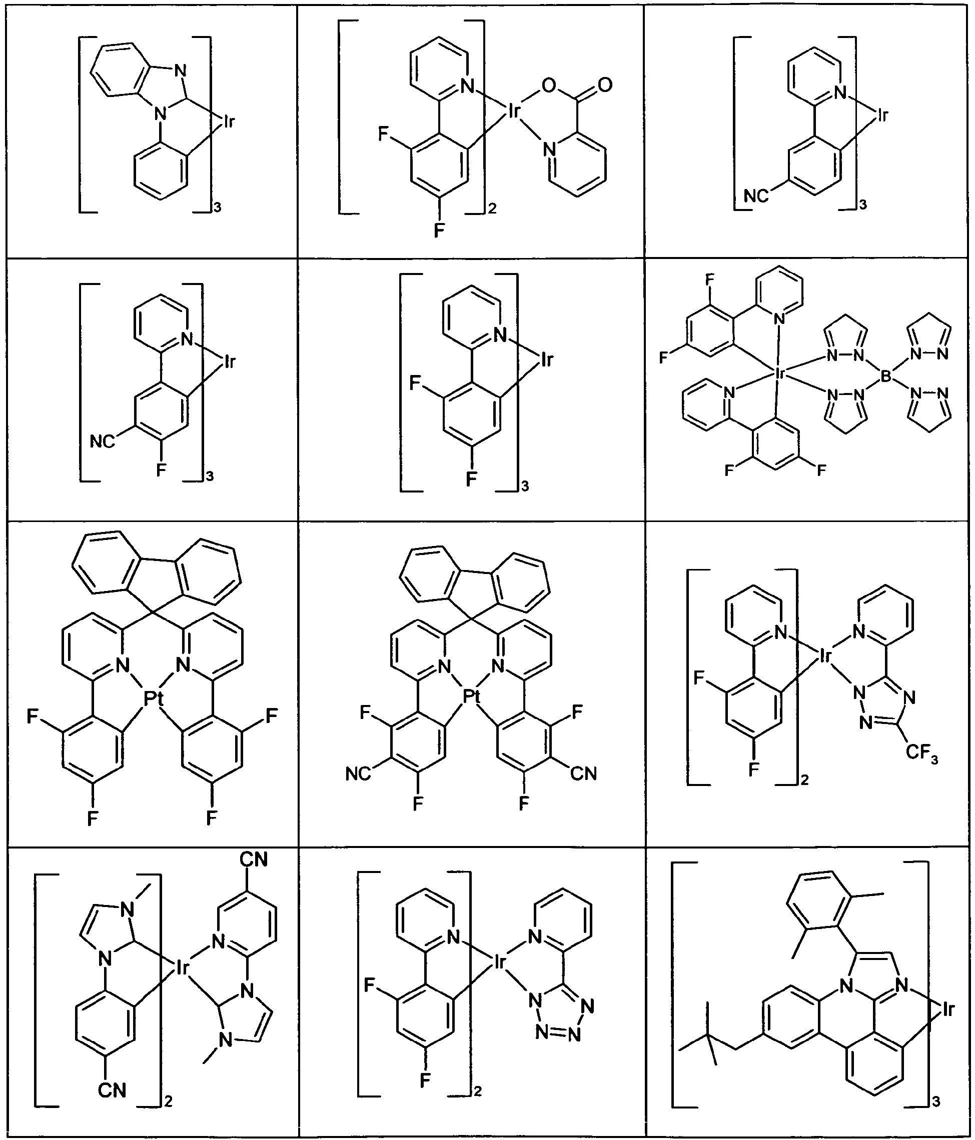

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Weiterhin bevorzugt sind die in WO 2010/012328 offenbarten kondensierten Kohlenwasserstoffe.

Geeignete fluoreszierende Dotanden sind weiterhin die in JP 2006100 1973, WO 2004/047499, WO 2006/098080, WO 2007/065678, US 2005/0260442 und WO 2004/092111 offenbarten Derivate dieser Strukturen.

Als Matrixmaterialien, bevorzugt für fluoreszierende Dotanden, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen Carbazolderivate (z. B. CBP (N,N-Biscarbazolylbiphenyl) oder Verbindungen gemäß WO 2005/039246, US 2005/0069729, JP 2004/288381, EP 1205527 oder WO 2008/086851), Triarylamine, Azacarbazole (z. B. gemäß EP 1617710, EP 1617711, EP 1731584, JP 2005/347160), Indolocarbazolderivate, z. B. gemäß WO 2007/063754 oder WO 2008/056746, Ketone (z. B. gemäß WO 2004/093207 oder WO 2010/006680), Phosphinoxide, Sulfoxide und Sulfone (z. B. gemäß WO 2005/003253), Oligophenylene, aromatische Amine (z. B. gemäß US 2005/0069729), bipolare Matrixmaterialien (z. B. gemäß WO 2007/137725), Silane (z. B. gemäß WO 2005/111172), Azaborole oder Boronester, z. B. gemäß WO 2006/117052, Triazinderivate, z. B. gemäß WO 201 0/015306, WO 2007/063754 oder WO 2008/056746, Zinkkomplexe (z. B. gemäß WO 20091062578), Aluminiumkomplexe (z. B. BAlq), Diazasilol- und Tetraazasilol-Derivate, z. B. gemäß WO 2010/054730, Indenocarbazolderivate, z. B. gemäß WO 2010/136109 und WO 2011/000455 oder Diazaphosphole, z. B. gemäß WO 2010/054730.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev, 2007, 107(4), 953-1010 offenbarten Verbindungen oder andere Materialien, wie sie gemäß dem Stand der Technik in diesen Schichten eingesetzt werden.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Ba/Ag oder Mg/Ag verwendet werden. Es kann auch bevorzugt sein, zwischen einer metallischen Kathode und dem organischen Halbleiter eine dünne Zwischenschicht eines Materials mit einer hohen Dielektrizitatskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et a/. ,Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1), bzw. (2) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Die organischen Elektrolumineszenzvorrichtungen enthaltend eine oder mehrere erfindungsgemäße Verbindungen können in Displays, als Lichtquellen in Beleuchtungsanwendungen sowie als Lichtquellen in medizinischen und/oder kosmetischen Anwendungen (z.B. Lichttherapie) eingesetzt werden.

Die erfindungsgemäßen Verbindungen zeichnen sich insbesondere dadurch aus, dass sie bei Verwendung in organischen Elektrolumineszenzvorrichtungen gute Leistungseffizienzen, geringe Betriebsspannungen und lange Lebensdauern der Vorrichtungen bewirken. Weiterhin sind die Verbindungen oxidationsstabil, temperaturstabil und weisen eine hohe Glasübergangstemperatur auf, was sowohl für die Prozessierbarkeit, beispielsweise aus Lösung oder aus der Gasphase, als auch für die Verwendung in elektronischen Vorrichtungen vorteilhaft ist.

Weiterhin weisen die Verbindungen eine hohe Lochbeweglichkeit auf, was insbesondere bei der Verwendung als Lochtransportmaterial bzw. Lochinjektionsmaterial hoch erwünscht ist.

Die Erfindung wird durch die nachfolgenden Anwendungsbeispiele näher erläutert, wobei die Erfindung nicht auf den Umfang der Beispiele beschränkt ist.

### Ausführungsbeispiele

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden.

### Beispiel 1: 9-(2-Bromo-phenyl)-9H-fluoren-9-ol

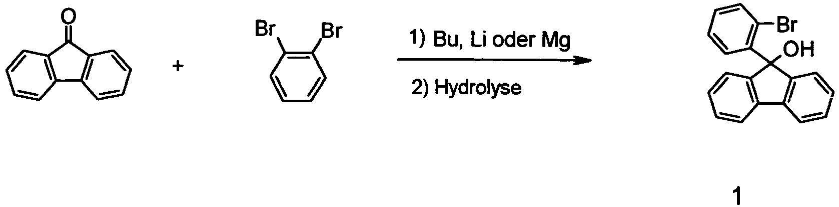

Aus mit Jod aktivierten 2.68 g (110 mmol) Magnesiumspänen und einer Mischung aus 25.9 g (110 mmol) 1,2-Dibrombenzen, 0.8 ml 1,2-Dichlorethan, 50 ml 1,2-Dimethoxyethan, 500 ml THF wird unter Begleitheizen mit einem 70 °C warmen Ölbad das entsprechende Grignard-Reagenz dargestellt. Nachdem das Magnesium vollständig abreagiert hat, lässt man auf Raumtemperatur erkalten und tropft dann eine Lösung von 18.0 g (100 mmol) Fluorenon, [486-25-9] in 300 ml THF zu, erwärmt die Reaktionsmischung für 4 h auf 50 °C und rührt dann 12 h bei Raumtemperatur nach. Man gibt 100 ml Wasser zu, rührt kurz nach, trennt die org. Phase ab und entfernt das Lösungsmittel im Vakuum. Anschließend wird 2 h bei 80 °C mit 500 mL Heptan nachgerührt. Nach Erkalten saugt man vom ausgefallenen Feststoff ab, wäscht diesen einmal mit 100 ml Heptan, zweimal mit je 100 ml Ethanol und kristallisiert abschließend aus Dioxan/EtOH um. Ausbeute: 24.6 g (73 mmol), 73%; Reinheit ca. 98 % n. ¹H-NMR.

Analog werden folgende Verbindungen erhalten:

| | Edukt 1 | Edukt 2 | Produkt | Ausbeute |
|---|---|---|---|---|
| 1a | 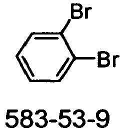 | 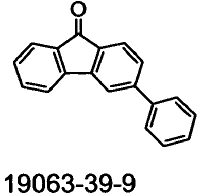 | 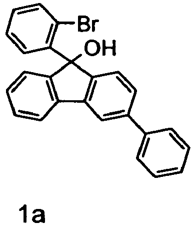 | 85% |
| 1b | 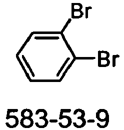 | 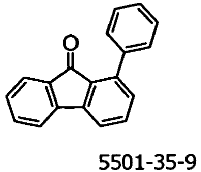 | 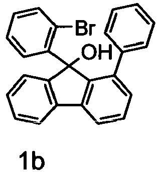 | 70% |
| 1c | 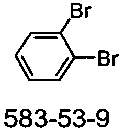 | 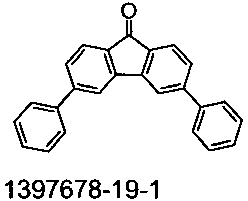 | 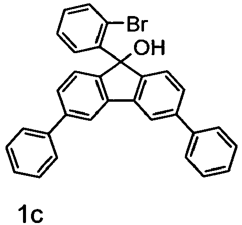 | 85% |
| 1d | 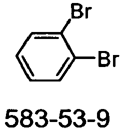 | 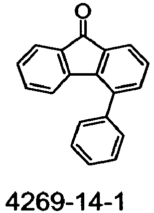 | 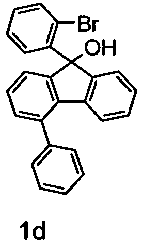 | 83% |
| 1e | 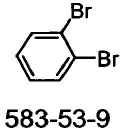 | 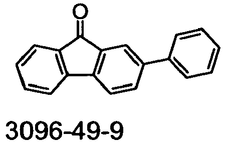 | 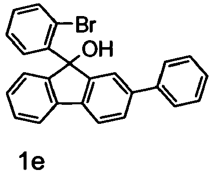 | 81 % |
| 1f | 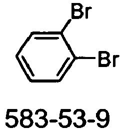 | 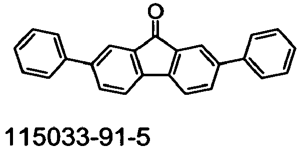 | 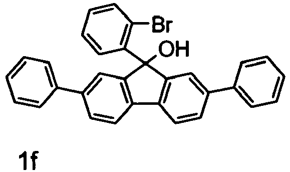 | 74% |
| 1g | 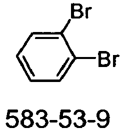 | 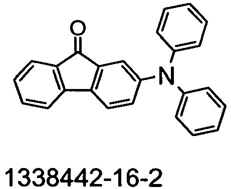 | 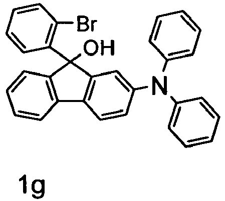 | 81% |
| 1h | 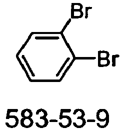 | 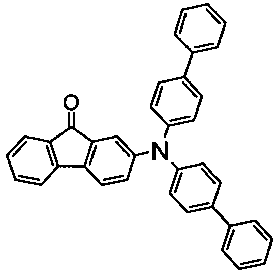 | 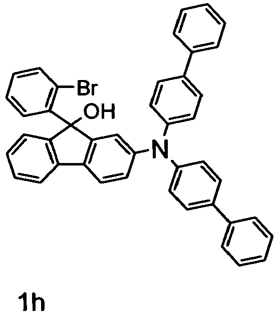 | 76% |
| 1i | 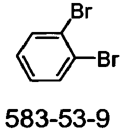 | 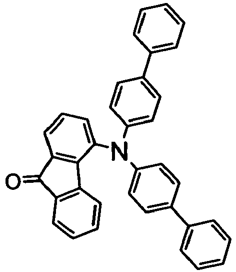 | 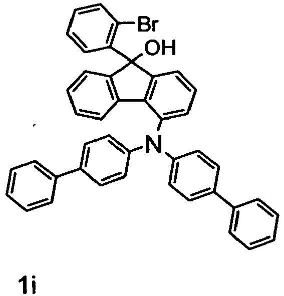 | 74% |
| 1j | 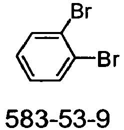 | 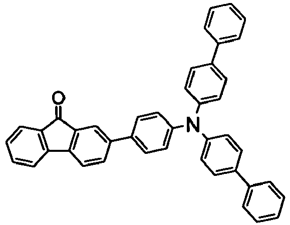 | 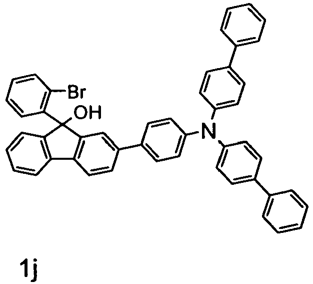 | 72% |

### Beispiel 2: Bis-biphenyl-4-yl-{4-[9-(2-brom-phenyl)-9H-fluoren-9-yl]-phenyl}-amin

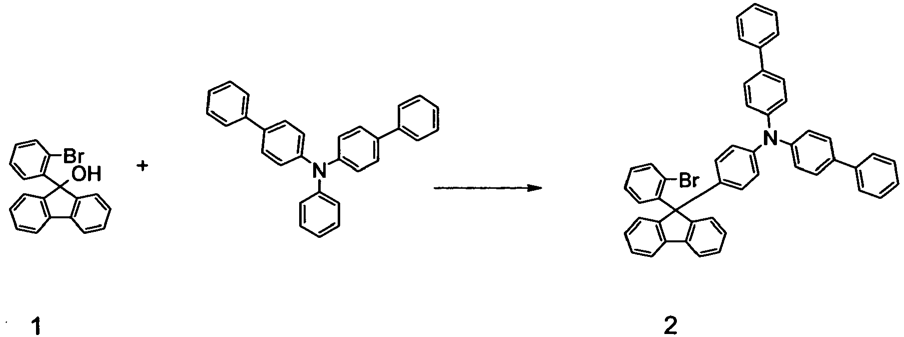

Ein Gemisch aus 16.85 g (50 mmol) 9-(2-Bromo-phenyl)-9H-fluoren-9-ol und 19.88 g (50 mmol) Bis-biphenyl-4-yl-phenyl-amin [122215-84-3], Trifluormethansulfonsäure [1493-13-6] 15 g (100 mmol, 8.8 mL) und 300 ml Dioxan wird 24 h unter Rückfluss erhitzt. Nach Erkalten versetzt man mit 200 ml Wasser, rührt 30 min. nach, trennt die org. Phase ab, filtriert diese über ein kurzes Celite-Bett und entfernt dann das Lösungsmittel im Vakuum. Der Rückstand wird zweimal aus Toluol/Heptan umkristallisiert. Ausbeute: 26.1 g (36.5 mmol), 73%; Reinheit ca. 98 % n. ¹H-NMR.

Analog werden folgende Verbindungen erhalten:

| Bsp. | Edukt 1 | Edukt 2 | Produkt: | Ausbeute |
|---|---|---|---|---|
| 2a | 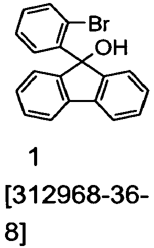 | 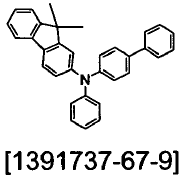 | 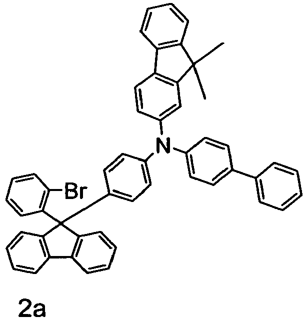 | 85% |
| 2b | 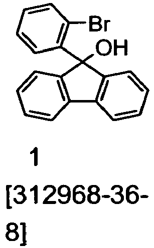 | 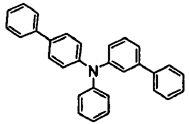 | 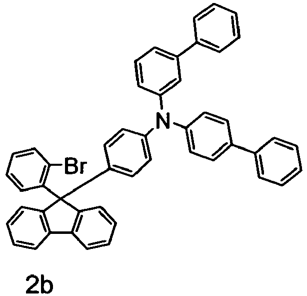 | 74% |
| 2c | 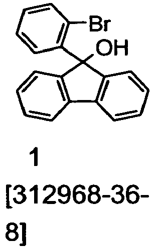 | 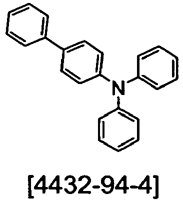 | 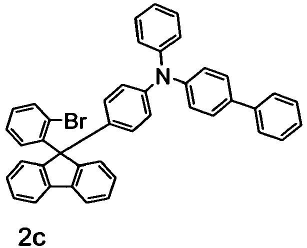 | 70% |
| 2e | 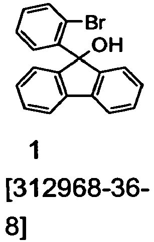 | 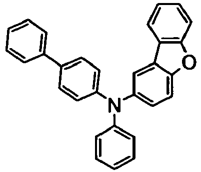 | 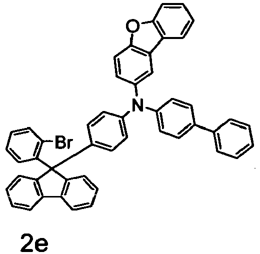 | 62% |
| 2f | 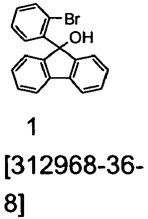 | 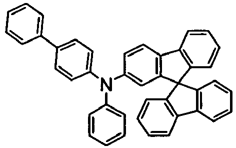 | 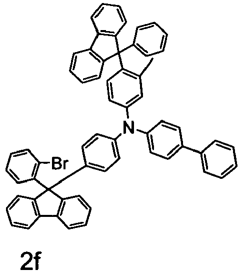 | 58% |
| 2g | 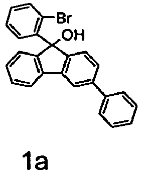 | 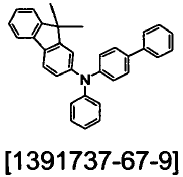 | 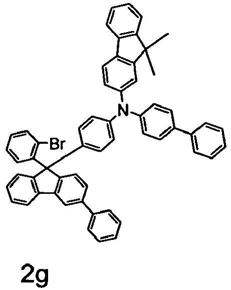 | 85% |
| 2h | 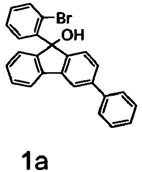 | 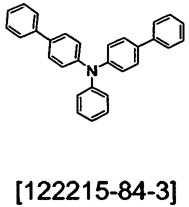 | 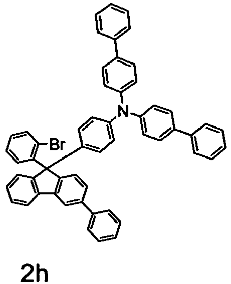 | 79% |
| 2i | 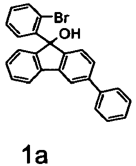 | 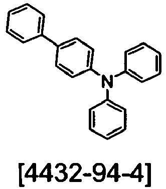 | 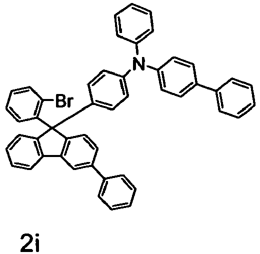 | 78% |
| 2j | 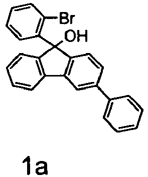 | 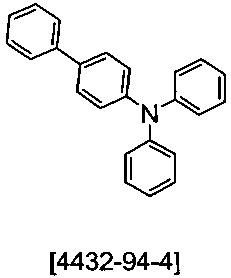 | 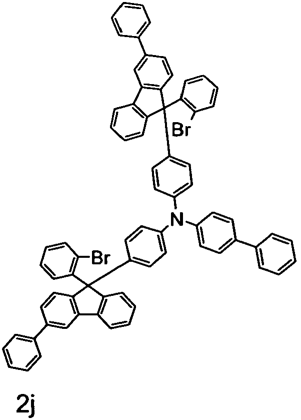 | 68% |
| 2k | 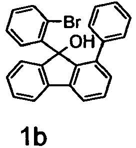 | 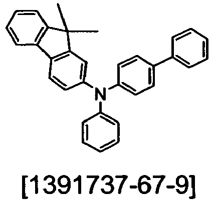 | 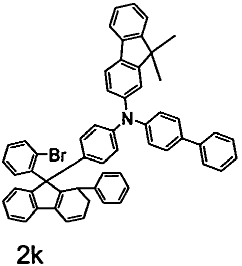 | 45% |
| 2l | 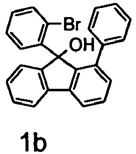 | 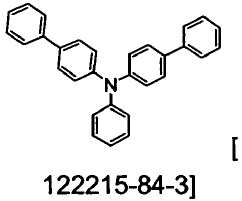 | 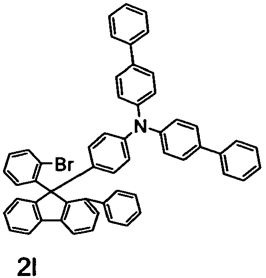 | 41 % |
| 2m | 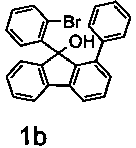 | 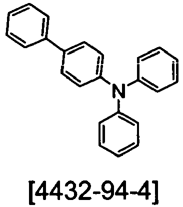 | 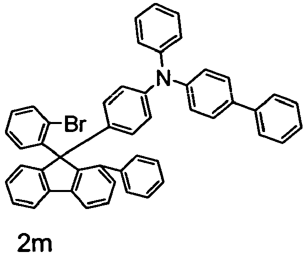 | 48% |
| 2n | 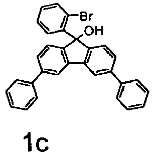 | 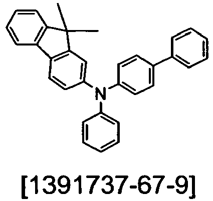 | 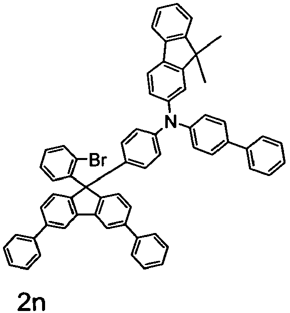 | 58% |
| 2o | 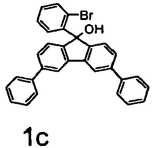 | 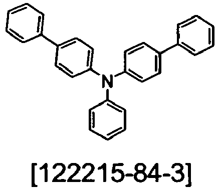 | 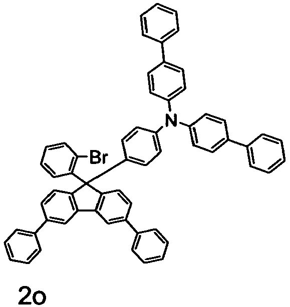 | 70% |
| 2p | 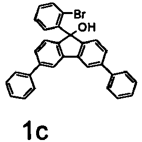 | 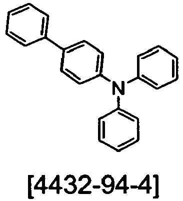 | 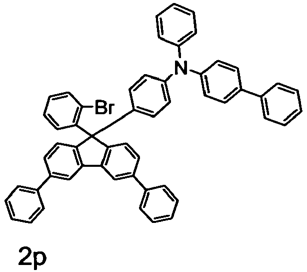 | 62% |
| 2r | 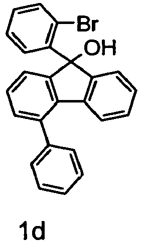 | 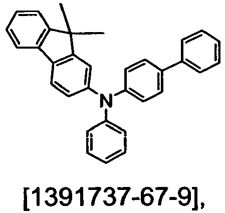 | 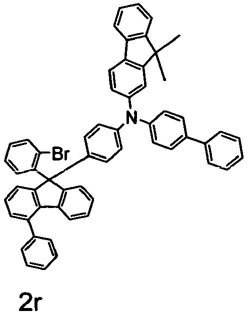 | 58% |
| 2s | 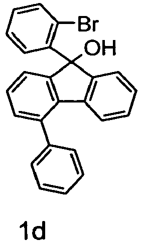 | 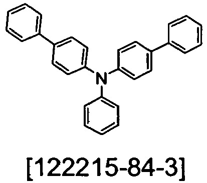 | 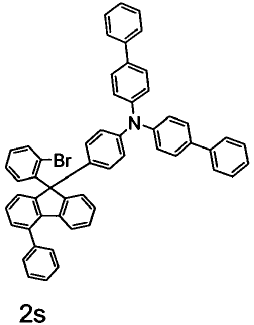 | 70% |
| 2t | 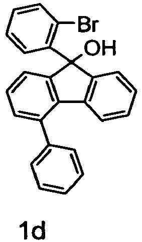 | 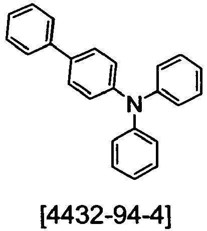 | 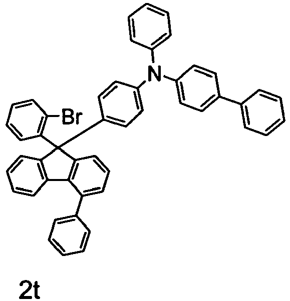 | 78% |
| 2u | 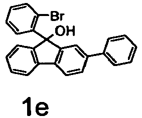 | 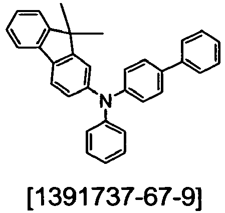 | 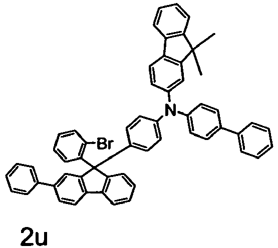 | 82% |
| 2v | 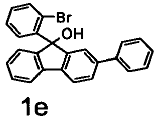 | 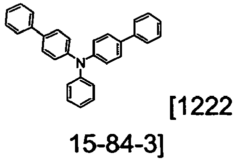 | 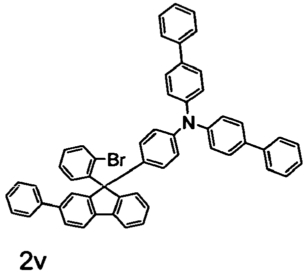 | 70% |
| 2w | 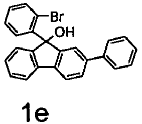 | 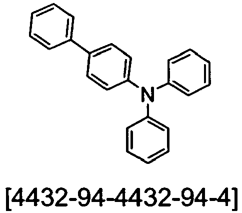 | 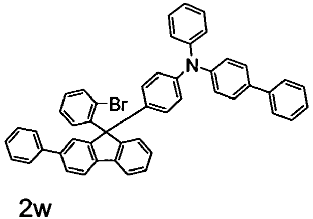 | 78% |
| 2x | 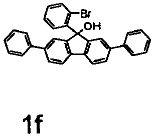 | 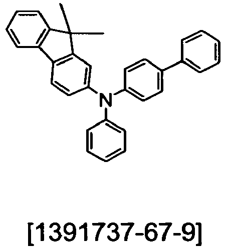 | 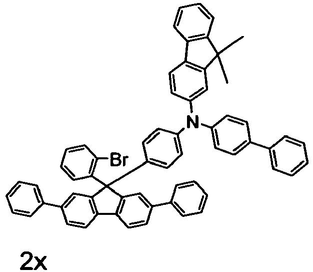 | 82% |
| 2y | 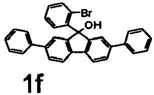 | 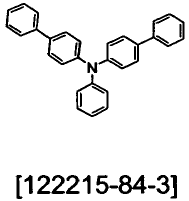 | 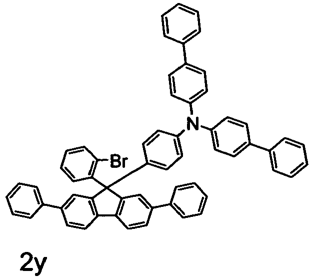 | 70% |
| 2z | 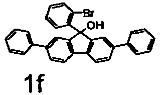 | 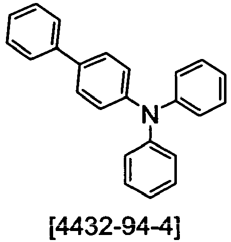 | 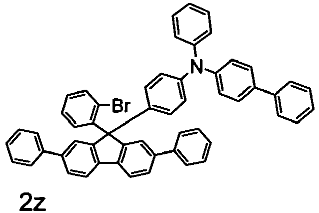 | 78% |
| 2aa | 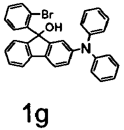 | 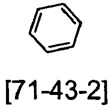 | 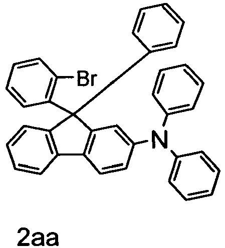 | 61% |
| 2bb | 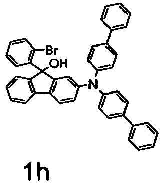 | 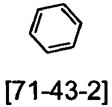 | 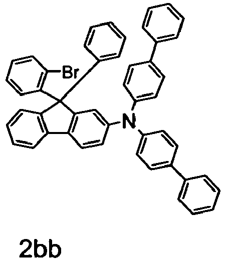 | 56% |
| 2cc | 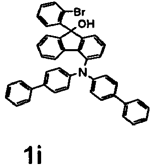 | 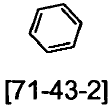 | 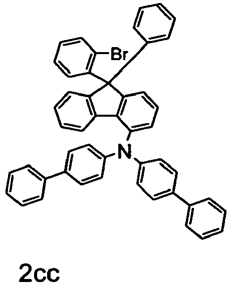 | 64% |
| 2dd | 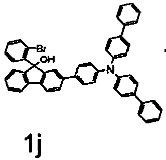 | 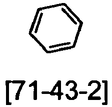 | 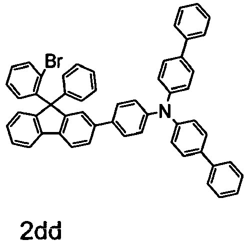 | 42% |
| 2ee | 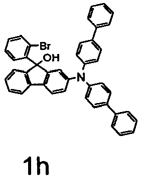 | 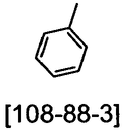 | 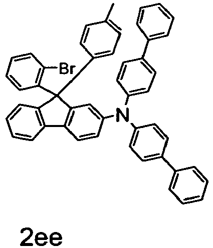 | 56% |

### Beispiel 3: 4-Biphenyl-2-(9,9'-dimethylfluorenyl)-amin- 3-cyano-benzen

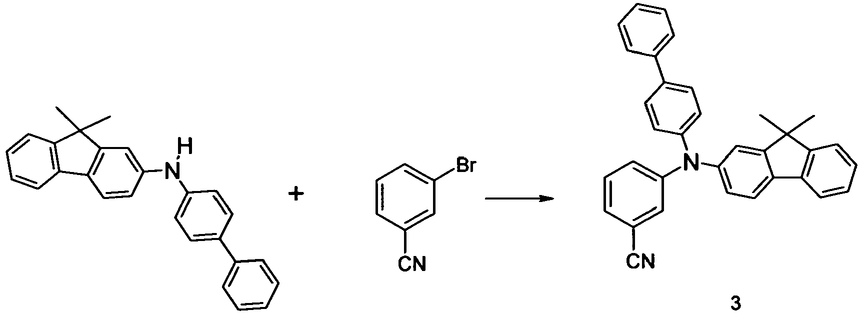

Ein Gemisch aus 9.1 g (50 mmol) 1-Brom-3-cyano-benzen, 21.7 g (60 mmol) 4-Biphenyl-2-(9,9'-dimethylfluorenyl)-amin, [897671-69-1], 7.7 g (80 mmol) Natrium-tert-butylat, 1.4 g (5 mmol) Tricyclohexylamin, 561 mg (2.5 mmol) Palladium(II)acetat und 300 ml Mesitylen wird 24 h unter Rückfluss erhitzt. Nach Erkalten versetzt man mit 200 ml Wasser, rührt 30 min. nach, trennt die org. Phase ab, filtriert diese über ein kurzes Celite-Bett und entfernt dann das Lösungsmittel im Vakuum. Der Rückstand wird fünfmal aus Heptan umkristallisiert . Ausbeute: 16.8 g (36.5 mmol), 73%; Reinheit ca. 98 % n. ¹H-NMR.

Analog werden folgende Verbindungen erhalten:

| Bsp. | Edukt 1 | Edukt 2 | Produkt | Ausbeute |
|---|---|---|---|---|
| 3a | 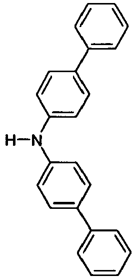 | 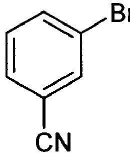 | 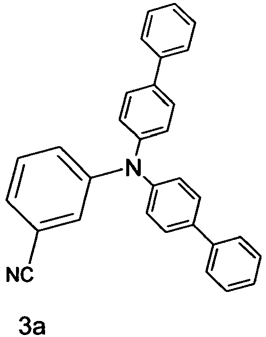 | 43 % |
| | [102113-98-4] | [6952-59-6] | | |
| 3b | 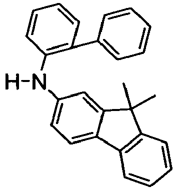 | 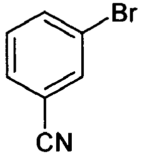 | 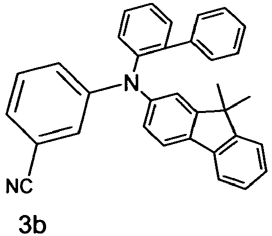 | 56 % |
| | [1198395-24-2] | [6952-59-6] | | |
| 3c | 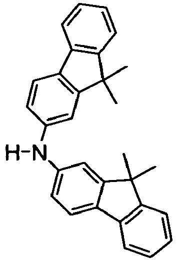 | 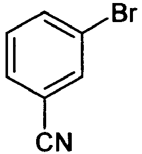 | 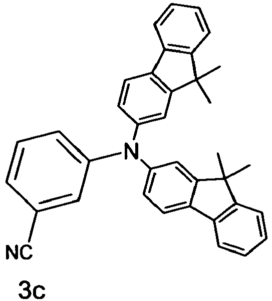 | 45 % |
| | [500717-23-7] | [6952-59-6] | | |
| 3d | 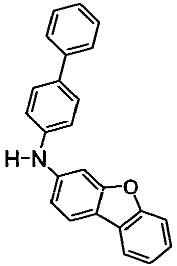 | 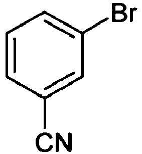 | 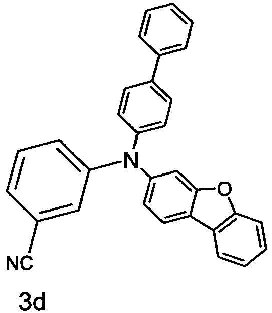 | 40 % |
| | [1290039-85-8] | [6952-59-6] | | |
| 3e | 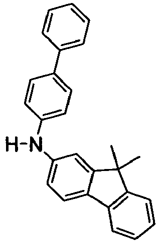 | 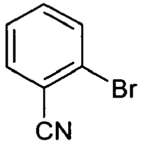 | 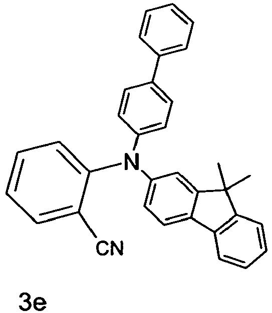 | 35% |
| | [897671-69-1] | [2042-37-7] | | |
| 3f | 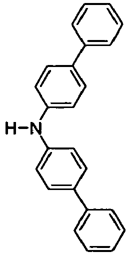 | 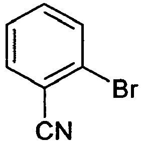 | 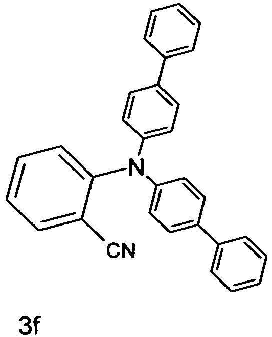 | 43 % |
| | [102113-98-4] | [2042-37-7] | | |
| 3g | 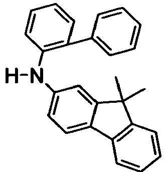 | 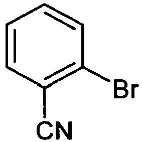 | 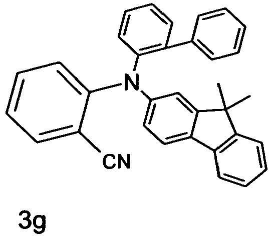 | 56 % |
| | [11983985-24-2] | [2042-37-7] | | |
| 3h | 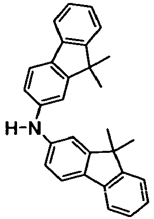 | 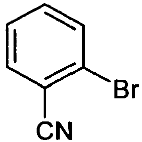 | 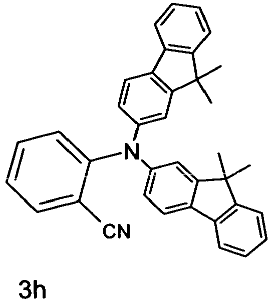 | 45 % |
| | [500717-23-7] | [2042-37-7] | | |
| 3i | 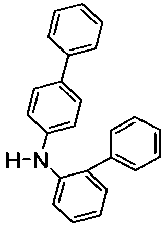 | 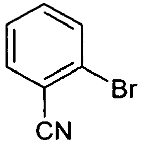 | 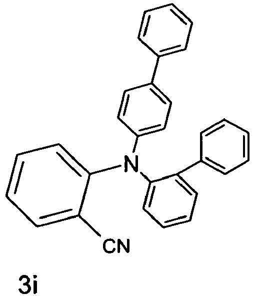 | 40 % |
| | | [2042-37-7] | | |

### Beispiel 4: {3-[Biphenyl-4-yl-(9,9-dimethyl-9H-fluoren-2-yl)-amino]-phenyl}-(2-bromo-phenyl)-methanon

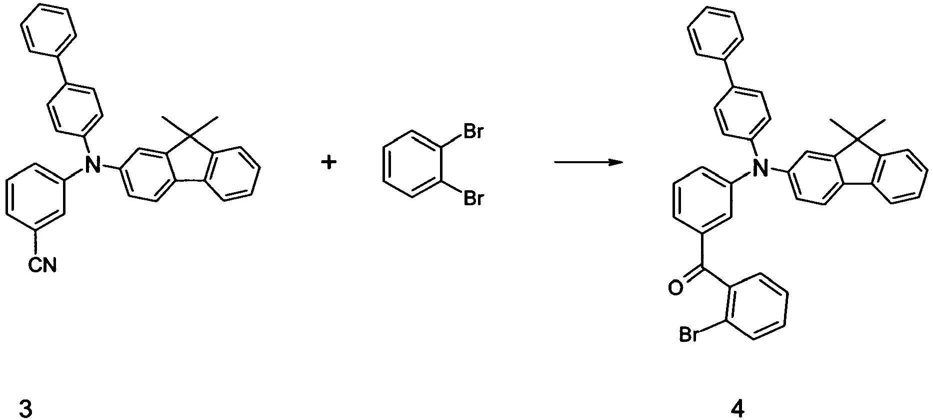

Aus mit Jod aktivierten 2.68 g (110 mmol) Magnesiumspänen und einer Mischung aus 25.9 g (110 mmol) 1,2-Dibrombenzen, 0.8 ml 1,2-Dichlorethan, 50 ml 1,2-Dimethoxyethan, 500 ml THF wird unter Begleitheizen mit einem 70 °C warmen Ölbad das entsprechende Grignard-Reagenz dargestellt. Nachdem das Magnesium vollständig abreagiert hat, lässt man auf Raumtemperatur erkalten und tropft dann eine Lösung von 46.2 g (100 mmol) 4-Biphenyl-2-(9,9'-dimethylfluorenyl)-amin-3-cyano-benzen in 300 ml THF zu, erwärmt die Reaktionsmischung für 4 h auf 50 °C und rührt dann 12 h bei Raumtemperatur nach. Man gibt 100 ml Wasser zu, rührt kurz nach, trennt die org. Phase ab und entfernt das Lösungsmittel im Vakuum. Anschließend wird 6 h bei 80 °C mit 500 mL MNP und 5 mL Essigsäure nachgerührt. Nach Erkalten saugt man vom ausgefallenen Feststoff ab, wäscht diesen einmal mit 100 ml Heptan, zweimal mit je 100 ml Ethanol und kristallisiert abschließend aus Dioxan/EtOH um. Ausbeute: 26.6 g (43 mmol), 39%; Reinheit ca. 98 % n. ¹H-NMR.

Analog werden folgende Verbindungen erhalten:

| Bsp. | Edukt 1 | Produkt | Ausbeute |
|---|---|---|---|
| 4a | 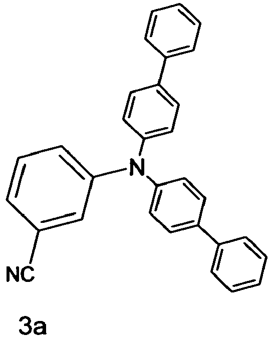 | 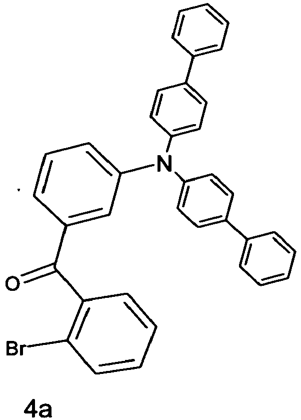 | 43 % |
| 4b | 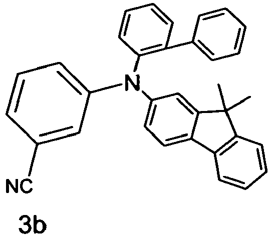 | 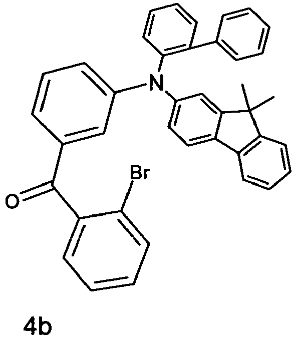 | 46 % |
| 4c | 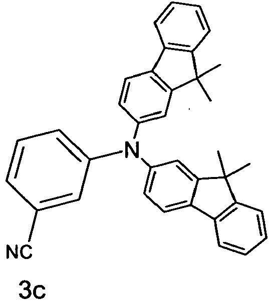 | 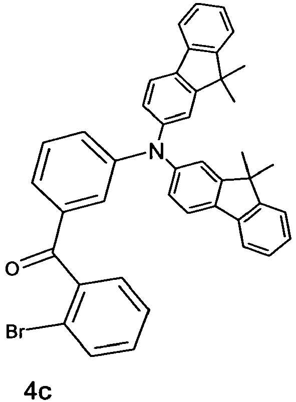 | 45 % |
| 4d | 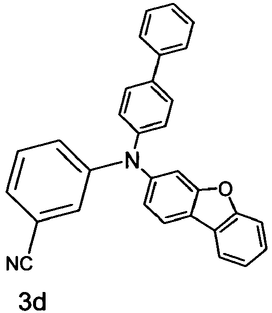 | 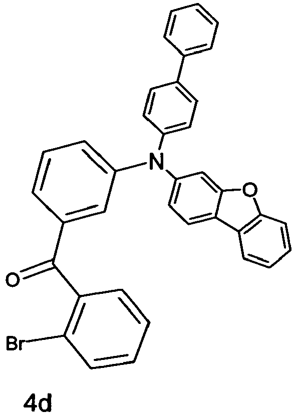 | 40 % |
| 4e | 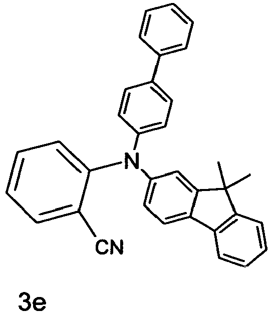 | 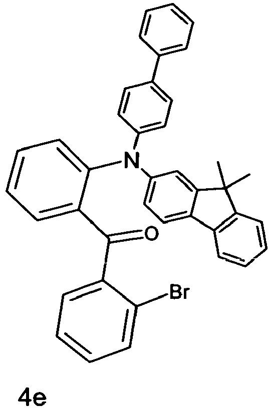 | 35% |
| 4f | 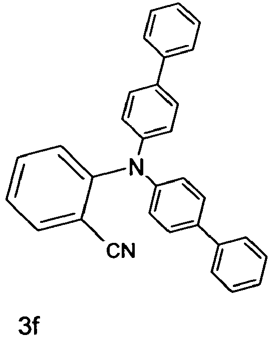 | 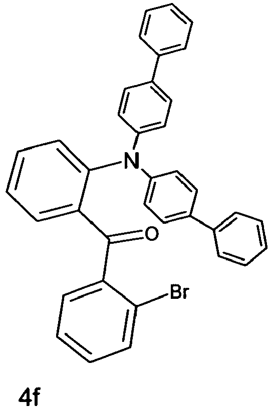 | 38 % |
| 4g | 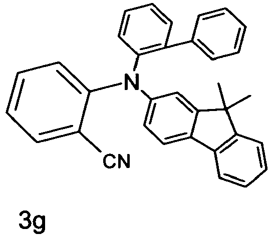 | 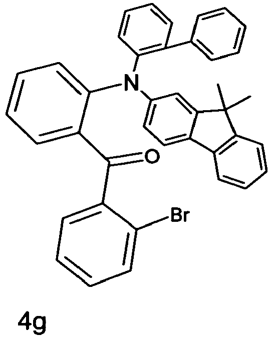 | 56 % |
| 4h | 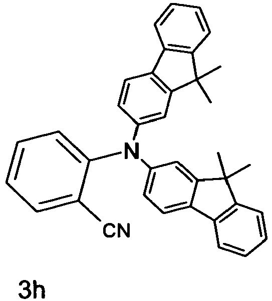 | 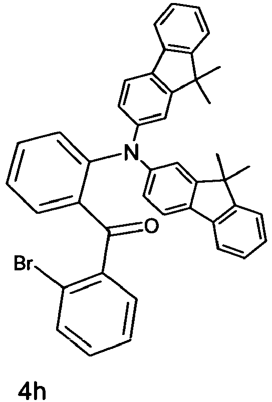 | 45 % |
| 4i | 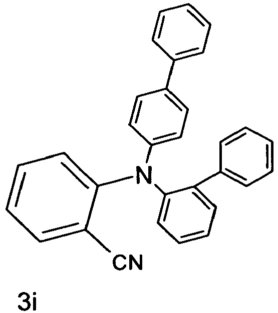 | 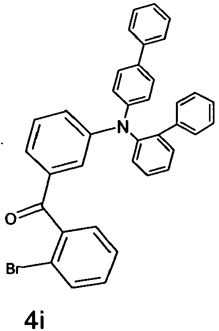 | 40 % |

### Beispiel 5: Biphenyl-4-yl-{3-[9-(2-brom-phenyl)-9H-fluoren-9-yl]-phenyl}-(9,9-dimethyl-9H-fluoren-2-yl)-amin

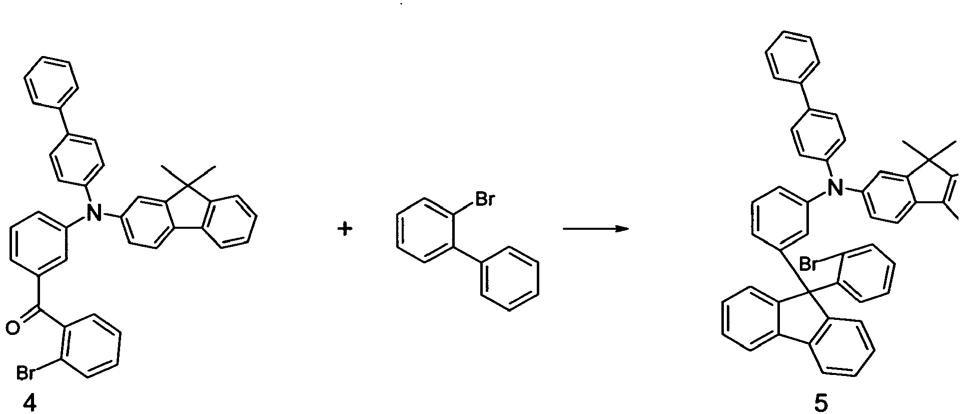

Aus mit Jod aktivierten 1,4 g (55 mmol) Magnesiumspänen und einer Mischung aus 12.9 g (55 mmol) 2-Brombiphenyl, 0.8 ml 1,2-Dichlorethan, 50 ml 1,2-Dimethoxyethan, 300 ml THF und 200 ml Toluol wird unter Begleitheizen mit einem 70 °C warmen Ölbad das entsprechende Grignard-Reagenz dargestellt. Nachdem das Magnesium vollständig abreagiert hat, lässt man auf Raumtemperatur erkalten und tropft dann eine Lösung von 31.1 g (50 mmol) {3-[Biphenyl-4-yl-(9,9-dimethyl-9H-fluoren-2-yl)-amino]-phenyl}-(2-bromo-phenyl)-methanon in 400 ml THF zu, erwärmt die Reaktionsmischung für 4 h auf 50 °C und rührt dann 12 h bei Raumtemperatur nach. Man gibt 100 ml Wasser zu, rührt kurz nach, trennt die org. Phase ab und entfernt das Lösungsmittel im Vakuum. Der Rückstand wird in der Wärme bei 40 °C in 500 ml Eisessig suspendiert, die Suspension wird mit 0.5 ml konz. Schwefelsäure versetzt, und anschließend 4 h bei 100 °C nachgerührt. Nach Erkalten saugt man vom ausgefallenen Feststoff ab, wäscht diesen einmal mit 100 ml Eisessig, dreimal mit je 100 ml Ethanol und kristallisiert abschließend aus Dioxan um. Ausbeute: 26.9 g (28 mmol), 68 %; Reinheit ca. 98 % n. ¹H-NMR.

Analog werden folgende Verbindungen erhalten:

| Bsp. | Edukt 1 | Produkt | Ausbeute |
|---|---|---|---|
| 5a | 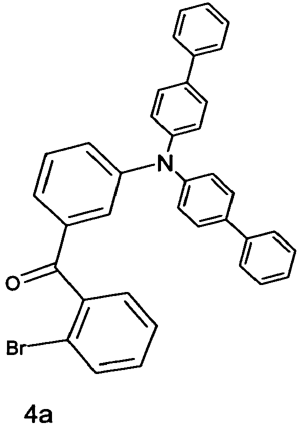 | 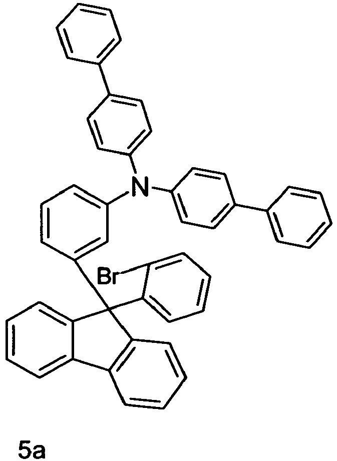 | 43 % |
| 5b | 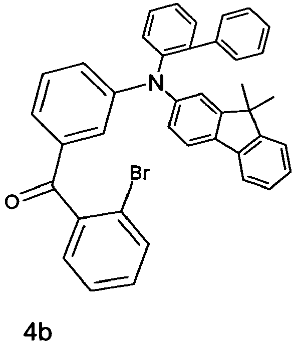 | 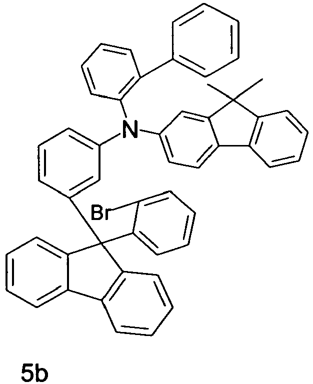 | 46 % |
| 5c | 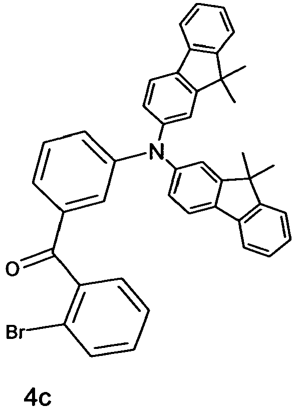 | 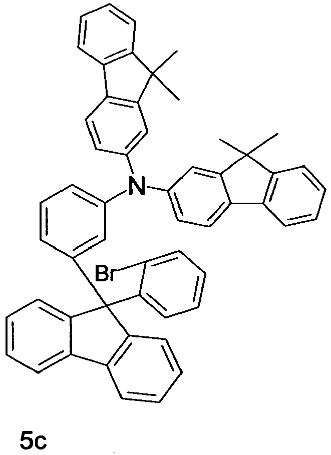 | 45 % |
| 5d | 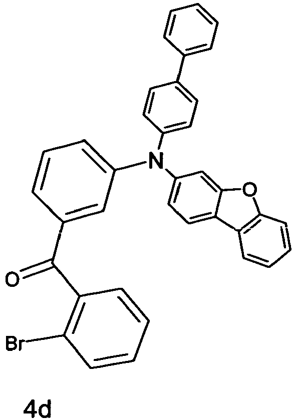 | 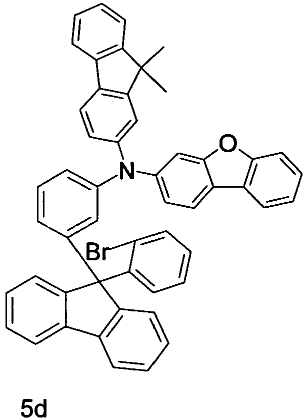 | 40 % |
| 5e | 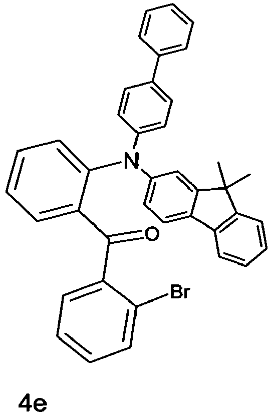 | 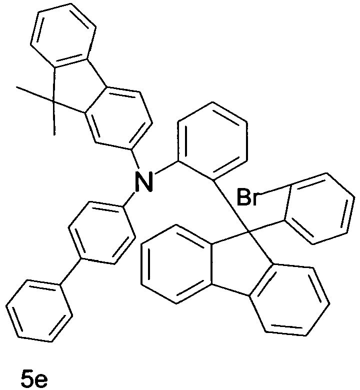 | 35% |
| 5f | 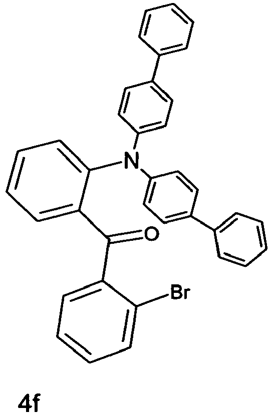 | 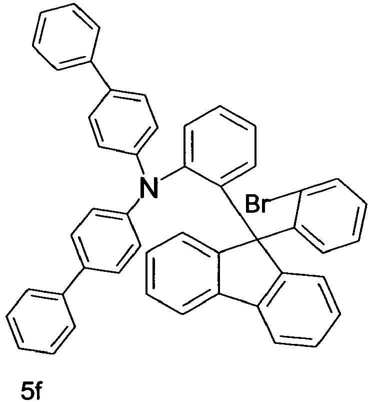 | 38 % |
| 5g | 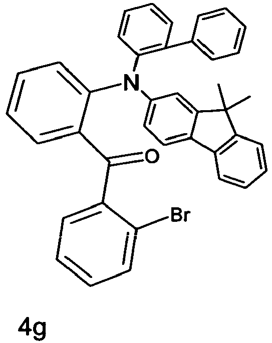 | 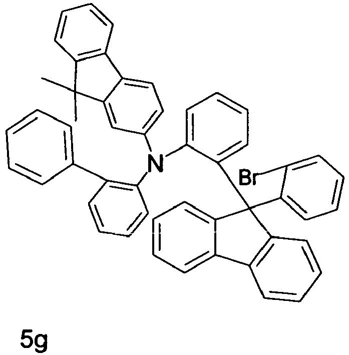 | 56 % |
| 5h | 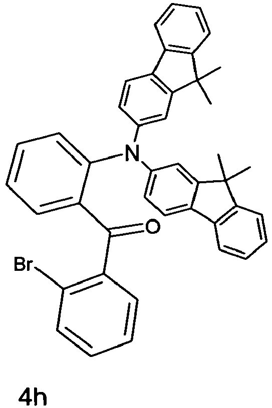 | 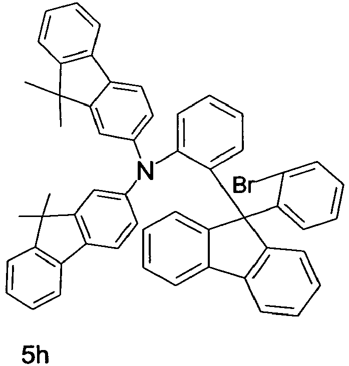 | 45 % |
| 5i | 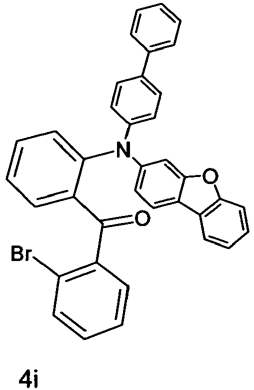 | 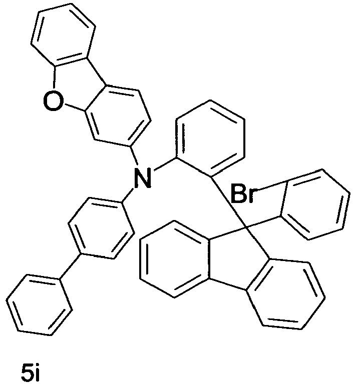 | 40 % |
| 5g | 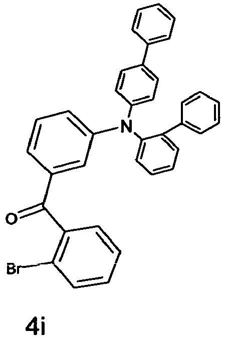 | 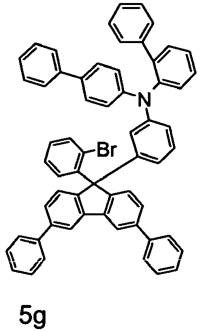 | 45 % |

### Synthese von erfindungsgemäßen Verbindungen:

### Beispiel 6: Biphenyl-4-yl-(9,9-dimethyl-9H-fluoren-2-yl)-(4-indeno[1,2,3-jk]fluoren-7b-yl-phenyl)-amin

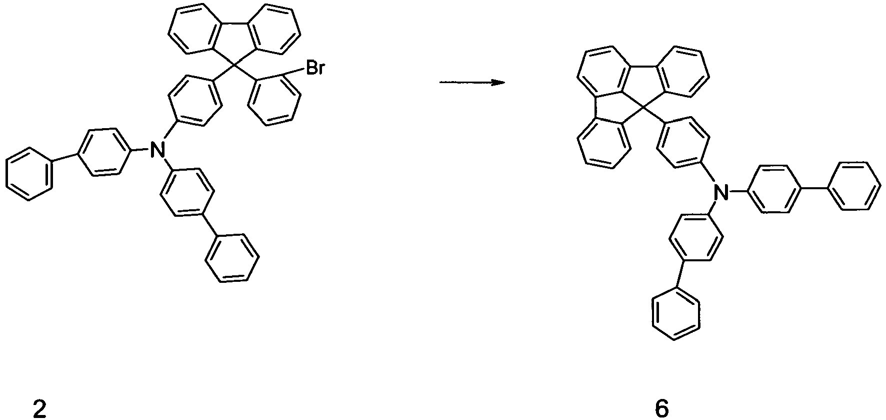

Ein Gemisch aus 38 g (53 mmol) Bis-biphenyl-4-yl-{4-[9-(2-brom-phenyl)-9H-fluoren-9-yl]-phenyl}-amin, 2,4 g (20 mmol) Palladium(II)acetat, 5,6 g (21,3 mmol) Triphenylphosphin, 28,14 g (267 mmol) Natriumcarbonat, 7,26 g (32 mmol) Benzyltriethylammoniumchlorid und 500 ml N,N-Dimethylacetamid wird 24 h unter Rückfluss erhitzt. Nach Erkalten versetzt man mit 200 ml Wasser, rührt 30 min. nach, trennt die org. Phase ab, filtriert diese über ein kurzes Celite-Bett und entfernt dann das Lösungsmittel im Vakuum. Der Rückstand wird fünfmal aus DMF/Heptan umkristallisiert und abschließend zweimal sublimiert (p ca. 10⁻⁶ mbar, T = 320 - 330 °C). Ausbeute: 14.5 g (22.8 mmol), 43 %; Reinheit: 99.9 % n. HPLC.

Analog werden folgende Verbindungen erhalten:

| Bsp. | Edukt | Produkt | Ausbeute |
|---|---|---|---|
| 6a | 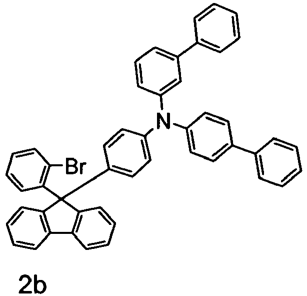 | 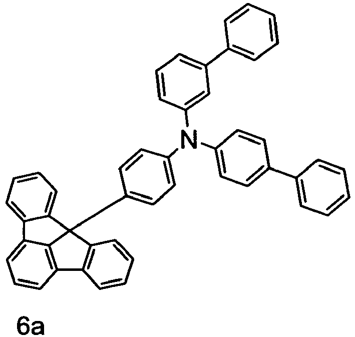 | 75 % |
| 6b | 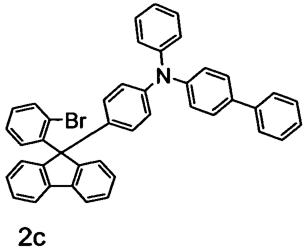 | 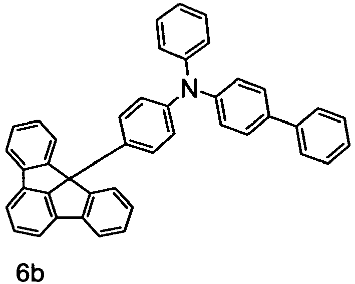 | 72 % |
| 6d | 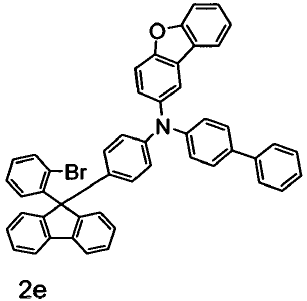 | 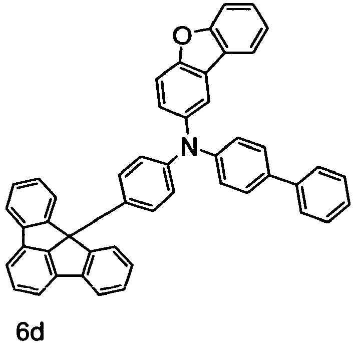 | 58 % |
| 6e | 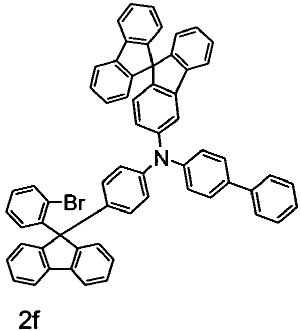 | 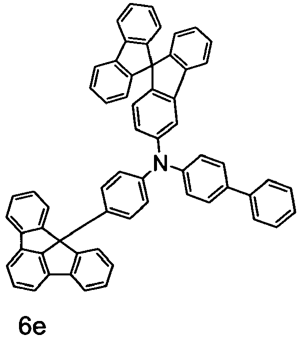 | 48% |
| 6f | 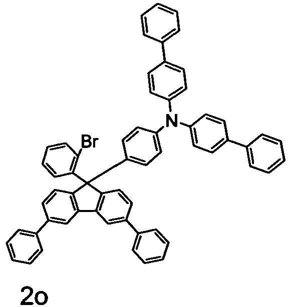 | 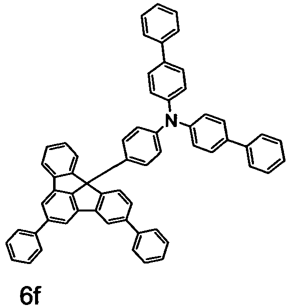 | 63 % |
| 6g | 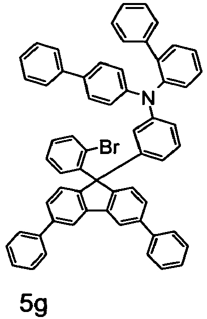 | 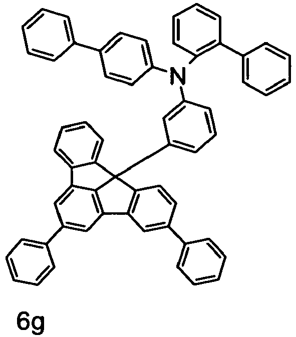 | 41% |
| 6h | 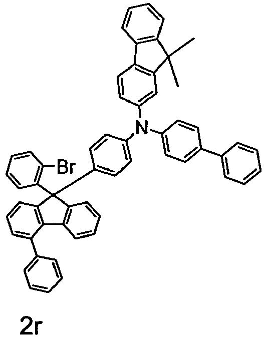 | 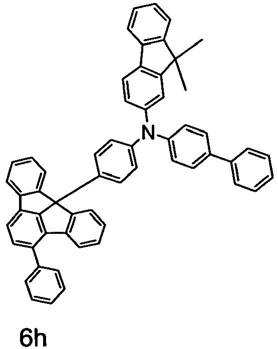 | 32% |
| 6j | 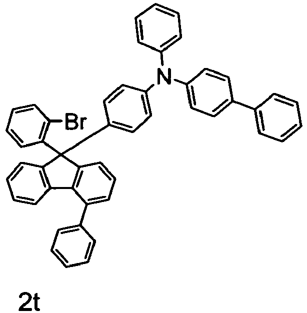 | 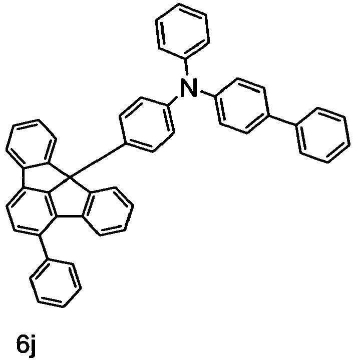 | 34% |
| 6k | 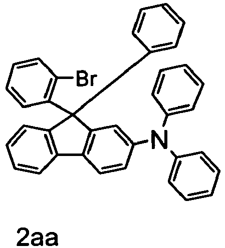 | 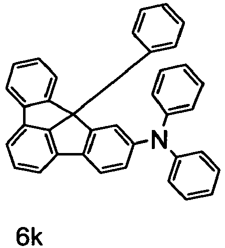 | 40% |
| 6l | 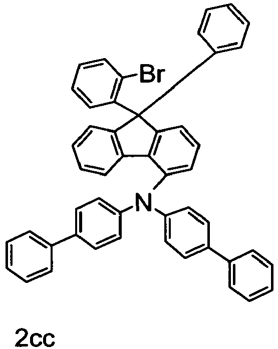 | 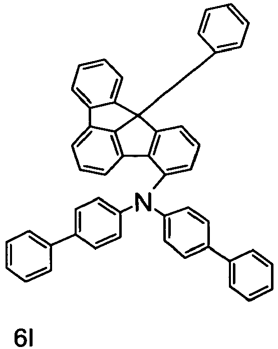 | 50 % |
| 6m | 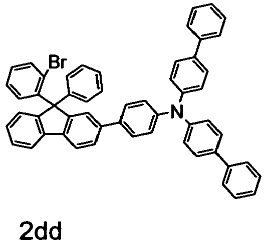 | 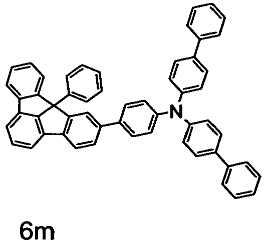 | 34% |
| 6n | 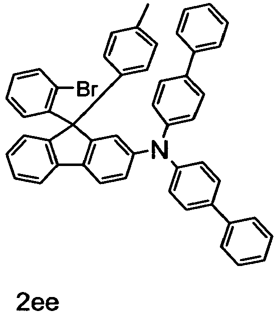 | 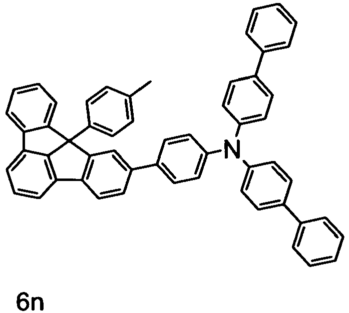 | 45% |
| 6o | 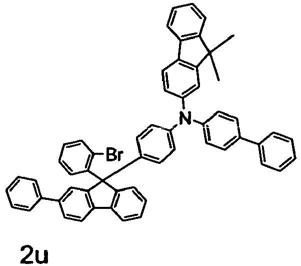 | 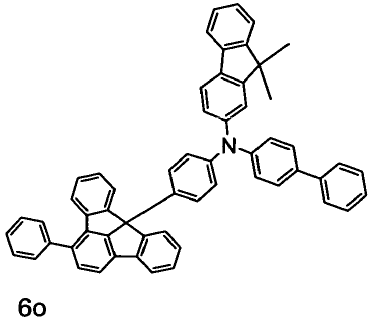 | 47% |
| 6p | 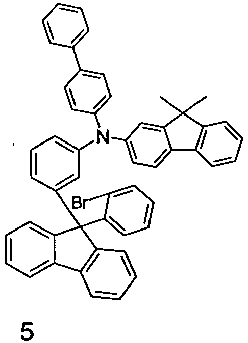 | 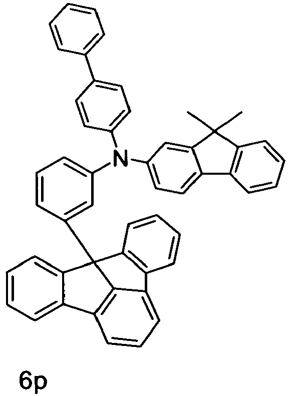 | 74% |
| 6r | 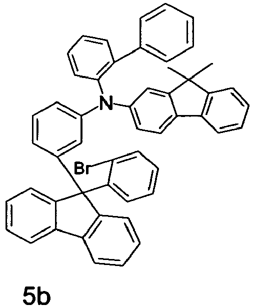 | 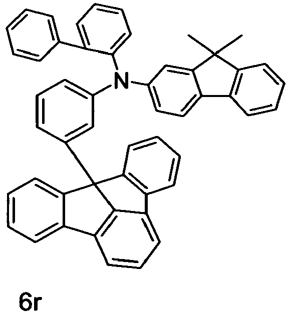 | 63% |
| 6s | 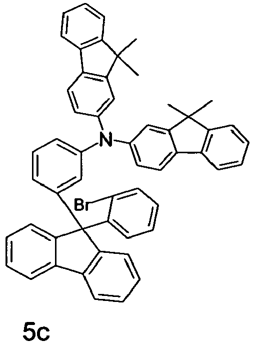 | 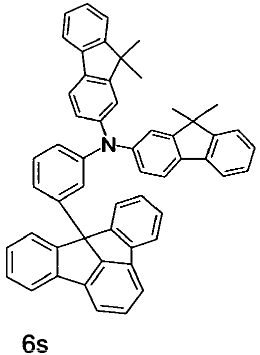 | 59% |
| 6t | 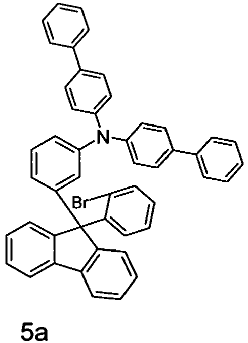 | 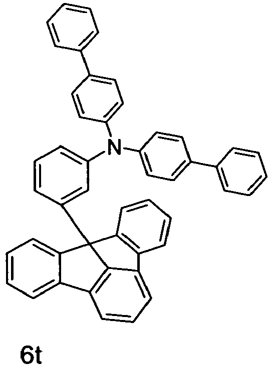 | 62% |
| 6u | 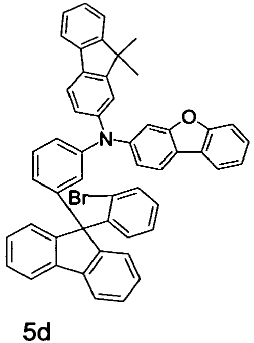 | 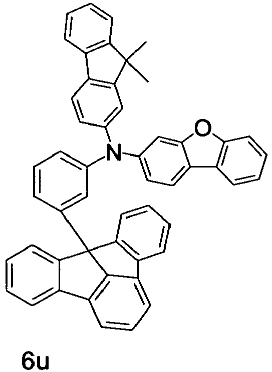 | 47% |
| 6v | 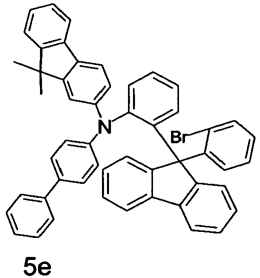 | 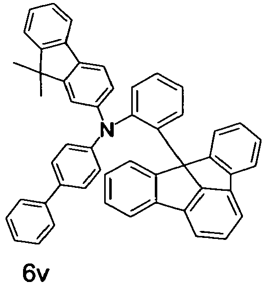 | 53% |
| 6w | 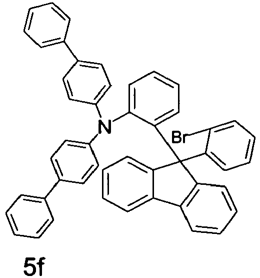 | 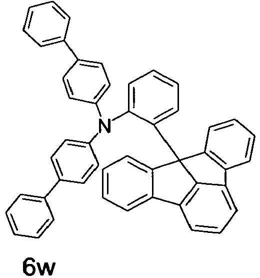 | 55% |
| 6x | 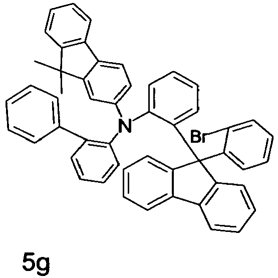 | 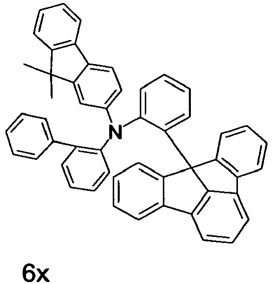 | 48% |
| 6y | 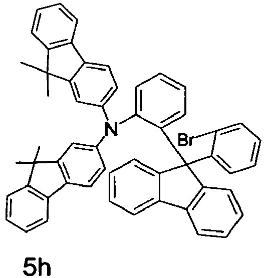 | 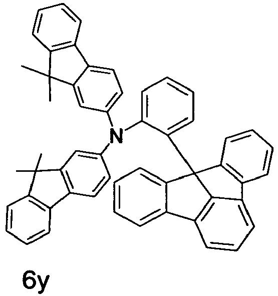 | 58% |
| 6z | 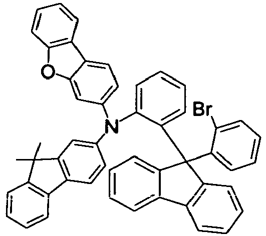 | 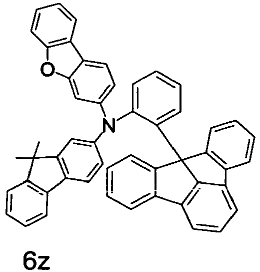 | 49% |
| 6aa | 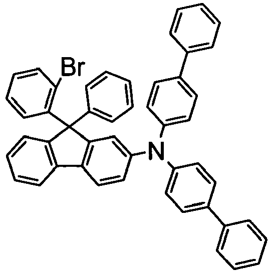 | 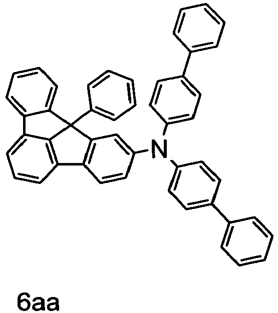 | 34 % |
| 6ab | 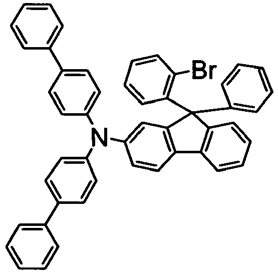 | 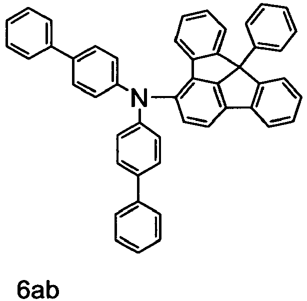 | 36 % |
| 6ac | 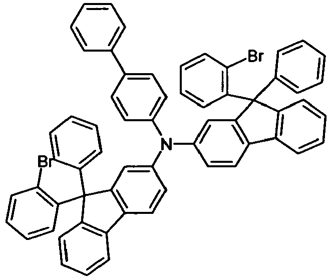 | 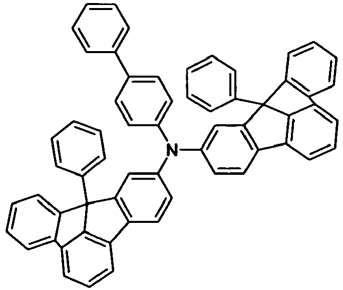 | 48 % |
| 6ad | 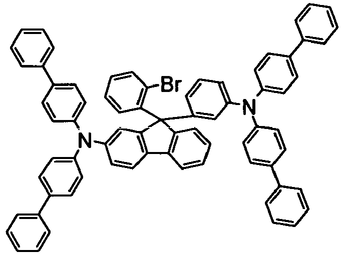 | 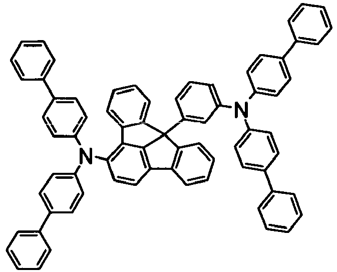 | 28 % |
| 6ae | 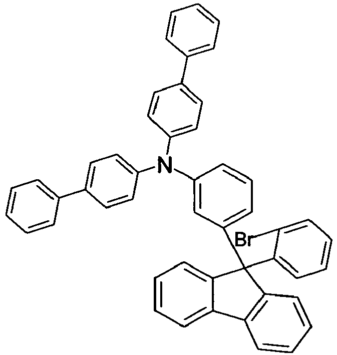 | 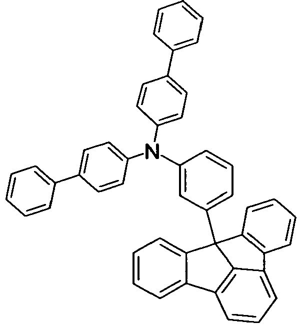 | 55 % |

### Teil B: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden erfinderischen Beispielen E1 bis E12 und in den Referenzbeispielen V1 und V2 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht A' (HIL1) / Lochtransportschicht A (HTL) / p-dotierte Lochtransportschicht B (HIL2) / Lochtransportschicht C (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, der Aufbau der verschiedenen hergestellten elektronischen Vorrichtungen in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB(5%) bedeutet hierbei, dass das Material H1 in einem Volumenanteil von 95% und SEB in einem Anteil von 5% in der Schicht vorliegt. Analog kann auch die Elektronentransportschicht oder die Lochinjektionsschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 10 mA/cm² bezeichnet die externe Quanteneffizienz bei einer Stromdichte von 10mA/cm². LD80 @ 60 mA/cm2 ist die Lebensdauer, bis zu der die Starthelligkeit der betrachteten OLED bei konstantem Strom von 60 mA/cm² auf 80 % der Anfangsintensität abgefallen ist.

| **Tabelle 1: Strukturen der verwendeten Materialien** | | |
|---|---|---|
| 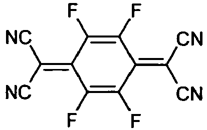 | 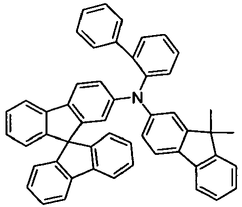 | 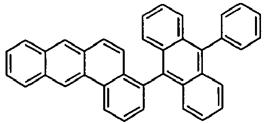 |
| F4TCNQ | HIM1 | H1 |
| 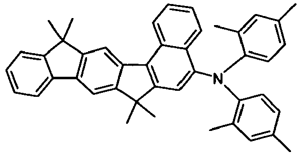 | 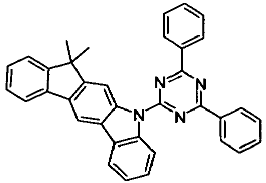 | 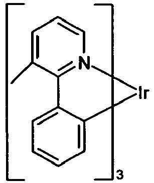 |
| SEB | H2 | TEG |
| 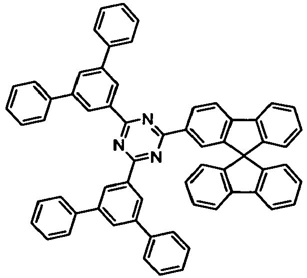 | 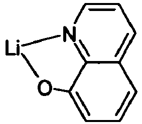 | |
| ETM | LiQ | |
| 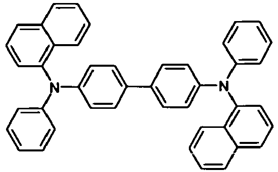 | 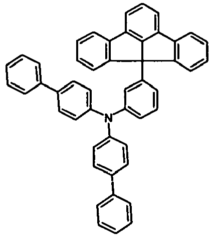 | 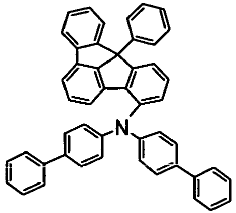 |
| NPB | HTM1 | HTM2 |
| 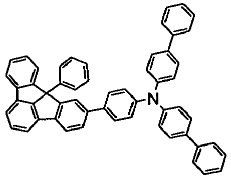 | 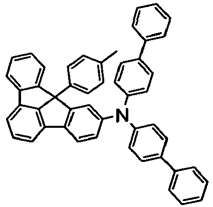 | 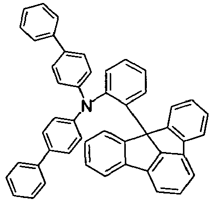 |
| HTM3 | HTM4 | HTM5 |
| 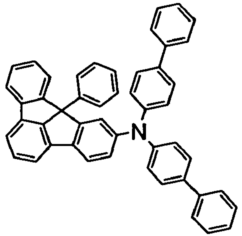 | 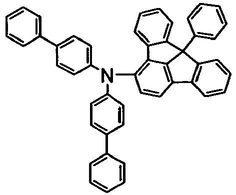 | |
| HTM6 | HTM7 | |

**Tabelle 2: Aufbau der OLEDs**

| **Bsp.** | ***HIL1*** | ***HTL*** | ***HIL2*** | ***EBL*** | ***EML*** | ***ETL*** | ***EIL*** |
|---|---|---|---|---|---|---|---|
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIM1:F4TCNQ(3%) 20 nm* | *HIM1 155 nm* | *NPB:F4TCNQ(3%) 20 nm* | *NPB 20 nm* | *H1:SEB(5%) 20 nm* | *ETM(50%):LiQ(50%) 30 nm* | *LiQ 1 nm* |
| *E1* | *HIM1:F4TCNQ(3%) 20 nm* | *HIM1 155 nm* | *HTM1:F4TCNQ(3%) 20 nm* | *HTM1 20 nm* | *H1:SEB(5%) 20 nm* | *ETM(50%):LiQ(50%) 30 nm* | *LiQ 1 nm* |
| *E2* | *HIM1:F4TCNQ(3%) 20 nm* | *HIM1 155 nm* | *HTM2:F4TCNQ(3%) 20 nm* | *HTM2 20 nm* | *H1:SEB(5%) 20 nm* | *ETM(50%):LiQ(50%) 30 nm* | *LiQ 1 nm* |
| *E3* | *HIM1:F4TCNQ(3%) 20 nm* | *HIM1 155 nm* | *HTM3:F4TCNQ(3%) 20 nm* | *HTM3 20 nm* | *H1:SEB(5%) 20 nm* | *ETM(50%):LiQ(50%) 30 nm* | *LiQ 1 nm* |
| *E4* | *HIM1:F4TCNQ(3%) 20 nm* | *HIM1 155 nm* | *HTM4:F4TCNQ(3%) 20 nm* | *HTM4 20 nm* | *H1:SEB(5%) 20 nm* | *ETM(50%):LiQ(50%) 30 nm* | *LiQ 1 nm* |
| *E5* | *HIM1:F4TCNQ(3%) 20 nm* | *HIM1 155 nm* | *HTM5:F4TCNQ(3%) 20 nm* | *HTM5 20 nm* | *H1:SEB(5%) 20 nm* | *ETM(50%):LiQ(50%) 30 nm* | *LiQ 1 nm* |
| *E6* | *HIM1:F4TCNQ(3%) 20 nm* | *HIM1 155 nm* | *HTM6:F4TCNQ(3%) 20 nm* | *HTM6 20 nm* | *H1:SEB(5%) 20 nm* | *ETM(5O%):LiQ(50%) 30 nm* | *LiQ 1 nm* |
| *E7* | *HIM1:F4TCNQ(3%) 20 nm* | *HIM1 155 nm* | *HTM7:F4TCNQ(3%) 20 nm* | *HTM7 20 nm* | *H1:SEB(5%) 20 nm* | *ETM(50%):LiQ(50%) 30 nm* | *LiQ 1 nm* |
| *V2* | *HIM1:F4TCNQ(3%) 20 nm* | *HIM1 210 nm* | *NPB:F4TCNQ(3%) 20 nm* | *NPB 20 nm* | *H2:TEG(5%) 30 nm* | *ETM(50%):LiQ(50%) 40 nm* | *LiQ 1 nm* |
| *E8* | *HIM1:F4TCNQ(3%) 20 nm* | *HIM1 210 nm* | *HTM1:F4TCNQ(3%) 20 nm* | *HTM1 20 nm* | *H2:TEG(5%) 30 nm* | *ETM(50%):LiQ(50%) 40 nm* | *LiQ 1 nm* |
| *E9* | *HIM1:F4TCNQ(3%) 20 nm* | *HIM1 210 nm* | *HTM2:F4TCNQ(3%) 20 nm* | *HTM2 20 nm* | *H2:TEG(5%) 30 nm* | *ETM(50%):LiQ(50%) 40 nm* | *LiQ 1 nm* |
| *E10* | *HIM1:F4TCNQ(3%) 20 nm* | *HIM1 210 nm* | *HTM3:F4TCNQ(3%) 20 nm* | *HTM3 20 nm* | *H2:TEG(5%) 30 nm* | *ETM(50%):LiQ(50%) 40 nm* | *LiQ 1 nm* |
| *E11* | *HIM1:F4TCNQ(3%) 20 nm* | *HIM1 210 nm* | *HTM4:F4TCNQ(3%) 20 nm* | *HTM4 20 nm* | *H2:TEG(5%) 30 nm* | *ETM(50%):LiQ(50%) 40 nm* | *LiQ 1 nm* |
| *E12* | *HIM1:F4TCNQ(3%) 20 nm* | *HIM1 210 nm* | *HTM5:F4TCNQ(3%) 20 nm* | *HTM5 20 nm* | *H2:TEG(5%) 30 nm* | *ETM(50%):LiQ(50%) 40 nm* | *LiQ 1 nm* |

### OLED Beispiel 1

Es wurde eine blaue fluoreszierende Referenzprobe V1 hergestellt und mit den erfindungsgemäßen Proben E1 bis E7 verglichen. Die Referenzprobe V1 hat bei einer Stromdichte von 10 mA/cm² eine externe Quanteneffizienz von 6.2 % und eine Lebensdauer (LD80 @ 60 mA/cm²) von 120 h. Verglichen damit haben die erfindungsgemäßen Proben E1 (7.5 %, 160 h), E2 (7.8 %, 250 h), E3 (7.6 %, 210 h), E4 (7.4 %, 200 h), E5 (8.0 %, 235 h), E6 (7.7 %, 270 h) und E7 (7.1 %, 260 h) sowohl bessere externe Quanteneffizienz bei einer Stromdichte von 10 mA/cm², als auch längere Lebensdauer (LD80 @ 60 mA/cm²).

### OLED Beispiel 2

Es wurde eine grüne phosphoreszierende Referenzprobe V2 hergestellt und mit den erfindungsgemäßen Proben E8 bis E12 verglichen. Die Referenzprobe V2 hat bei einer Stromdichte von 2 mA/cm² eine externe Quanteneffizienz von 11.7 % und eine Lebensdauer (LD80 @ 20 mA/cm²) von 80 h. Verglichen damit haben die erfindungsgemäßen Proben E8 (17.2 %, 135 h), E9 (18.7 %, 110 h), E10 (17.4 %, 145 h), E11 (19.0 %, 105 h) und E12 (20.2 %, 160 h) sowohl bessere externe Quanteneffizienz bei einer Stromdichte von 2 mA/cm², als auch längere Lebensdauer (LD80 @ 20 mA/cm²).

## Patentansprüche

1. Verbindung gemäß Formel (1), 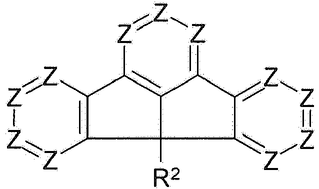 wobei für die auftretenden Symbole gilt:
Z ist gleich CR¹;
Ar¹ ist bei jedem Auftreten gleich oder verschieden ein aromatisches oder heteroaromatisches Ringsystem mit 5 bis 60 aromatischen Ringatomen, das mit einem oder mehreren Resten R³ substituiert sein kann; dabei können zwei Reste Ar¹, welche an dasselbe N-Atom oder P-Atom binden, auch durch eine Einfachbindung oder eine Brücke ausgewählt aus N(R⁴), C(R⁴)₂, O oder S, miteinander verbrückt sein.
R¹ ist bei jedem Auftreten gleich oder verschieden H, D, F, Cl, Br, I, CN, NO₂, N(Ar¹)₂, N(R³)₂, C(=O)Ar¹, C(=O)R³, P(=O)(Ar¹)₂, P(=O)(R³)₂, B(OR³)₂, CHO, Si(R³)₂, OSO₂R³, S(=O)R³, S(=O)₂R³, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen oder eine Alkenyl- oder Alkinylgruppe mit 2 bis 40 C-Atomen, wobei die Alkyl-, Alkoxy-, Thioalkyl-, Alkenyl- oder Alkinylgruppe jeweils mit einem oder mehreren Resten R³ substituiert sein kann und wobei eine oder mehrere benachbarte oder nicht benachbarte CH₂-Gruppen durch -R³C=CR³-, -C≡C-,C=O, C=S, C=Se, C=NR³, -C(=O)-NR³-, P(=O)(R³), -C(=O)-O-, Si(R³)₂, NR³, -O-, -S-, SO oder SO₂ ersetzt sein können und wobei ein oder mehrere H-Atome durch D, F, Cl, Br, I, CN oder NO₂ ersetzt sein können, oder ein aromatisches Ringsystem mit 6 bis 60 aromatischen Ringatomen, das jeweils mit einem oder mehreren Reste(n) R³ substituiert sein kann, oder ein heteroaromatisches Ringsystem mit 5 bis 60 aromatischen Ringatomen, das jeweils mit einem oder mehreren Reste(n) R³ substituiert sein kann, wobei zwei oder mehr Reste R¹ miteinander verknüpft sein können und einen Ring bilden können;
R² ist ein aromatisches Ringsystem mit 6 bis 60 aromatischen Ringatomen, das jeweils mit einem oder mehreren Reste(n) R³ substituiert sein kann;
R³ ist bei jedem Auftreten gleich oder verschieden H, D, F, Cl, Br, I, CN, NO₂, N(R⁴)₂, C(=O)Ar¹, C(=O)R⁴, P(=O)(Ar¹)₂, B(OR⁴)₂, CHO, Si(R⁴)₂, OSO₂R⁴, S(=O)R⁴, S(=O)₂R⁴, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen oder eine Alkenyl- oder Alkinylgruppe mit 2 bis 40 C-Atomen, wobei die Alkyl-, Alkoxy-, Thioalkyl-, Alkenyl- oder Alkinylgruppe jeweils mit einem oder mehreren Resten R⁴ substituiert sein kann und wobei eine oder mehrere benachbarte oder nicht benachbarte CH₂-Gruppen durch -R⁴C=CR⁴-, -C≡C-,C=O, C=S, C=Se, C=NR⁴, -C(=O)-NR⁴-, P(=O)(R⁴), -C(=O)-O-, Si(R⁴)₂, NR⁴, -O-, -S-, SO oder SO₂ ersetzt sein können und wobei ein oder mehrere H-Atome durch D, F, Cl, Br, I, CN oder NO₂ ersetzt sein können, oder ein aromatisches Ringsystem mit 6 bis 60 aromatischen Ringatomen, das jeweils mit einem oder mehreren Reste(n) R⁴ substituiert sein kann, oder ein heteroaromatisches Ringsystem mit 5 bis 60 aromatischen Ringatomen, das jeweils mit einem oder mehreren Reste(n) R⁴ substituiert sein kann, wobei zwei oder mehr Reste R⁴ miteinander verknüpft sein können und einen Ring bilden können;
R⁴ ist bei jedem Auftreten gleich oder verschieden, H, D, F, CN oder ein aliphatischer Rest mit 1 bis 20 C-Atomen, oder ein aromatisches Ringsystem mit 6 bis 60 aromatischen Ringatomen oder ein heteroaromatisches Ringsystem mit 5 bis 60 aromatischen Ringatomen, wobei in dem aliphatischen Rest, dem aromatischen Ringsystem oder dem heteroaromatischen Ringsystem ein oder mehrere H-Atome durch D, F, Cl, Br, I, CN oder eine Alkylgruppe mit 1 bis 5 C-Atomen ersetzt sein können, wobei zwei oder mehr Reste R⁴ miteinander verknüpft sein können und einen Ring bilden können;
wobei R² und/oder mindestens ein R¹ der Formel (1) mindestens ein aromatisches oder heteroaromatisches Ringsystem umfasst; und
wenn mindestens ein R¹ mindestens ein aromatisches oder heteroaromatisches Ringsystem umfasst, die Gesamtanzahl von aromatischen Ringatomen in allen R¹ und R² mindestens 12 beträgt; und
wenn R¹ kein aromatisches oder heteroaromatisches Ringsystem umfasst, R² mindestens 24 aromatische Ringatome sowie kein weiteres Indeno[1,2,3-jk]fluoren-Gerüst umfasst.

2. Verbindung nach Anspruch 1, **dadurch gekennzeichnet, dass** R¹ an mindestens einer der Positionen 5 oder 10 des Indeno[1,2,3-jk]fluoren-Gerüsts bei jedem Auftreten gleich oder verschieden H, D, F, CN, NO₂, N(Ar¹)₂, N(R³)₂, C(=O)Ar¹, P(=O)(Ar¹)₂, P(=O)(R³)₂, Si(R³)₂, OSO₂R³, S(=O)R³, S(=O)₂R³, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen oder eine Alkenyl- oder Alkinylgruppe mit 2 bis 40 C-Atomen, wobei die Alkyl-, Alkoxy-, Thioalkyl-, Alkenyl- oder Alkinylgruppe jeweils mit einem oder mehreren Resten R³ substituiert sein kann und wobei eine oder mehrere benachbarte oder nicht benachbarte CH₂-Gruppen durch -R³C=CR³-, -C≡C-,C=O, C=S, C=Se, C=NR³, -C(=O)-NR³-, P(=O)(R³), -C(=O)-O-, Si(R³)₂, NR³, -O-, -S-, SO oder SO₂ ersetzt sein können und wobei ein oder mehrere H-Atome durch D, F, Cl, Br, I, CN oder NO₂ ersetzt sein können, oder ein aromatisches Ringsystem mit 6 bis 60 aromatischen Ringatomen, das jeweils mit einem oder mehreren Reste(n) R³ substituiert sein kann, oder ein heteroaromatisches Ringsystem mit 5 bis 60 aromatischen Ringatomen ist, das jeweils mit einem oder mehreren Reste(n) R³ substituiert sein kann, wobei zwei oder mehr Reste R¹ miteinander verknüpft sein können und einen Ring bilden können.

3. Verbindung nach Anspruch 2,**dadurch gekennzeichnet, dass** R¹ an den Positionen 5 oder 10 des Indeno[1,2,3-jk]fluoren-Gerüsts, soweit jeweils vorhanden, keine Reste enthält ausgewählt aus der Gruppe enthaltend Cl, Br, I, B(OR³)₂ und CHO.

4. Verbindung nach einem oder mehreren der Ansprüche 1 bis 3, **dadurch gekennzeichnet, dass** mindestens ein R¹ nicht H ist.

5. Verbindung nach einem der Ansprüche 1 bis 4, **dadurch gekennzeichnet, dass** mindestens ein R¹ ein aromatisches oder heteroaromatisches Ringsystem umfasst.

6. Verfahren zur Herstellung einer Verbindung nach einem oder mehreren der Ansprüche 1 bis 5, **dadurch gekennzeichnet, dass** der Grundkörper der Formel (1) durch eine oder mehrere Kupplungsreaktionen aufgebaut wird.

7. Formulierung, enthaltend mindestens eine Verbindung nach einem oder mehreren der Ansprüche 1 bis 5.

8. Elektronische Vorrichtung, enthaltend mindestens eine Verbindung nach einem oder mehreren der Ansprüche 1 bis 5.

9. Elektronische Vorrichtung nach Anspruch 8, **dadurch gekennzeichnet, dass** sie ausgewählt ist aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

10. Elektronische Vorrichtung nach Anspruch 9, gewählt aus organischen Elektrolumineszenzvorrichtungen (OLEDs), **dadurch gekennzeichnet, dass** die Verbindung nach einem oder mehreren der Ansprüche 1 bis 5 in einer oder mehreren der folgenden Funktionen eingesetzt wird:
- als Lochtransportmaterial in einer Lochtransport- oder Lochinjektionsschicht,
- als Lochinjektionsmaterial,
- als Lochblockiermaterial,
- als Emitter für fluoreszierende Emissionsschichten,
- als Matrixmaterial in einer emittierenden Schicht,
- als Elektronenblockiermaterial,
- als Material für eine Zwischenschicht.

## Claims

1. Compound of the formula (1), 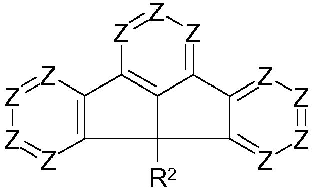 where the following applies to the symbols occurring:
Z is equal to CR¹;
Ar¹ is on each occurrence, identically or differently, an aromatic or heteroaromatic ring system having 5 to 60 aromatic ring atoms, which may be substituted by one or more radicals R³; two radicals Ar¹ which are bonded to the same N atom or P atom may also be bridged to one another by a single bond or a bridge selected from N(R⁴), C(R⁴)₂, O or S;
R¹ is on each occurrence, identically or differently, H, D, F, Cl, Br, I, CN, NO₂, N(Ar¹)₂, N(R³)₂, C(=O)Ar¹, C(=O)R³, P(=O)(Ar¹)₂, P(=O)(R³)₂, B(OR³)₂, CHO, Si(R³)₂, OSO₂R³, S(=O)R³, S(=O)₂R³, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, where the alkyl, alkoxy, thioalkyl, alkenyl or alkynyl group may in each case be substituted by one or more radicals R³ and where one or more adjacent or non-adjacent CH₂ groups may be replaced by -R³C=CR³-, -C≡C-, C=O, C=S, C=Se, C=NR³, -C(=O)-NR³-, P(=O)(R³), -C(=O)-O-, Si(R³)₂, NR³, -O-, -S-, SO or SO₂ and where one or more H atoms may be replaced by D, F, Cl, Br, I, CN or NO₂, or an aromatic ring system having 6 to 60 aromatic ring atoms, which may in each case be substituted by one or more radical(s) R³, or a heteroaromatic ring system having 5 to 60 aromatic ring atoms, which may in each case be substituted by one or more radical(s) R³, where two or more radicals R¹ may be linked to one another and may form a ring;
R² is an aromatic ring system having 6 to 60 aromatic ring atoms, which may in each case be substituted by one or more radical(s) R^{3;}
R³ is on each occurrence, identically or differently, H, D, F, Cl, Br, I, CN, NO₂, N(R⁴)₂, C(=O)Ar¹, C(=O)R⁴, P(=O)(Ar¹)₂, B(OR⁴)₂, CHO, Si(R⁴)₂, OSO₂R⁴, S(=O)R⁴, S(=O)₂R⁴, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, where the alkyl, alkoxy, thioalkyl, alkenyl or alkynyl group may in each case be substituted by one or more radicals R⁴ and where one or more adjacent or non-adjacent CH₂ groups may be replaced by -R⁴C=CR⁴-, -C≡C-, C=O, C=S, C=Se, C=NR⁴, -C(=O)-NR⁴-, P(=O)(R⁴), -C(=O)-O-, Si(R⁴)₂, NR⁴, -O-, -S-, SO or SO₂ and where one or more H atoms may be replaced by D, F, Cl, Br, I, CN or NO₂, or an aromatic ring system having 6 to 60 aromatic ring atoms, which may in each case be substituted by one or more radical(s) R⁴, or a heteroaromatic ring system having 5 to 60 aromatic ring atoms, which may in each case be substituted by one or more radical(s) R⁴, where two or more radicals R⁴ may be linked to one another and may form a ring;
R⁴ is on each occurrence, identically or differently, H, D, F, CN or an aliphatic radical having 1 to 20 C atoms, or an aromatic ring system having 6 to 60 aromatic ring atoms or a heteroaromatic ring system having 5 to 60 aromatic ring atoms, where one or more H atoms in the aliphatic radical, the aromatic ring system or the heteroaromatic ring system may be replaced by D, F, Cl, Br, I, CN or an alkyl group having 1 to 5 C atoms, where two or more radicals R⁴ may be linked to one another and may form a ring;
where R² and/or at least one R¹ in the formula (1) contains at least one aromatic or heteroaromatic ring system; and,
if at least one R¹ contains at least one aromatic or heteroaromatic ring system, the total number of aromatic ring atoms in all R¹ and R² is at least 12; and,
if R¹ does not contain an aromatic or heteroaromatic ring system, R² contains at least 24 aromatic ring atoms and no further indeno-[1,2,3-jk]fluorene structure.

2. Compound according to Claim 1, **characterised in that** R¹ at at least one of positions 5 or 10 of the indeno[1,2,3-jk]fluorene structure is on each occurrence, identically or differently, H, D, F, CN, NO₂, N(Ar¹)₂, N(R³)₂, C(=O)Ar¹, P(=O)(Ar¹)₂, P(=O)(R³)₂, Si(R³)₂, OSO₂R³, S(=O)R³, S(=O)₂R³, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, where the alkyl, alkoxy, thioalkyl, alkenyl or alkynyl group may in each case be substituted by one or more radicals R³ and where one or more adjacent or non-adjacent CH₂ groups may be replaced by-R³C=CR³-, -C≡C-, C=O, C=S, C=Se, C=NR³, -C(=O)-NR³-, P(=O)(R³), -C(=O)-O-, Si(R³)₂, NR³, -O-, -S-, SO or SO₂ and where one or more H atoms may be replaced by D, F, Cl, Br, I, CN or NO₂, or an aromatic ring system having 6 to 60 aromatic ring atoms, which may in each case be substituted by one or more radical(s) R³, or a heteroaromatic ring system having 5 to 60 aromatic ring atoms, which may in each case be substituted by one or more radical(s) R³, where two or more radicals R¹ may be linked to one another and may form a ring.

3. Compound according to Claim 2, **characterised in that** R¹ at positions 5 or 10 of the indeno[1,2,3-jk]fluorene structure, if present, contains no radicals selected from the group comprising Cl, Br, I, B(OR³)₂ and CHO.

4. Compound according to one or more of Claims 1 to 3, **characterised in that** at least one R¹ is not H.

5. Compound according to one of Claims 1 to 4, **characterised in that** at least one R¹ contains an aromatic or heteroaromatic ring system.

6. Process for the preparation of a compound according to one or more of Claims 1 to 5, **characterised in that** the basic structure of the formula (1) is built up by one or more coupling reactions.

7. Formulation comprising at least one compound according to one or more of Claims 1 to 5.

8. Electronic device containing at least one compound according to one or more of Claims 1 to 5.

9. Electronic device according to Claim 8, **characterised in that** it is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

10. Electronic device according to Claim 9, selected from organic electroluminescent devices (OLEDs), **characterised in that** the compound according to one or more of Claims 1 to 5 is employed in one or more of the following functions:
- as hole-transport material in a hole-transport or hole-injection layer,
- as hole-injection material,
- as hole-blocking material,
- as emitter for fluorescent emission layers,
- as matrix material in an emitting layer,
- as electron-blocking material,
- as material for an interlayer.

## Revendications

1. Composé de la formule (1) : 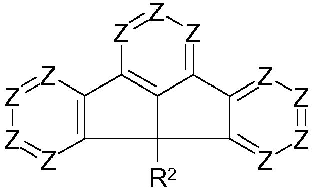 dans laquelle ce qui suit s'applique aux symboles qui sont rencontrés :
Z est égal à CR¹ ;
Ar¹ est pour chaque occurrence, de manière identique ou différente, un système de cycle aromatique ou hétéroaromatique qui comporte de 5 à 60 atomes de cycle aromatique, lequel peut être substitué par un radical ou par plusieurs radicaux R³; deux radicaux Ar¹ qui sont liés au même atome de N ou au même atome de P peuvent également être pontés l'un à l'autre au moyen d'une liaison simple ou d'un pont qui est sélectionné parmi N(R⁴), C(R⁴)₂, O ou S ;
R¹ est pour chaque occurrence, de manière identique ou différente, H, D, F, Cl, Br, I, CN, NO₂, N(Ar¹)₂, N(R³)₂, C(=O)Ar¹, C(=O)R³, P(=O)(Ar¹)₂, P(=O)(R³)₂, B(OR³)₂, CHO, Si(R³)₂, OSO₂R³, S(=O)R³, S(=O)₂R³, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, où le groupe alkyle, alcoxy, thioalkyle, alkényle ou alkynyle peut dans chaque cas être substitué par un radical ou par plusieurs radicaux R³ et où un ou plusieurs groupe(s) CH₂ adjacents ou non adjacents peut/peuvent être remplacé(s) par -R³C=CR³-, -C≡C-, C=O, C=S, C=Se, C=NR³, -C(=O)-NR³-, P(=O)(R³), -C(=O)-O-, Si(R³)₂, NR³, -O-, -S-, SO ou SO₂ et où un ou plusieurs atome(s) de H peut/peuvent être remplacé(s) par D, F, Cl, Br, I, CN ou NO₂, ou un système de cycle aromatique qui comporte de 6 à 60 atomes de cycle aromatique, lequel peut dans chaque cas être substitué par un radical ou par plusieurs radicaux R³, ou un système de cycle hétéroaromatique qui comporte de 5 à 60 atomes de cycle aromatique, lequel peut dans chaque cas être substitué par un radical ou par plusieurs radicaux R³, où deux radicaux R¹ ou plus peuvent être liés l'un à l'autre ou les uns aux autres et peuvent former un cycle ;
R² est un système de cycle aromatique qui comporte de 6 à 60 atomes de cycle aromatique, lequel peut dans chaque cas être substitué par un radical ou par plusieurs radicaux R³;
R³ est pour chaque occurrence, de manière identique ou différente, H, D, F, Cl, Br, I, CN, NO₂, N(R⁴)₂, C(=O)Ar¹, C(=O)R⁴, P(=O)(Ar¹)₂, B(OR⁴)₂, CHO, Si(R⁴)₂, OSO₂R⁴, S(=O)R⁴, S(=O)₂R⁴, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, où les groupes alkyle, alcoxy, thioalkyle, alkényle ou alkynyle peuvent dans chaque cas être substitués par un radical ou par plusieurs radicaux R⁴ et où un ou plusieurs groupe(s) CH₂ adjacents ou non adjacents peut/ peuvent être remplacé(s) par-R⁴C=CR⁴-, -C≡C-, C=O, C=S, C=Se, C=NR⁴, -C(=O)-NR⁴-, P(=O)(R⁴), -C(=O)-O-, Si(R⁴)2, NR⁴, -O-, -S-, SO ou SO₂ et où un ou plusieurs atome(s) de H peut/peuvent être remplacé(s) par D, F, Cl, Br, I, CN ou NO₂, ou un système de cycle aromatique qui comporte de 6 à 60 atomes de cycle aromatique, lequel peut dans chaque cas être substitué par un radical ou par plusieurs radicaux R⁴, ou un système de cycle hétéroaromatique qui comporte de 5 à 60 atomes de cycle aromatique, lequel peut dans chaque cas être substitué par un radical ou par plusieurs radicaux R⁴, où deux radicaux R⁴ ou plus peuvent être liés l'un à l'autre ou les uns aux autres et peuvent former un cycle ;
R⁴ est pour chaque occurrence, de manière identique ou différente, H, D, F, CN ou un radical aliphatique qui comporte de 1 à 20 atome(s) de C, où un système de cycle aromatique qui comporte de 6 à 60 atomes de cycle aromatique ou un système de cycle hétéroaromatique qui comporte de 5 à 60 atomes de cycle aromatique, où un ou plusieurs atome(s) de H dans le radical aliphatique, dans le système de cycle aromatique ou dans le système de cycle hétéroaromatique peut/peuvent être remplacé(s) par D, F, Cl, Br, I, CN ou un groupe alkyle qui comporte de 1 à 5 atome(s) de C, où deux radicaux R⁴ ou plus peuvent être liés l'un à l'autre ou les uns aux autres et peuvent former un cycle ;
où R² et/ou au moins un R¹ dans la formule (1) contiennent/contient au moins un système de cycle aromatique ou hétéroaromatique ; et
si au moins un R¹ contient au moins un système de cycle aromatique ou hétéroaromatique, le nombre total d'atomes de cycle aromatique dans tous les R¹ et R² est d'au moins 12 ; et
si R¹ ne contient pas un système de cycle aromatique ou hétéroaromatique, R² contient au moins 24 atomes de cycle aromatique et pas d'autre structure indéno[1,2,3-jk]fluorène.

2. Composé selon la revendication 1, **caractérisé en ce que** R¹ au niveau d'au moins l'une de positions 5 ou 10 de la structure indéno-[1,2,3-jk]fluorène est pour chaque occurrence, de manière identique ou différente, H, D, F, CN, NO₂, N(Ar¹)₂, N(R³)₂, C(=O)Ar¹, P(=O)(Ar¹)₂, P(=O)(R³)₂, Si(R³)₂, OSO₂R³, S(=O)R³, S(=O)₂R³, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, où le groupe alkyle, alcoxy, thioalkyle, alkényle ou alkynyle peut dans chaque cas être substitué par un radical ou par plusieurs radicaux R³ et où un ou plusieurs groupe(s) CH₂ adjacents ou non adjacents peut/peuvent être remplacé(s) par -R³C=CR³-, -C≡C-, C=O, C=S, C=Se, C=NR³, -C(=O)-NR³-, P(=O)(R³), -C(=O)-O-, Si(R³)₂, NR³, -O-, -S-, SO ou SO₂ et où un ou plusieurs atome(s) de H peut/peuvent être remplacé(s) par D, F, Cl, Br, I, CN ou NO₂, ou un système de cycle aromatique qui comporte de 6 à 60 atomes de cycle aromatique, lequel peut dans chaque cas être substitué par un radical ou par plusieurs radicaux R³, ou un système de cycle hétéroaromatique qui comporte de 5 à 60 atomes de cycle aromatique, lequel peut dans chaque cas être substitué par un radical ou par plusieurs radicaux R³, où deux radicaux R¹ ou plus peuvent être liés l'un à l'autre ou les uns aux autres et peuvent former un cycle.

3. Composé selon la revendication 2, **caractérisé en ce que** R¹ au niveau des positions 5 ou 10 de la structure indéno[1,2,3-jk]fluorène, s'il est présent, ne contient pas de radicaux sélectionnés parmi le groupe qui comprend Cl, Br, I, B(OR³)₂ et CHO.

4. Composé selon une ou plusieurs des revendications 1 à 3, **caractérisé en ce qu'**au moins un R¹ n'est pas H.

5. Composé selon l'une des revendications 1 à 4, **caractérisé en ce qu'**au moins un R¹ contient un système de cycle aromatique ou hétéroaromatique.

6. Procédé pour la préparation d'un composé selon une ou plusieurs des revendications 1 à 5, **caractérisé en ce que** la structure de base de la formule (1) est constituée au moyen d'une ou de plusieurs réaction(s) de couplage.

7. Formulation comprenant au moins un composé selon une ou plusieurs des revendications 1 à 5.

8. Dispositif électronique contenant au moins un composé selon une ou plusieurs des revendications 1 à 5.

9. Dispositif électronique selon la revendication 8, **caractérisé en ce qu'**il est sélectionné parmi les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED).

10. Dispositif électronique selon la revendication 9, sélectionné parmi les dispositifs électroluminescents organiques (OLED), **caractérisé en ce que** le composé selon une ou plusieurs des revendications 1 à 5 est utilisé au niveau d'une ou de plusieurs des fonctions qui suivent :
- en tant que matériau de transport de trous dans une couche de transport de trous ou d'injection de trous ;
- en tant que matériau d'injection de trous ;
- en tant que matériau de blocage de trous ;
- en tant qu'émetteur pour des couches à émission fluorescente ;
- en tant que matériau de matrice dans une couche d'émission ;
- en tant que matériau de blocage d'électrons ;
- en tant que matériau pour une intercouche.
